# EUROPEAN PATENT SPECIFICATION

(11) **EP 3 384 054 B1**
(45) Date of publication and mention of the grant of the patent: **29.11.2023**
(21) Application number: 16871195.0
(22) Date of filing: 25.08.2016
(51) Int. Cl.: C12Q 1/6883, C12Q 1/6886

(54) **MULTIPLEX CELLULAR REFERENCE MATERIALS**
ZELLULARE MULTIPLEX-REFERENZMATERIALIEN
MATÉRIAUX CELLULAIRES DE RÉFÉRENCE MULTIPLEXÉS

(30) Priority: 01.12.2015 US 201562261514 P; 16.04.2016 US 201662323659 P
(43) Date of publication of application: 10.10.2018
(73) Proprietor: LGC Clinical Diagnostics, Inc., Milford, MA 01757 (US)
(72) Inventor: HUANG, Catherine, Elkridge, MD 21075 (US); ANEKELLA, Bharathi, Clarksburg, MD 20871 (US); NGUYEN, Lequan, T., Olney, MD 20832 (US)
(74) Representative: Potter Clarkson
(86) International application number: PCT/US2016/048668
(87) International publication number: WO 2017/095487

(56) References cited:
- WO-A1-2015/069791
- WO-A1-2015/073080
- WO-A1-2015/118513
- US-A1- 2010 286 143
- US-A1- 2011 014 622
- US-A1- 2012 108 460
- Thermo Fisher: "AcroMetrix(TM) Oncology Hotspot Control For In Vitro Diagnostic Use 969056 AcroMetrix Oncology Hotspot Control", , 1 January 2015 (2015-01-01), XP055320492, Retrieved from the Internet: URL:https://tools.thermofisher.com/content /sfs/manuals/MAN0010820-AMX-Oncology-Hotsp ot-Ctrl-EN.pdf [retrieved on 2016-11-17]
- ALEXANDRA R FERNANDES ET AL: "Genetics of hypertrophic cardiomyopathy: advances and pitfalls in molecular diagnosis and therapy", THE APPLICATION OF CLINICAL GENETICS, 1 October 2014 (2014-10-01), page 195, XP055597712, DOI: 10.2147/TACG.S49126
- LUC^|^IACUTE;A N^|^UACUTE;^|^NTILDE;EZ ET AL: "Somatic MYH7, MYBPC3, TPM1, TNNT2 and TNNI3 Mutations in Sporadic Hypertrophic Cardiomyopathy", CIRCULATION JOURNAL, vol. 77, no. 9, 1 January 2013 (2013-01-01), pages 2358-2365, XP055597713, JP ISSN: 1346-9843, DOI: 10.1253/circj.CJ-13-0294

## Description

### BACKGROUND

Cell-based reference materials are useful as process controls in analyzing samples or validating methods. Conventional reference materials are limited, however, in that a library of controls may be necessary to analyze a sample with unknown features. For example, certain cancer assays screen for a number of different biomarkers, and each biomarker may require a different reference material, which complicates the analysis. Streamlined approaches for analyzing samples with unknown features are therefore desirable.

WO 2015/073080 A1 discloses a plurality of nucleic acid sequences comprising multiple variants of a reference sequence. The disclosure further provides plasmids, cells, methods and kits comprising the same.

### SUMMARY

The invention is as set out in the claims.

Aspects of the disclosure relate to a nucleic acid, comprising a plurality of nucleotide sequences, wherein each nucleotide sequence of the plurality comprises a genotype that is associated with a disease or condition. Each nucleotide sequence is 600-10,000 base pairs long; and the plurality of nucleotide sequences comprises nucleotide sequences from at least 2 different chromosomes. For example, each nucleotide sequence of the plurality may be a human nucleotide sequence, and each genotype may be a somatic or inheritable mutation that is associated with a neoplasm. Some aspects relate to a cell comprising the nucleic acid, *e.g.,* wherein the cell is a human cell. Other aspects relate to a biological reference material comprising a plurality of cells comprising the nucleic acid, and optionally a plurality of cells that do not comprise the nucleic acid (*e.g*., untransfected cells). The cells of the reference material may be fixed (*e.g*., with formalin) and embedded in paraffin. Alternatively, the biological reference material comprises a plurality of cells comprising the nucleic acid and a liquid, optionally wherein the liquid is plasma.

### BRIEF DESCRIPTION OF THE DRAWINGS

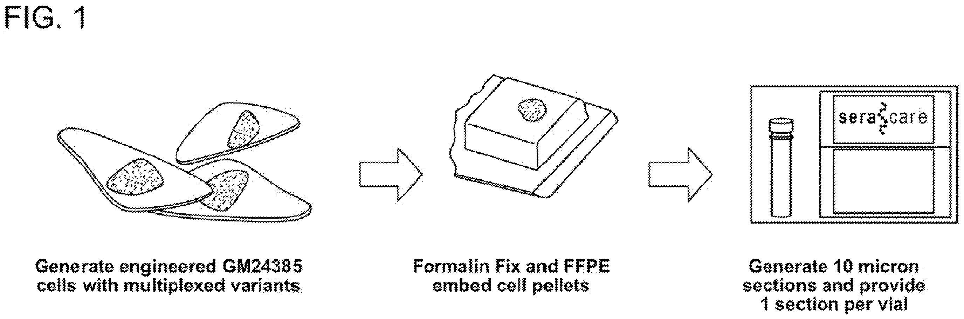

**Figure 1** is a diagram of the production of a biological reference material. Human cells, such as the well-characterized GM24385 cell line, may be transfected with a nucleic acid comprising a number of different genotypes (multiplexed variants). The genotypes may be from one or more genes, and each genotype may be associated with a disease, such as a neoplasm. The cells may be fixed *(e.g.,* in formalin), embedded in paraffin, and sectioned. The final biological reference material may be sections of formalin-fixed paraffin-embedded (FFPE) cells provided in a vial or tube.

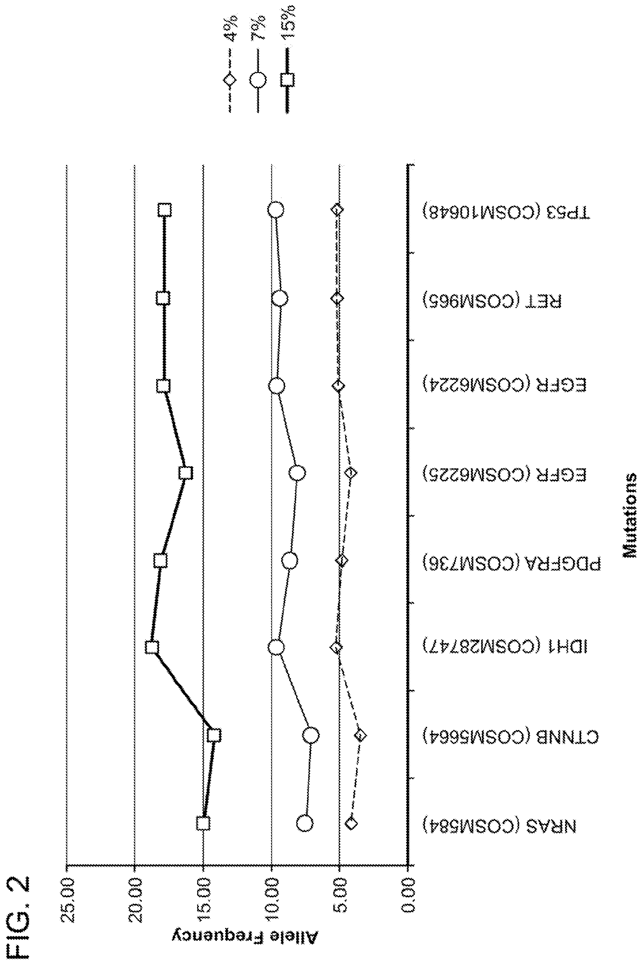

**Figure 2** is a graph depicting the digital PCR quantification of eight different genotypes in reference materials comprising GM24385 cells, wherein some of the GM24385 cells contain a nucleic acid comprising each genotype. The cells have been diluted with GM24385 cells that do not contain the nucleic acid, such that the percentage of each genotype relative to the wild type allele in the reference material is about 4%, about 7%, or about 15%.

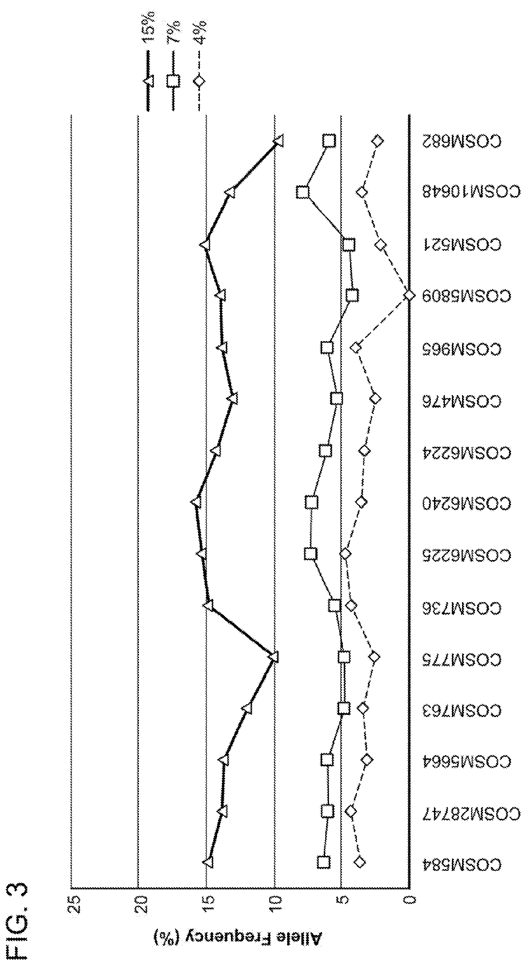

**Figure 3** is a graph depicting next generation sequencing quantification of fifteen different genotypes in reference materials comprising GM24385 cells, wherein some of the GM24385 cells contain a nucleic acid comprising each genotype. The cells have been diluted with GM24385 cells that do not contain the nucleic acid, such that the percentage of each genotype relative to the wild type allele in the reference material is about 4%, about 7%, or about 15%.

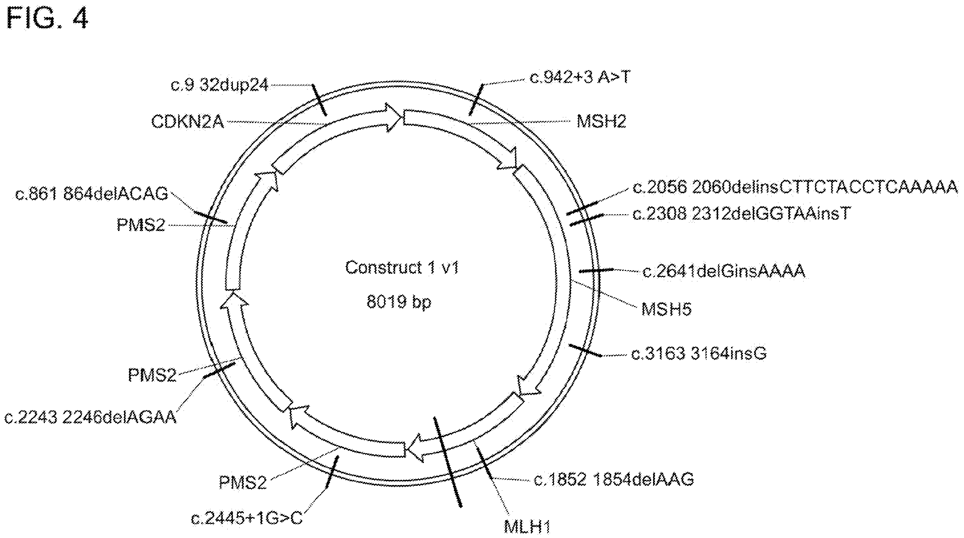

**Figure 4** is a plasmid map of a plasmid described in Example 2, which comprises inheritable genetic mutations to subsequences of various genes, including mutation c.942+3A>T to gene MSH2 (mutS homolog 2); mutations c.2056_2060delinsCTTCTACCTCAAAAA (SEQ ID NO: 13), c.2308_2312delGGTAAinsT, c.2641delGinsAAAA, and c.3163_3164insGto gene MSH6 (mutS homolog 6); mutation c.1852_1854delAAG to gene MLH1 (mutL homolog 1); mutations c.2445+1G>C, c.2243_2246delAGAA, and c.861_864delACAG to gene PMS2 (PMS1 homolog 2, mismatch repair system component); and mutation c.9_32dup24 to gene CDKN2A (cyclin dependent kinase inhibitor 2A).

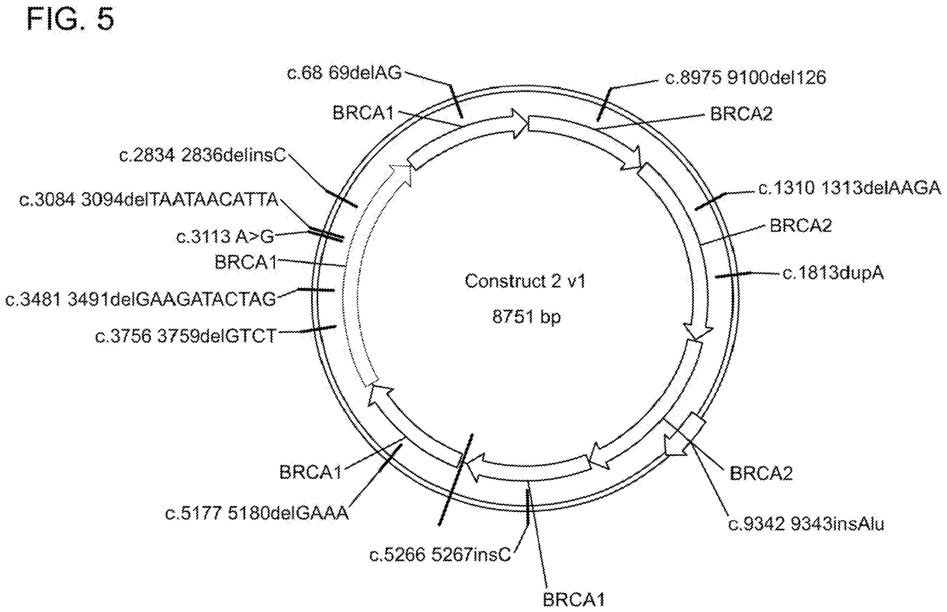

**Figure 5** is a plasmid map of a plasmid described in Example 2, which comprises inheritable genetic mutations to subsequences of various genes, including mutations c.8975_9100del126, c.1310_1313delAAGA, c.1813dupA, and c.9342_9343insAluY to gene BRCA2 (breast cancer 2) and mutations c.5266_5267insC, c.5177_5180delGAAA, c.3756_3759delGTCT, c.3481_3491delGAAGATACTAG (SEQ ID NO: 14), c.3113A>G, c.3084_3094delTAATAACATTA (SEQ ID NO: 15), c.2834_2836delinsC, and c.68_69delAG to gene BRCA1 (breast cancer 1).

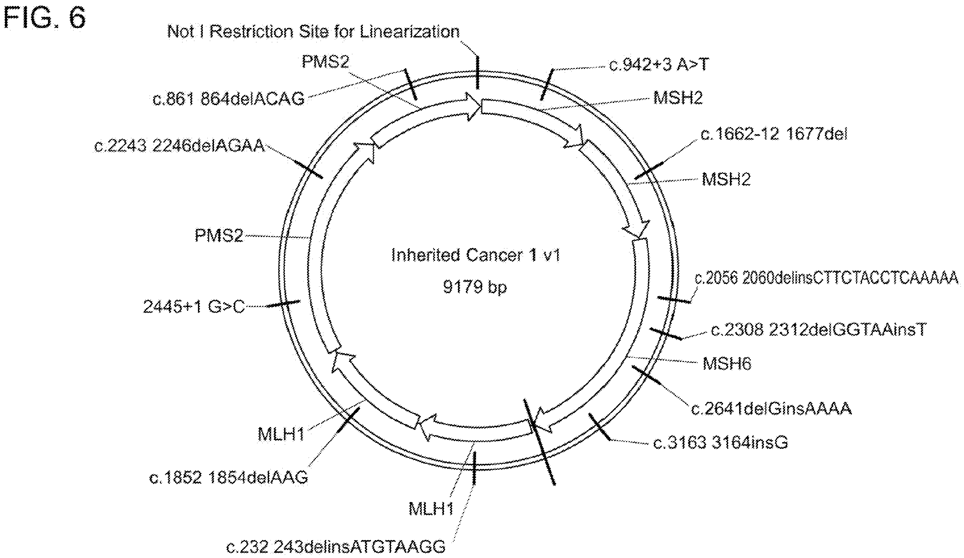

**Figure 6** is a plasmid map of a plasmid described in Example 2, which comprises inheritable genetic mutations to subsequences of various genes, including mutation c.942+3A>T to gene MSH2 (mutS homolog 2); mutation c.1662-12 1677del to gene MSH2; mutations c.2056_2060delinsCTTCTACCTCAAAAA (SEQ ID NO: 13), c.2308_2312delGGTAAinsT, c.2641delGinsAAAA, and c.3163_3164insG to gene MSH6 (mutS homolog 6), which each occur in the same nucleotide sequence; mutation c.232_243delinsATGTAAGGto gene MLH1 (mutL homolog 1), mutation c.1852_1854delAAG to gene MLH1; and mutations c.2445+1G>C, c.2243_2246delAGAA, and c.861_864delACAG to gene PMS2 (PMS1 homolog 2, mismatch repair system component).

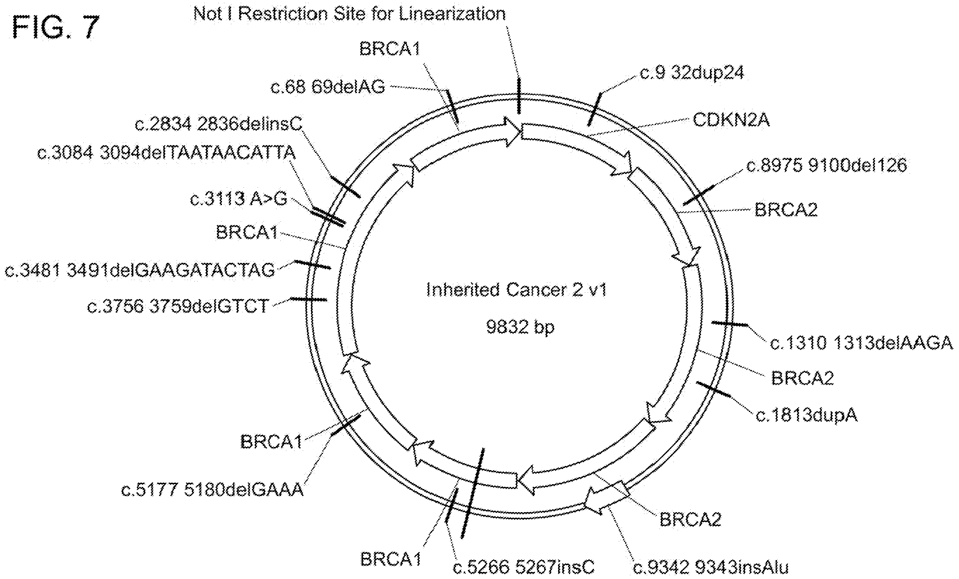

**Figure 7** is a plasmid map of a plasmid described in Example 2, which comprises inheritable genetic mutations to subsequences of various genes, including mutation c.9_32dup24 to gene CDKN2A (cyclin dependent kinase inhibitor 2A); mutations c.8975_9100del126, c.1310_1313delAAGA, c.1813dupA, and c.9342_9343insAluY to gene BRCA2 (breast cancer 2); and mutations c.5266_5267insC, c.5177_5180delGAAA, c.3756_3759delGTCT, c.3481_3491delGAAGATACTAG (SEQ ID NO: 14), c.3113A>G, c.3084_3094delTAATAACATTA (SEQ ID NO: 15), c.2834_2836delinsC, and c.68_69delAG to gene BRCA1 (breast cancer 1).

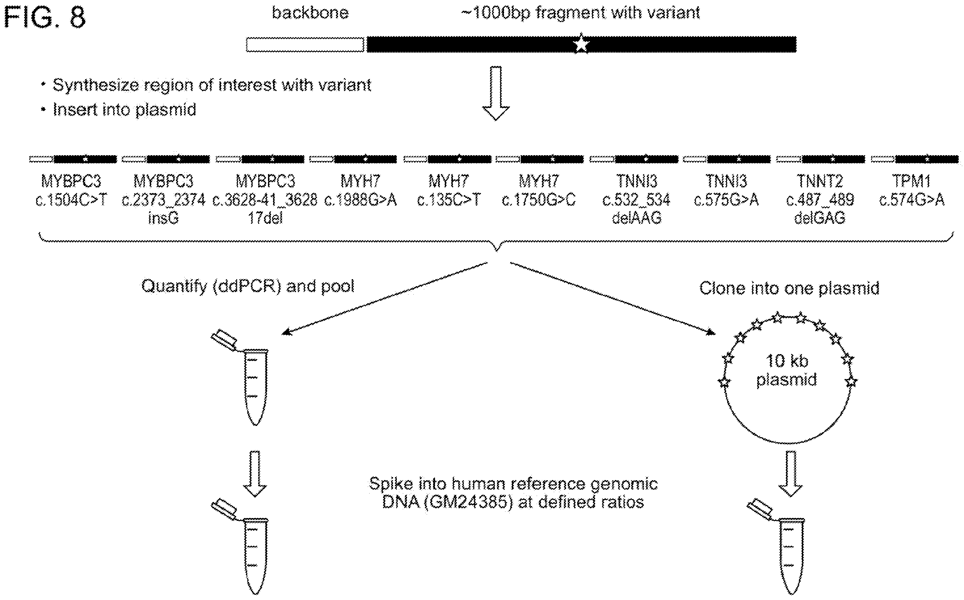

**Figure 8** depicts the strategy for engineering a multiplex reference material comprising 10 different genotypes that are associated with cardiomyopathy. 10 nucleotide sequences comprising point mutations or indels associated with cardiomyopathy were cloned into a single plasmid, and the plasmid was combined with genomic DNA at various different ratios. The 10 nucleotide sequences were also cloned into 10 different plasmids and combined with genomic DNA at various different ratios as a control, to assess the performance of the multiplexed format relative to a pooled format.

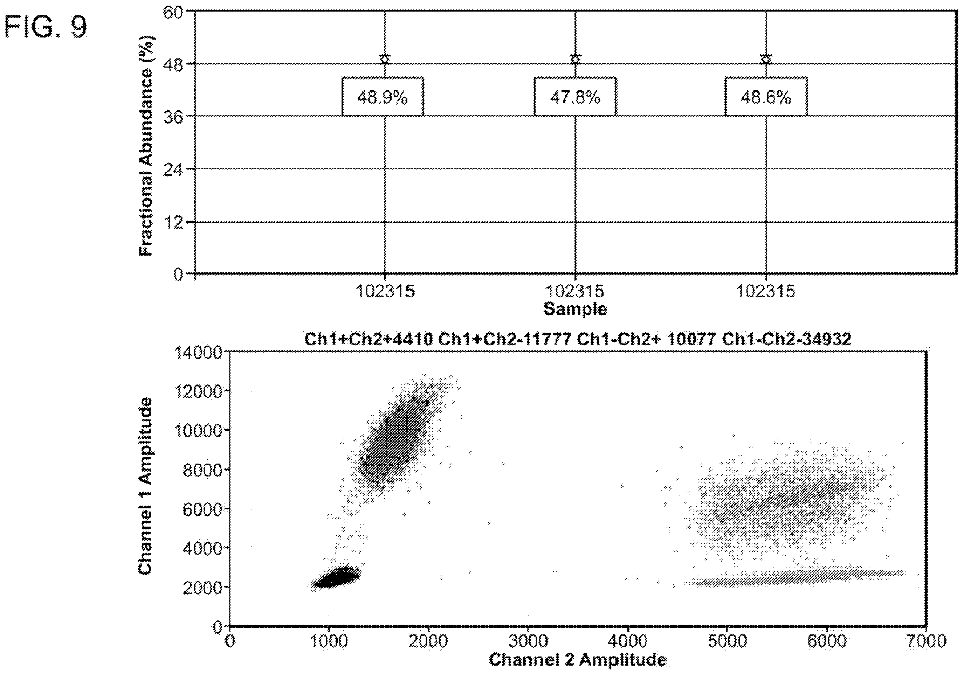

**Figure 9** shows droplet digital PCR results for multiplex cardiomyopathy reference material lot 102315, which comprises a linearized plasmid of the reference material and genomic DNA mixed together at an allelic frequency of 50%, to serve as a heterozygous reference material. PCR correctly identified mutant MYBPC3 3628-41_3628-17del at the expected frequency.

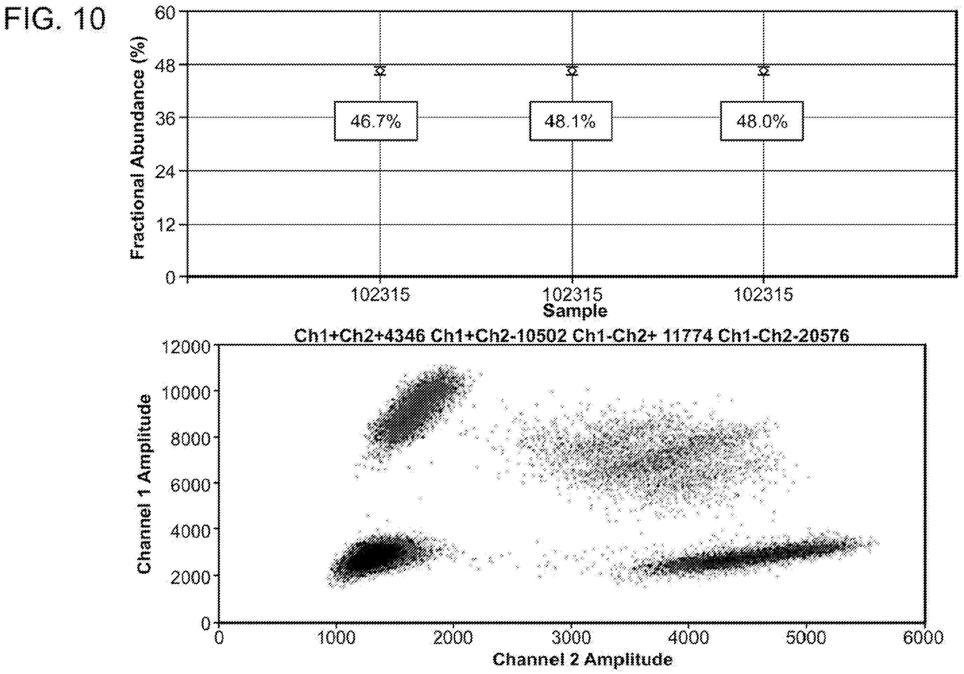

**Figure 10** shows droplet digital PCT results for multiplex cardiomyopathy reference material lot 102315, which comprises a linearized plasmid of the reference material and genomic DNA mixed together at an allelic frequency of 50%, to serve as a heterozygous reference material. PCR correctly identified mutant TNNI3 532_534delAAG at the expected frequency.

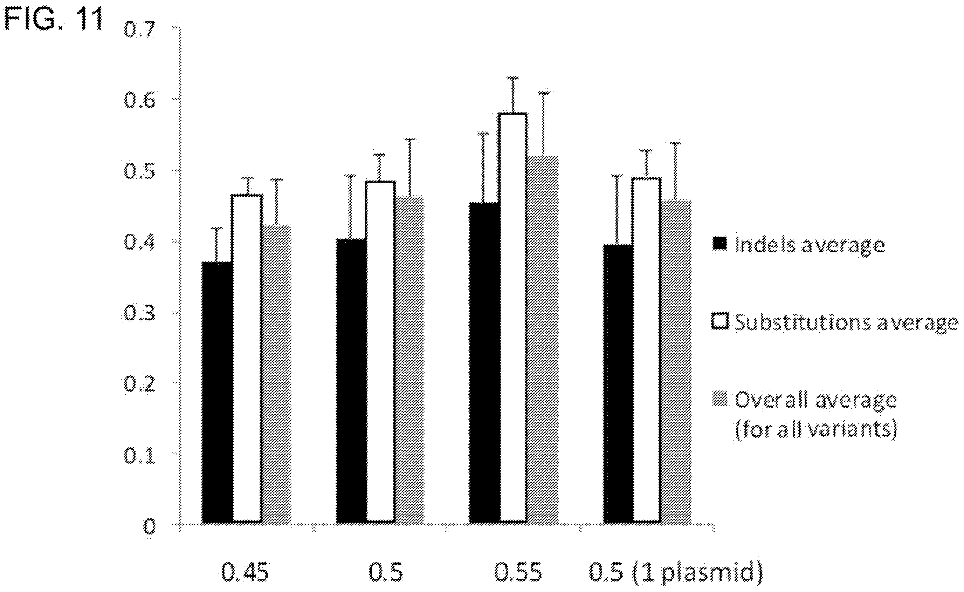

**Figure 11** shows the calculated average allelic frequency of four reference materials as determined by next generation sequencing. From left to right, the bars labeled "0.45" correspond to lot 102304, a mixture of 10 different plasmids and genomic DNA at a 45% allelic frequency; the bars labeled "0.5" correspond to lot 102305, a mixture of 10 different plasmids and genomic DNA at a 50% allelic frequency; the bars labeled "0.55" correspond to lot 102306, a mixture of 10 different plasmids and genomic DNA at a 05% allelic frequency; and the bars labeled "0.5" correspond to lot 102315, a mixture of the multiplex plasmid and genomic DNA at a 50% allelic frequency.

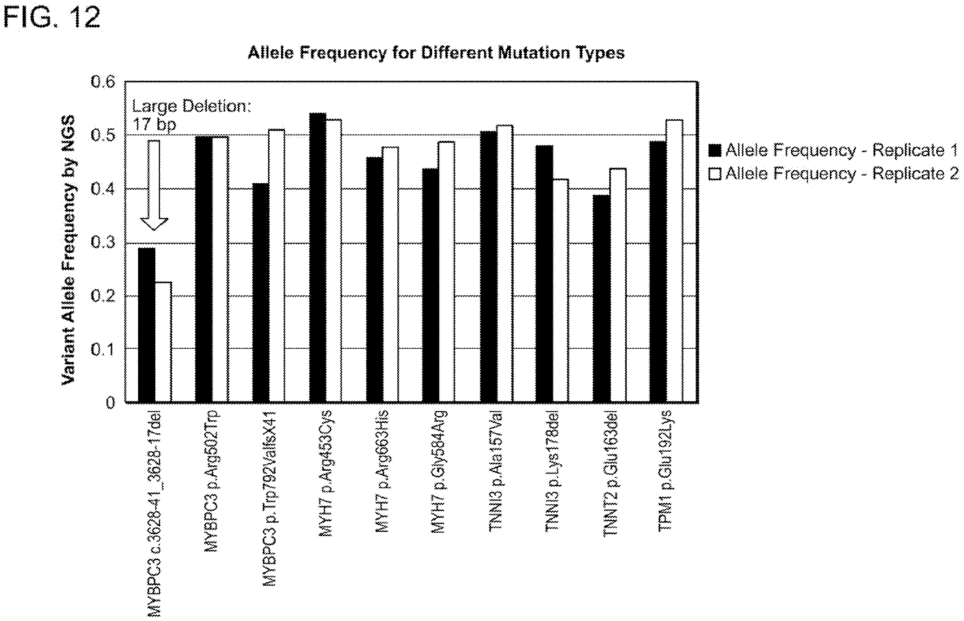

**Figure 12** shows the observed allelic frequency of lot 102315, a mixture of a multiplex plasmid and genomic DNA at a 50% allelic frequency, as determined by next generation sequencing. The 3628-41_3628-17del mutation displayed a considerably lower allele frequency than the other variants.

### DETAILED DESCRIPTION

Aspects provided herein relate to nucleic acids comprising a plurality of nucleotide sequences wherein each nucleotide sequence of the plurality comprises a genotype that is associated with a disease or condition; each nucleotide sequence is 600-10,000 base pairs long; and the plurality of nucleotide sequences comprises sequences from at least 2 different chromosomes. The nucleic acids are suitable for use in producing biological reference materials. A biological reference material may comprisea plurality of ells comprising such a nucleic acid and paraffin, wherein the plurality of cells are fixed and embedded in the paraffin. Alternatively the biological reference material comprises a plurality of cells comprising the nucleic acid and a liquid. A single nucleic acid comprising several different genotypes of interest may be used to transfect a group of cells to generate a reference material comprising each genotype of the nucleic acid. The single nucleic acid format is desirable for many reasons. For example, having a number of genotypes on a single nucleic acid simplifies quantification of the nucleic acid because one nucleic acid needs to be accurately quantified only once. This format also enables "mega" mixes (mixtures of multiple nucleic acids, each bearing multiple different genotypes) allowing hundreds of genotypes to be incorporated into the same control, *e.g*., thereby allowing a biosynthetic control that mimics multiple heterozygous variants. Additionally, nucleic acids comprising a number of different genotypes allows one to transfect quantitatively each genotype into a cell at the same concentration. Advantages for end users include confirmation that genotypes were assessed in a given assay, and confirmation that difficult to sequence genotypes were detected in a sequencing run by using the reference material as a positive control. Finally, multiplexed controls are cheaper than libraries of numerous single-mutant controls.

### I. Nucleic Acids

In some aspects, the disclosure relates to a nucleic acid, comprising a plurality of nucleotide sequences, wherein each nucleotide sequence of the plurality comprises a genotype that is associated with a disease or condition; each nucleotide sequence is 600-10,000 base pairs long; and the plurality of nucleotide sequences comprises nucleotide sequences from at least 2 different chromosomes. The nucleic acid may be DNA or RNA. When the term refers to RNA, each thymine T of a nucleotide sequence may be substituted with uracil U. A nucleic acid as described herein may be referred to as a "full-length nucleic acid" for clarity, *e.g.,* to differentiate a nucleic acid and fragment thereof.

A genotype may be associated with a disease or condition if a subject having the genotype has an increased risk of developing the disease or condition. For example, a BRCA mutation increases the risk that a subject will develop breast cancer. A genotype may be associated with a disease or condition if its presence or absence correlates with the progression or severity of a disease or condition. For example, certain somatic mutations correlate with the aggressiveness of cancer, *e.g., ras* gain-of-function mutations are somatic mutations that correlate with aggressiveness in various neoplasms, including adenocarcinomas, transitional cell carcinomas, neuroblastomas, AML, CML, CMML, JMML, ALL, Burkitt's lymphoma, Hodgkin's lymphoma, plasma cell myeloma, hepatocellular carcinoma, large cell lung carcinoma, non-small cell lung carcinoma, squamous cell carcinoma, lung neoplasia, ductal adenocarcinomas, endocrine tumors, basal cell carcinoma, malignant melanomas, angiosarcoma, leiomyosarcoma, liposarcoma, rhabdomyosarcoma, myxoma, malignant fibrous histiocytoma-pleomorphic sarcoma, germinoma, seminoma, anaplastic carcinoma, follicular carcinoma, papillary carcinoma, and Hurthle cell carcinoma. The disease or condition may be any one of the foregoing neoplasms.

The disease or condition may be an inheritable cancer (*e.g*., such as cancer associated with a BRCA1 or BRCA2 mutation). The disease or condition may be a cancer syndrome, such as hereditary breast-ovarian cancer syndrome or hereditary non-polyposis colon cancer (Lynch syndrome). The disease or condition may be an environmental cancer, *i.e.,* a cancer that is not inherited genetically.

The disease or condition may be cardiomyopathy, hypercholesterolemia, developmental delay, or congenital hearing loss.

The disease or condition may be a genetic disorder, *e.g.*, an autosomal recessive genetic disorder. The disease or condition may be an autosomal dominant, autosomal recessive, X-linked dominant, X-linked recessive, Y-linked, or mitochondrial genetic disorder.

The disease or condition may be a single-gene disorder or a multi-gene disorder.

The disease or condition may be familial hypercholesterolemia, polycystic kidney disease, neurofibromatosis type I, hereditary spherocytosis, Marfan syndrome, or Huntington's disease.

The disease or condition may be sickle cell anemia, cystic fibrosis, Tay-Sachs disease, phenylketonuria, mucopolysaccharidoses, lysosomal acid lipase deficiency, glycogen storage disease, or galactosemia.

The disease or condition may be Duchenne muscular dystrophy or hemophilia.

The disease or condition may be asthma, an autoimmune diseases (*e*.*g*., multiple sclerosis), a ciliopathy, diabetes, heart disease, hypertension, inflammatory bowel disease, intellectual disability, mood disorder, obesity, refractive error, or infertility.

A plurality of nucleotide sequences comprises at least 2 nucleotide sequences, e.g., at least 2 nucleotide sequences that do not overlap on the nucleic acid. A plurality of nucleotide sequences may comprise at least 3, at least 4, at least 5, at least 6, at least 7, at least 8, at least 9, or at least 10 nucleotide sequences. A plurality of nucleotide sequences may comprise 2 to 1000 nucleotide sequences (*e.g*., 2 to 1000 nucleotide sequences that do not overlap). A plurality of nucleotide sequences may comprise 2 to 100 nucleotide sequences, such as 2 to 50, 2 to 20, 2 to 12, 3 to 1000, 3 to 100, 3 to 50, 3 to 20, 3 to 12, 4 to 1000, 4 to 100, 4 to 50, 4 to 20, 4 to 12, 5 to 1000, 5 to 100, 5 to 50, 5 to 20, 5 to 12, 6 to 1000, 6 to 100, 6 to 50, 6 to 20, 6 to 12, 7 to 1000, 7 to 100, 7 to 50, 7 to 20, 7 to 12, 8 to 1000, 8 to 100, 8 to 50, 8 to 20, 8 to 12, 9 to 1000, 9 to 100, 9 to 50, 9 to 20, 9 to 12, 10 to 1000, 10 to 100, 10 to 50, 10 to 20, 10 to 16, or 10 to 12 nucleotide sequences. A plurality of nucleotide sequences may consist of 2, 3, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, or 200 different nucleotide sequences.

In certain embodiments, each nucleotide sequence of a plurality is the nucleotide sequence of a naturally-occurring gene or a subsequence thereof. A naturally-occurring gene includes healthy genotypes and genotypes that are associated with a disease or condition. The term "genotype" refers to a genetic trait, such as allelomorphism, polymorphism, splice variant, regulatory variant, mutation, indel (insertion or deletion mutation), trinucleotide repeat, premature stop codon, translocation, somatic rearrangement, gene fusion, or the presence of a foreign or exogenous nucleotide sequence, such as a virus, provirus, or bacteria. For example, a nucleotide sequence of the plurality may comprise a subsequence of a gene, wherein the subsequence comprises a allelomorphism or a somatic mutation, such as an indel. Each nucleotide sequence of a plurality comprises a genotype, *e.g.,* a mutation or polymorphism. The genotype may be a single nucleotide polymorphism, point mutation, premature stop codon, trinucleotide repeat, translocation, somatic rearrangement, allelomorph, single nucleotide variant, insertion or deletion ("indel"), regulatory variant, or gene fusion. A nucleotide sequence of a plurality may comprise a healthy genotype at a position in which mutations are known to occur. A nucleotide sequence of a plurality may comprise an exon of a gene or a subsequence of an exon. A genotype may thereby either cause the mutation, deletion, and/or insertion of at least one amino acid of the polypeptide or protein encoded by the gene (or exon) or cause a truncation or addition to the polypeptide or protein encoded by the gene (or exon), *e.g.,* if the genotype inserts or deletes a stop codon, respectively. A nucleotide sequence of a plurality may consist of a nucleotide sequence of a gene or a subsequence thereof. For example, a nucleotide sequence of a plurality may consist of an exon of a gene or a subsequence of an exon.

A nucleotide sequence of a plurality of nucleotide sequences may be a subsequence of a gene *(e.g.,* a human gene) or regulatory region thereof.

In certain embodiments, each nucleotide sequence of a plurality is sufficiently long to be identified by nucleic acid sequencing, *e.g.*, next generation sequencing (NGS). In certain embodiments, a nucleotide sequence of a plurality comprises a genotype of interest at a position that can be identified by nucleic acid sequencing. For example, the genotype of interest, such as a mutation, may be positioned at or near the middle of a nucleotide sequence.

A nucleic acid may be about 1000 nucleotides to about 100,000 nucleotides long, such as about 3000 to about 60,000 nucleotides long, about 5000 to about 50,000 nucleotides long, or about 8000 to about 20,000 nucleotides long.

A nucleotide sequence of a plurality is at least 600 nucleotides (or base pairs) long. A nucleotide sequence of a plurality may be 600 to 10,000 nucleotides (or base pairs) long, such as 600 to 5000, 600 to 2000, 600 to 1000, 600 to 2000, 600 to 3000, or 600 to 1200 nucleotides (or base pairs) long. Accordingly, a subsequence of a gene (and/or regulatory region thereof) may be 600 to 10,000 nucleotides (or base pairs) long, such as 600 to 5000, 600 to 2000, 600 to 1000, 600 to 3000, or 600 to 1200 nucleotides (or base pairs) long.

A nucleotide sequence of a plurality may comprise a genotype of interest at a position that is at least 20 nucleotides (or base pairs) from the 5' end and/or 3' end of the nucleotide sequence, such as at least 25, 30, 40, 50, 60, 70, 80, 90, 100, 120, 150, 200, or 250 nucleotides (or base pairs) from the 5' and/or 3' end of the nucleotide sequence. A nucleotide sequence of a plurality may comprise a genotype of interest at a position that is 20 to 5000 nucleotides (or base pairs) from the 5' end and/or 3' end of the nucleotide sequence, such as 25 to 5000, 30 to 5000, 40 to 5000, 50 to 5000, 60 to 5000, 70 to 5000, 80 to 5000, 90 to 5000, 100 to 5000, 120 to 5000, 150 to 5000, 200 to 5000, 250 to 5000, 25 to 2000, 30 to 2000, 40 to 2000, 50 to 2000, 60 to 2000, 70 to 2000, 80 to 2000, 90 to 2000, 100 to 2000, 120 to 2000, 150 to 2000, 200 to 2000, 250 to 2000, 25 to 1000, 30 to 1000, 40 to 1000, 50 to 1000, 60 to 1000, 70 to 1000, 80 to 1000, 90 to 1000, 100 to 1000, 120 to 1000, 150 to 1000, 200 to 1000, 250 to 1000, 25 to 750, 30 to 750, 40 to 750, 50 to 750, 60 to 750, 70 to 750, 80 to 750, 90 to 750, 100 to 750, 120 to 750, 150 to 750, 200 to 750, 250 to 750, 25 to 500, 30 to 500, 40 to 500, 50 to 500, 60 to 500, 70 to 500, 80 to 500, 90 to 500, 100 to 500, 120 to 500, 150 to 500, 200 to 500, or 250 to 500 nucleotides from the 5' and/or 3' end of the nucleotide sequence.

In some embodiments, a nucleic acid may comprise poly-adenosine, e.g., a 3' poly-adenosine tail (poly-A tail). DNA or RNA may comprise poly-adenosine. If DNA comprises poly-adenosine, the DNA may be double-stranded, such that a complementary poly-thymidine sequence is transcribed into mRNA comprising a poly-adenosine tail.

A nucleic acid may be methylated or substantially free of methylated nucleotides.

In some embodiments, the nucleic acid comprises an origin of replication. The origin of replication may allow for cloning and/or batch-production of the nucleic acid. The origin of replication may be an origin of replication from yeast (*e.g., Saccharomyces cerevisiae*) or bacteria (*e.g., Escherichia coli*)*, e.g.,* such that the nucleic acid may be cloned and/or produced in yeast (*e.g., Saccharomyces cerevisiae*) or bacteria (*e.g., Escherichia coli*).

In some embodiments, the nucleic acid comprises a promoter, *e.g.*, when the nucleic acid is DNA. A promoter binds to an RNA polymerase, such as SP6 RNA polymerase. A promoter may be a SP6 promoter. The nucleotide sequence of a promoter may be of a different species *(e.g.,* virus, bacteria, yeast) than a nucleotide sequence of a plurality, *e.g.,* for *in vitro* transcription of the plurality of nucleotide sequences, which may be human nucleotide sequences, or nucleotide sequences of human pathogens. The nucleotide sequence of a promoter may be of a different species (*e.g.*, virus, bacteria, yeast) than each nucleotide sequence of a plurality.

In some embodiments, the nucleic acid is a plasmid, such as a supercoiled plasmid, relaxed circular plasmid, or linear plasmid.

Also described herein is a plurality of nucleic acid fragments, wherein each nucleic acid of the plurality of nucleic acid fragments is a fragment of a full-length nucleic acid as described herein, *supra,* and each nucleotide sequence of the plurality of nucleotide sequences of the full-length nucleic acid is encoded by at least one nucleic acid fragment of the plurality of nucleic acid fragments. A plurality of nucleic acid fragments may be obtained, for example, by processing multiple copies of a single, full-length DNA nucleic acid comprising a plurality of nucleotide sequences, *e.g.*, by transfecting cells with the single, full-length DNA nucleic acid *(e.g.,* by electroporation), fixing the cells *(e.g.,* with formalin), embedding the cells *(e.g.,* in paraffin), and/or extracting nucleic acids *(e.g.,* DNA) from the cells. The processing of a multiple copies of a single, full-length DNA nucleic acid corresponding to one of the nucleic acids described herein, *supra,* may degrade the single, full-length DNA nucleic acid into smaller DNA fragments, *e.g.,* a plurality of nucleic acid fragments. This plurality of nucleic acid fragments may comprise the same plurality of nucleotide sequences as the single DNA nucleic acid, but any given nucleotide sequence of the plurality of nucleotide sequences may occur on different nucleic acid fragments of the plurality of nucleic acid fragments rather than on the same nucleic acid fragment. Next generation sequencing may be used to identify nucleotide sequences that occur across two or more nucleic acid fragments of a plurality of nucleic acid fragments. Thus, the sequencing of a plurality of nucleic acid fragments should identify the same plurality of nucleotide sequences as the sequencing of the single, full-length DNA nucleic acid from which the plurality of nucleic acid fragments originated. A plurality of nucleic acid fragments may be admixed with cellular nucleic acids *(e.g.,* RNA and/or DNA) from cells transfected with the single, full-length DNA nucleic acid and/or untransfected cells (*e.g*., untransfected cells added to a reference material, *see infra*). Thus, a plurality of nucleic acid fragments may be admixed with cellular DNA, *e.g.,* genomic DNA.

Also described herein is a method for making a nucleic acid as described herein *(e.g.,* at a temperature at which the DNA polymerase displays polymerase activity). The method may comprise incubating a reaction mixture comprising a DNA template, DNA polymerase, and ribonucleotide triphosphates, thereby making an DNA nucleic acid. The DNA template may also be a nucleic acid as described herein.

Also described herein is a reaction mixture comprising a nucleic acid as described herein, a polymerase, and either ribonucleotide triphosphates or deoxyribonucleotide triphosphates (dNTPs). The polymerase may be a DNA polymerase (*e.g*., for use with deoxyribonucleotide triphosphates) or an RNA polymerase (*e.g*., for use with ribonucleotide triphosphates). The polymerase may be from a different species than a nucleotide sequence of a plurality. The reaction mixture may comprise an DNAse inhibitor, *e.g.,* from a different species than a nucleotide sequence of a plurality.

A nucleic acid may comprise nucleotide sequences of any origin, such as viral, bacterial, protist, fungal, plant, or animal origin. In certain embodiments, the nucleotide sequences of a plurality are human nucleotide sequences. The nucleotide sequences of a plurality may also comprise nucleotide sequences from human pathogens, *e.g.,* a nucleic acid may comprise viral, bacterial, protist, or fungal nucleotide sequences, wherein the virus, bacterium, protist, or fungus is a human pathogen.

In some aspects, the disclosure relates to a composition comprising a nucleic acid as described herein and genomic DNA. In certain embodiments, the ratio of (a) the copy number of a nucleotide sequence corresponding to a gene in the nucleic acid relative to (b) the copy number of the gene in the genomic DNA is about 1:15,000 to about 500:1 in the composition, such as about 1:10,000 to about 500:1, about 1:5,000 to about 500:1, about 1:2,000 to about 500:1, about 1:1,000 to about 500:1, about 1:500 to about 500:1, 1:5,000 to about 100:1, about 1:2,000 to about 100:1, about 1:1,000 to about 100:1, about 1:500 to about 100:1, about 1:250 to about 100:1, about 1:200 to about 100:1, about 1:100 to about 100:1, about 1:50 to about 50:1, about 1:25 to about 25:1, about 1:20 to about 20:1, or about 1:10 to about 10:1 in the composition. In certain embodiments, the ratio of (a) the copy number of a nucleotide sequence corresponding to a gene in the nucleic acid relative to (b) the copy number of the gene in the genomic DNA is about 1:100 to about 2:1, about 1:50 to about 1:1, or about 1:30 to about 1:2. In certain embodiments, the ratio of (a) the copy number of a nucleotide sequence corresponding to a gene in the nucleic acid relative to (b) the copy number of the gene in the genomic DNA is about 1:25, about 1:20, about 1:15, about 1:10, or about 1:5.

In certain embodiments, the ratio of the copy number of the nucleic acid to the copy number of the genomic DNA in the composition is about 1:15,000 to about 500:1 in the composition, such as about 1:10,000 to about 500:1, about 1:5,000 to about 500:1, about 1:2,000 to about 500:1, about 1:1,000 to about 500:1, about 1:500 to about 500:1, 1:5,000 to about 100:1, about 1:2,000 to about 100:1, about 1:1,000 to about 100:1, about 1:500 to about 100:1, about 1:250 to about 100:1, about 1:200 to about 100:1, about 1:100 to about 100:1, about 1:50 to about 50:1, about 1:25 to about 25:1, about 1:20 to about 20:1, or about 1:10 to about 10:1 in the composition. In certain embodiments, the ratio of the copy number of the nucleic acid to the copy number of the genomic DNA in the composition is about 1:100 to about 2:1, about 1:50 to about 1:1, or about 1:30 to about 1:2. In certain embodiments, the ratio of the copy number of the nucleic acid to the copy number of the genomic DNA in the composition is about 1:25, about 1:20, about 1:15, about 1:10, or about 1:5.

A composition may comprise at least two nucleic acids as described herein, *e.g.,* wherein at least two of the nucleic acids comprise different pluralities of nucleotide sequences. For example, a composition may comprise a plurality of nucleic acids as described herein, wherein 2 to 50, 2 to 40, 2 to 30, 2 to 20, 2 to 10, 2 to 9, 2 to 8, 2 to 7, 2 to 6, 2 to 5, or 2 to 4 nucleic acids of the plurality each comprise different pluralities of nucleotide sequences.

A nucleic acid comprises nucleotide sequences from different genes. At least 2, 3, 4, 5, 6, 7, 8, 9, or 10 nucleotide sequences of a plurality of nucleotides sequences may be from different human genes. A nucleic acid comprises nucleotide sequences from genes that occur on different genes. A plurality of nucleotides sequences may comprise nucleotide sequences from genes that occur on 2, 3, 4, 5, 6, 7, 8, 9, or 10 different human genes.

A nucleic acid comprises nucleotide sequences from at least two different chromosomes. At least 2, 3, 4, 5, 6, 7, 8, 9, or 10 nucleotide sequences of a plurality of nucleotides sequences may be from at least two different human chromosomes. A nucleic acid comprises nucleotide sequences from genes that occur on at least two different chromosomes. A plurality of nucleotides sequences may comprise nucleotide sequences from genes that occur on 2, 3, 4, 5, 6, 7, 8, 9, or 10 different human chromosomes.

### II. EMBODIMENTS RELATED TO CANCER

The disease or condition may be, for example, a neoplasm, such as cancer. Neoplasms include lung cancer, lymphoid cancer, acute lymphoid leukemia, acute myeloid leukemia, chronic myelogenous leukemia, Burkitt's lymphoma, Hodgkin's lymphoma, plasma cell myeloma, biliary tract cancer, bladder cancer, liver cancer, pancreatic cancer, prostate cancer, skin cancer, thyroid cancer, stomach cancer, large intestine cancer, colon cancer, urinary tract cancer, central nervous system cancer, neuroblastoma, kidney cancer, breast cancer, cervical cancer, testicular cancer, and soft tissue cancer. The disease or condition may be adenocarcinoma, transitional cell carcinoma, breast carcinoma, cervical adenocarcinoma, colon adenocarcinoma, colon adenoma, neuroblastoma, AML, CML, CMML, JMML, ALL, Burkitt's lymphoma, Hodgkin's lymphoma, plasma cell myeloma, hepatocellular carcinoma, large cell lung carcinoma, non-small cell lung carcinoma, squamous cell lung carcinoma, lung neoplasia, ductal adenocarcinoma, endocrine tumor, prostate adenocarcinoma, basal cell skin carcinoma, squamous cell skin carcinoma, melanoma, malignant melanoma, angiosarcoma, leiomyosarcoma, liposarcoma, rhabdomyosarcoma, myxoma, malignant fibrous histiocytoma-pleomorphic sarcoma, stomach adenocarcinoma, germinoma, seminoma, anaplastic carcinoma, follicular carcinoma, papillary carcinoma, or Hurthle cell carcinoma. A nucleotide sequence of a plurality of nucleotide sequences may be associated with a solid tumor. Each nucleotide sequence of a plurality of nucleotide sequences may be associated with a solid tumor.

In some embodiments, a nucleotide sequence of the plurality comprises a subsequence of a gene selected from the group consisting of ABL1, ALK, BCR, CBFB, FGFR1, JAK2, KMT2A, MECOM, MKL1, NOTCH1, NUP214, PDGFRA, PDGFRB, PICALM, RARA, RUNX1, RUNX1T1, TAL1, and TCF3. In some embodiments, each nucleotide sequence of the plurality comprises a subsequence of a gene selected from the group consisting of ABL1, ALK, BCR, CBFB, FGFR1, JAK2, KMT2A, MECOM, MKL1, NOTCH1, NUP214, PDGFRA, PDGFRB, PICALM, RARA, RUNX1, RUNX1T1, TAL1, and TCF3.

In some embodiments, a nucleotide sequence of the plurality comprises a subsequence of a gene selected from the group consisting of MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPN11, FLT3, RB1, PARP2, ARHGAP5, AKT1, RAD51, IDH2, TP53, NF1, SMAD4, AKT2, ERCC1, and GNAS. In some embodiments, each nucleotide sequence of the plurality comprises a subsequence of a gene selected from the group consisting of MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPN11, FLT3, RB1, PARP2, ARHGAP5, AKT1, RAD51, IDH2, TP53, NF1, SMAD4, AKT2, ERCC1, and GNAS.

In some embodiments, a nucleotide sequence of the plurality comprises a subsequence of a gene selected from the group consisting of AKT1, ATM, BRAF, CDKN2A, CSF1R, EGFR, ERBB2 ("HER2"), ERBB4 ("HER4"), FGFR1, FGFR2, FGFR3, GNA11, HRAS, JAK2, JAK3, KDR, KIT, KRAS, MET, NOTCH1, NRAS, PDGFRA, PIK3CA, PTEN, RET, and STK11. In some embodiments, each nucleotide sequence of the plurality comprises a subsequence of a gene selected from the group consisting of AKT1, ATM, BRAF, CDKN2A, CSF1R, EGFR, ERBB2 ("HER2"), ERBB4 ("HER4"), FGFR1, FGFR2, FGFR3, GNA11, HRAS, JAK2, JAK3, KDR, KIT, KRAS, MET, NOTCH1, NRAS, PDGFRA, PIK3CA, PTEN, RET, and STK11.

In some embodiments, a nucleotide sequence of the plurality comprises a subsequence of a gene selected from the group consisting of ABL1, AKT1, ALK, APC, AR, AR1D1A, ARAF, ATM, BCL2, BCR, BRAF, BRC42, BRCA1, BRCA2, BRIP1, CCND1, CCND2, CCNE1, CDH1, CDK4, CDK6, CDKN2A, CDKN2B, CSF1R, CTNNB1, DDR2, EGFR, ERBB2, ERBB3, ERBB4, ESR1, ETV1, ETV4, ETV6, EWSR1, EZH2, FANCA, FANCC, FANCD2, FANCE, FANCF, FANCG, FANCL, FBXW7, FGFR1, FGFR2, FGFR3, FLT3, FOXL2, GATA3, GNA11, GNAQ, GNAS, HER/ERBB2, HNF1A, HPAS, HRAS, IDH1, IDH2, IHD2, JAK2, JAK3, KDR, KIT, KRAS, MAP2K1, MAP2K2, MET, MLH1, MLL, MPL, MSH2, MSH6, MTOR, MYC, MYCN, NF1, NF2, NFE2L2, NOTCH1, NPM1, NRAS, NTRK1, PALB2, PDGFRA, PDGFRB, PIK3CA, PMS2, PTCH1, PTEN, PTPN11, RAFT, RARA, RB1, RET, RHEB, RHOA, RIT1, ROS1, SMAD4, SMARCB1, SMO, SRC, STK11, TERT, TMPRSS2, TP53, TSC1, TSC2, and VHL. In some embodiments, each nucleotide sequence of the plurality comprises a subsequence of a gene selected from the group consisting of ABL1, AKT1, ALK, APC, AR, AR1D1A, ARAF, ATM, BCL2, BCR, BRAF, BRC42, BRCA1, BRCA2, BRIP1, CCND1, CCND2, CCNE1, CDH1, CDK4, CDK6, CDKN2A, CDKN2B, CSF1R, CTNNB1, DDR2, EGFR, ERBB2, ERBB3, ERBB4, ESR1, ETV1, ETV4, ETV6, EWSR1, EZH2, FANCA, FANCC, FANCD2, FANCE, FANCF, FANCG, FANCL, FBXW7, FGFR1, FGFR2, FGFR3, FLT3, FOXL2, GATA3, GNA11, GNAQ, GNAS, HER/ERBB2, HNF1A, HPAS, HRAS, IDH1, IDH2, IHD2, JAK2, JAK3, KDR, KIT, KRAS, MAP2K1, MAP2K2, MET, MLH1, MLL, MPL, MSH2, MSH6, MTOR, MYC, MYCN, NF1, NF2, NFE2L2, NOTCH1, NPM1, NRAS, NTRK1, PALB2, PDGFRA, PDGFRB, PIK3CA, PMS2, PTCH1, PTEN, PTPN11, RAFT, RARA, RB1, RET, RHEB, RHOA, RIT1, ROS1, SMAD4, SMARCB1, SMO, SRC, STK11, TERT, TMPRSS2, TP53, TSC1, TSC2, and VHL.

In some embodiments, a nucleotide sequence of the plurality comprises a subsequence of a gene selected from the group consisting of BRAF, EGFR, ERBB2, and KRAS. In some embodiments, each nucleotide sequence of the plurality comprises a subsequence of a gene selected from the group consisting of BRAF, EGFR, ERBB2, and KRAS.

Each subsequence may comprise a mutation that is associated with the disease or condition or a healthy genotype at a position wherein such mutations are known to occur.

In some embodiments, a nucleotide sequence of the plurality comprises a subsequence of a gene selected from the group consisting of BRAF, EGFR, ERBB2, and KRAS, and the subsequence comprises a mutation selected from the group consisting of mutation V600E to gene BRAF, mutation T790M to gene EGFR, mutation delL747-P753insS to gene EGFR, mutation A775_G776insYVMA to gene ERBB2, and mutation G12D to gene KRAS. In some embodiments, each nucleotide sequence of the plurality comprises a subsequence of a gene selected from the group consisting of BRAF, EGFR, ERBB2, and KRAS, and each subsequence comprises a mutation selected from the group consisting of mutation V600E to gene BRAF, mutation T790M to gene EGFR, mutation delL747-P753insS to gene EGFR, mutation A775_G776insYVMA to gene ERBB2, and mutation G12D to gene KRAS.

A number of different germline and somatic mutations correlate with cancer, and a genotype of a nucleotide sequence may be selected from such mutations.

The COSMIC (Catalogue of Somatic Mutations in Cancer) database may be used to identify genes and genotypes (*e.g.*, variants) that are associated with neoplasms, which may be used to select a nucleotide sequence of a plurality of nucleotide sequences (http://cancer.sanger.ac.uk/cosmic). A nucleotide sequence of a plurality of nucleotide sequences may be a subsequence of a gene selected from any gene in the COSMIC database, *e.g.,* wherein the subsequence comprises a genotype *(e.g.,* variant, such as a mutation) that is associated with cancer. In some embodiments, at least 1, 2, 3, 4, 5, 6, 7, 8, 9, or 10 nucleotide sequences of a plurality of nucleotide sequences may be subsequences of genes selected from the genes in the COSMIC database. Each nucleotide sequence of a plurality of nucleotide sequences may be a subsequence of a gene selected from any gene in the COSMIC database.

A nucleotide sequence of a plurality of nucleotide sequences may be a subsequence of a nucleotide sequence in the COSMIC database, *e.g.,* wherein the subsequence comprises a genotype that is associated with cancer. At least 1, 2, 3, 4, 5, 6, 7, 8, 9, or 10 nucleotide sequences of a plurality of nucleotide sequences may be subsequences of the nucleotide sequences in the COSMIC database. Each nucleotide sequence of a plurality of nucleotide sequences may be a subsequence of a nucleotide sequence in the COSMIC database.

A nucleotide sequence of a plurality of nucleotide sequences may be a subsequence of a gene selected from ABI1, ABL1, ABL2, ACSL3, ACUR1, AF15Q14, AF1Q, AF3P21, AF5Q31, AKAP9, AKT1, AKT2, AKT3, AL017, ALDH2, ALK, AMER1, APC, APEX1, AR, AR1D1A, ARAF, ARHGAP5, ARHGEF12, ARHH, ARID1A, ARID2, ARNT, ASPSCR1, ASXL1, ATF1, ATIC, ATM, ATP11B, ATP1A1, ATP2B3, ATR, ATRX, AXIN1, BAP1, BCL10, BCL11A, BCL11B, BCL2, BCL2L1, BCL3, BCL5, BCL6, BCL7A, BCL9, BCOR, BCR, BHD, BIRC2, BIRC3, BLM, BMPR1A, BRAF, BRC42, BRCA1, BRCA2, BRD3, BRD4, BRIP1, BTG1, BUB1B, C12ORF9, C15ORF21, C15ORF55, C16ORF75, C2ORF44, CACNA1D, CALR, CAMTA1, CANT1, CARD11, CARS, CASP8, CBFA2T1, CBFA2T3, CBFB, CBL, CBLB, CBLC, CCDC6, CCNB1IP1, CCND1, CCND2, CCND3, CCNE1, CD273, CD274, CD44, CD74, CD79A, CD79B, CDC73, CDH1, CDH11, CDK12, CDK4, CDK6, CDKN2A, CDKN2A(PL4), CDKN2B, CDKN2C, CDX2, CEBPA, CEP1, CEP89, CHCHD7, CHEK2, CHIC2, CHN1, CIC, CIITA, CLIP1, CLTC, CLTCL1, CMKOR1, CNOT3, COL1A1, COL2A1, COPEB, COX6C, CREB1, CREB3L1, CREB3L2, CREBBP, CRLF2, CRTC3, CSF1R, CSF3R, CSNK2A1, CTNNB1, CUX1, CYLD, D10S170, DAXX, DCTN1, DDB2, DDIT3, DDR2, DDX10, DDX5, DDX6, DEK, DICERL, DNM2, DNMT3A, DLTX4, EBFL, ECT2L, EGFR, EIF3E, EIF4A2, ELF4, ELK4, ELKS, ELL, ELN, EML4, EP300, EPS 15, ERBB2, ERBB2 ("HER2"), ERBB3, ERBB4 ("HER4"), ERC1, ERCC1, ERCC2, ERCC3, ERCC4, ERCC5, ERG, ESR1, ETV1, ETV4, ETV5, ETV6, EVI1, EWSR1, EXT1, EXT2, EZH2, EZR, FACL6, FAM46C, FANCA, FANCC, FANCD2, FANCE, FANCF, FANCG, FANCL, FAS, FBXOI1, FBXW7, FCGR2B, FEV, FGFR1, FGFR10P, FGFR2, FGFR3, FGFR4, FH, FHIT, FIP1L1, FLJ27352, FLT3, FLU, FNBP1, FOX03A, FOX04, FOXA1, FOXL2, FOXOIA, FOXP1, FSTL3, FUBP1, FUS, FVT1, GABRA6, GABRG2, GAS6, GAS7, GATA1, GATA2, GATA3, GMPS, GNA11, GNAQ, GNAS, GOLGA5, GOPC, GPC3, GPHN, GRAF, H3F3A, H3F3B, HCMOGT-1, HEAB, HERPUD1, HEY1, HIP1, HIST1H3B, HIST1H4L, HLA-A, HLF, HLXB9, HMGA1, HMGA2, HNF1A, HNRNPA2B1, HOOK3, HOXA11, HOXA13, HOXA9, HOXC11, HOXC13, HOXD11, HOXD13, HPAS, HRAS, HSPCA, HSPCB, IDH1, IDH2, IGF1R, IGH, IGK, IGL, IHD2, IKZF1, IL2, IL21R, IL6, IL6ST, IL7R, IRF4, IRTA1, ITK, JAK1, JAK2, JAK3, JAZF1, JUN, KCNJ5, KDM5A, KDM5C, KDM6A, KDR, KIAA1549, KIAA1598, KIF5B, KIT, KLF4, KLK2, KMT2A, KMT2D, KRAS, KTN1, LAF4, LASP1, LCK, LCP1, LCX, LHFP, LIFR, LM02, LMNA, LMOL, LPP, LRIG3, LSM14A, LYL1, MAF, MAFB, MALAT1, MALT1, MAML2, MAP2K1, MAP2K2, MAP2K4, MAX, MCL1, MDM2, MDM4, MDS1, MDS2, MECOM, MECT1, MED 12, MEN1, MET, MITF, MKL1, MLF1, MLH1, MLL, MLL3, MLLT1, MLLT10, MLLT2, MLLT3, MLLT4, MLLT6, MLLT7, MN1, MPL, MSF, MSH2, MSH6, MSI2, MSN, MTCP1, MTOR, MUC1, MUTYH, MY018A, MY05A, MYB, MYC, MYC1, MYCL1, MYCN, MYD88, MYH11, MYH9, MYST4, NAB2, NACA, NBSL, NCOA1, NCOA2, NCOA4, NDRG1, NF1, NF2, NFATC2, NFE2L2, NFIB, NFKB2, NIN, NKX2- 1, NKX2-1, NKX2-8, NONO, NOTCH1, NOTCH2, NPM1, NR4A3, NRAS, NRAS/CSDE1, NRG1, NSD1, NT5C2, NTRK1, NTRK3, NUMA1, NUP214, NUP98, NUTM2A, NUTM2B, OLIG2, OMD, P2RY8, PAFAH1B2, PALB2, PARP1, PARP2, PAX3, PAX5, PAX7, PAX8, PBRM1, PBX1, PCM1, PCSK7, PDCD1LG2, PDE4DIP, PDGFB, PDGFRA, PDGFRB, PERI, PHF6, PHOX2B, PICALM, PIK3CA, PIK3R1, PIM1, PLAG1, PLCG1, PML, PMS1, PMS2, PMX1, PNP, PNUTL1, POT1, POU2AF1, POU5F1, PPARG, PPFIBP1, PPP2R1A, PRCC, PRDM1, PRDM16, PRF1, PRKAR1A, PSIP1, PTCH1, PTEN, PTPN11, PTPRB, PTPRC, PTPRK, PWWP2A, RAB5EP, RAC1, RAD21, RAD51, RAD51L1, RAF1, RAFT, RALGDS, RANBP17, RAP1GDS1, RARA, RB1, RBI, RBM15, RECQL4, REL, RET, RHEB, RHOA, RIT1, RNF43, ROS1, RPL10, RPL22, RPL5, RPN1, RPS6KB1, RSP02, RSP03, RUNDC2A, RUNX1, RUNX1T1, RUNXBP2, SBDS, SDC4, SDH5, SDHB, SDHC, SDHD, SET, SETBP1, SETD2, SF3B1, SFPQ, SFRS3, SH2B3, SH3GL1, SIL, SLC34A2, SLC45A3, SMAD4, SMARCA4, SMARCB1, SMARCE1, SMO, SOCS1, SOX2, SRC, SRGAP3, SRSF2, SS18, SS18L1, SSX1, SSX2, SSX4, STAG2, STAT3, STAT5B, STAT6, STK11, STL, SUFU, SUZ12, SYK, TAF15, TAL1, TAL2, TALI, TBL1XR1, TCEA1, TCF1, TCF12, TCF3, TCF7L2, TCL1A, TCL6, TERT, TET2, TFE3, TFEB, TFG, TFPT, TFRC, THRAP3, TIAF1, TIF1, TLX1, TLX3, TMPRSS2, TNFAIP3, TNFRSF14, TNFRSF17, TOPI, TP53, TPM3, TPM4, TPR, TRA, TRAF7, TRB, TRD, TRIM27, TRIM33, TRIP11, TRRAP, TSC1, TSC2, TSHR, TTL, U2AF1, UBR5, USP6, VHL, VT11A, WAS, WHSC1, WHSC1L1, WIF1, WRN, WT1, WWTR1, XPA, XPC, XPO1, YWHAE, ZCCHC8, ZNF145, ZNF198, ZNF217, ZNF278, ZNF331, ZNF384, ZNF521, ZNF9, and ZRSR2, and/or the respective regulatory regions of any one of the foregoing. At least 1, 2, 3, 4, 5, 6, 7, 8, 9, 10, 11, 12, 13, 14, 15, 16, 17, 18, 19, 20, 1, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotide sequences of a plurality of nucleotide sequences may be subsequences of any of the foregoing genes and/or the respective regulatory regions thereof. Each nucleotide sequence of a plurality of nucleotide sequences may be a subsequence of a gene selected from any one of the foregoing genes and/or their respective regulatory regions. Each of the genes described in this paragraph correspond to a "gene name" listed in the COSMIC database (http://cancer.sanger.ac.uk/cosmic). The COSMIC database may therefore be used to identify synonyms of the gene name, the sequence of a gene, and variants (*e.g*., genotypes) that may be used to select a nucleotide sequence of a plurality of nucleotide sequences.

A nucleotide sequence of a plurality of nucleotide sequences may be a subsequence of a gene selected from the group consisting of MSH2 (mutS homolog 2), MSH6 (mutS homolog 6), MI,H1 (mutL homolog 1), PMS2 (PMS1 homolog 2, mismatch repair system component), CDKN2A (cyclin dependent kinase inhibitor 2A), BRCA2 (breast cancer 2), and BRCA1 (breast cancer 1), and/or the respective regulatory regions of any one of the foregoing. At least 1, 2, 3, 4, 5, 6, 7, 8, 9, or 10 nucleotide sequences of a plurality of nucleotide sequences may be subsequences of genes selected from the group consisting of MSH2, MSH6, MLH1, PMS2, CDKN2A, BRCA2, and BRCA1, and/or the respective regulatory regions of any of the foregoing. Each nucleotide sequence of a plurality of nucleotide sequences may comprise a subsequence of a gene selected from the group consisting of MSH2, MSH6, MLH1, PMS2, CDKN2A, BRCA2, and BRCA1, and/or the respective regulatory regions of any of the foregoing.

A nucleotide sequence of a plurality of nucleotide sequences may be a subsequence of a gene selected from the group consisting of BMPR1A (bone morphogenetic protein receptor, type IA), BRCA1 (breast cancer 1), BRCA2 (breast cancer 2), CDH1 (cadherin-1), CDKN2A (cyclin dependent kinase inhibitor 2A), EPCAM (epithelial cell adhesion molecule), MI,H1 (mutL homolog 1), MSH2 (mutS homolog 2), MSH6 (mutS homolog 6), MUTYH (mutY DNA glycosylase), PALB2 (partner and localizer of BRCA2), PMS2 (PMS1 homolog 2, mismatch repair system component), PTEN (phosphatase and tensin homolog), SMAD4 (SMAD family member 4; mothers against decapentaplegic homolog 4), STK11 (serine/threonine kinase 11), and TP53 (tumor protein p53), and/or the respective regulatory regions of any one of the foregoing. At least 1, 2, 3, 4, 5, 6, 7, 8, 9, or 10 nucleotide sequences of a plurality of nucleotide sequences may be subsequences of genes selected from the group consisting of BMPR1A, BRCA1, BRCA2, CDH1, CDKN2A, EPCAM, MLH1, MSH2, MSH6, MUTYH, PALB2, PMS2, PTEN, SMAD4, STK11, and TP53, and/or the respective regulatory regions of any of the foregoing. Each nucleotide sequence of a plurality of nucleotide sequences may comprise a subsequence of a gene selected from the group consisting of BMPR1A, BRCA1, BRCA2, CDH1, CDKN2A, EPCAM, MLH1, MSH2, MSH6, MUTYH, PALB2, PMS2, PTEN, SMAD4, STK11, and TP53, and/or the respective regulatory regions of any of the foregoing.

A nucleotide sequence of a plurality of nucleotide sequences may be a subsequence of a gene selected from the group consisting of AKT1, AKT2, AKT3, APC, ARHGAP5, ATM, ATR, BRAF, CTNNB1, DNMT3A, EGFR, ERBB2, ERCC1, EZH2, FBXW7, FGFR3, FLT3, GABRA6, GABRG2, GNAQ, GNAS, IDH1, IDH2, JAK2, KIT, KRAS, MET, MLH1, MPL, MSH2, MTOR, MYD88, NF1, NPM1, NRAS/CSDE1, PARP1, PARP2, PDGFRA, PIK3CA, PTEN, PTPN11, RAD51, RB1, RET, SMAD4, TP53, and VHL, and/or the respective regulatory regions of any one of the foregoing. At least 1, 2, 3, 4, 5, 6, 7, 8, 9, or 10 nucleotide sequences of a plurality of nucleotide sequences may be subsequences of genes selected from the group consisting of AKT1, AKT2, AKT3, APC, ARHGAP5, ATM, ATR, BRAF, CTNNB1, DNMT3A, EGFR, ERBB2, ERCC1, EZH2, FBXW7, FGFR3, FLT3, GABRA6, GABRG2, GNAQ, GNAS, IDH1, IDH2, JAK2, KIT, KRAS, MET, MLH1, MPL, MSH2, MTOR, MYD88, NF1, NPM1, NRAS/CSDE1, PARP1, PARP2, PDGFRA, PIK3CA, PTEN, PTPN11, RAD51, RB1, RET, SMAD4, TP53, and VHL, and/or the respective regulatory regions of any of the foregoing. Each nucleotide sequence of a plurality of nucleotide sequences may comprise a subsequence of a gene selected from the group consisting of AKT1, AKT2, AKT3, APC, ARHGAP5, ATM, ATR, BRAF, CTNNB1, DNMT3A, EGFR, ERBB2, ERCC1, EZH2, FBXW7, FGFR3, FLT3, GABRA6, GABRG2, GNAQ, GNAS, IDH1, IDH2, JAK2, KIT, KRAS, MET, MLH1, MPL, MSH2, MTOR, MYD88, NF1, NPM1, NRAS/CSDE1, PARP1, PARP2, PDGFRA, PIK3CA, PTEN, PTPN11, RAD51, RB1, RET, SMAD4, TP53, and VHL, and/or the respective regulatory regions of any of the foregoing. Mutations to the foregoing genes that are associated with cancer are listed in Table 1.

**Table 1. Selected somatic mutations listed in the COSMIC database.**

| **Gene** | **Position** | **REF** | **ALT** | **Strand** | **CDS** | **AA** | **COSID** |
|---|---|---|---|---|---|---|---|
| MTOR | 11291097 | T | A | - | 2664 A>T | L888F | COSM94356 |
| MPL | 43815009 | G | T | + | 1544 G>T | W515L | COSM18918 |
| NRAS | 115256529 | T | C | - | 182 A>G | Q61R | COSM584 |
| PARP1 | 226551691 | TC | T | - | 2738 | G913fs*4 | COSM21691 |
| | | | | | del G | | |
| AKT3 | 243809253 | T | A | - | 371 A>T | Q124L | COSM48227 |
| DNMT3A | 25457243 | G | A | - | 2644 C>T | R882C | COSM53042 |
| MSH2 | 47705449 | TG | T | + | 2250 | G751fs*12 | COSM111644 |
| | | | | | del G | | |
| MSH2 | 47705558 | ACT | A | + | 2359_2360 | L787fs* 11 | COSM26122 |
| | | | | | del CT | | |
| IDH1 | 209113113 | G | A | - | 3 94C>T | R132C | COSM28747 |
| VHL | 10188282 | TTGAC | T | + | 426_429 | G144fs*14 | COSM18578 |
| | | | | | del TGAC | | |
| MLH1 | 37067240 | T | A | + | 1151 T>A | V384D | COSM26085 |
| MYD88 | 38182641 | T | C | + | 794 T>C | L265P | COSM85940 |
| CTNNB1 | 41266124 | A | G | + | 121 A>G | T41A | COSM5664 |
| ATR | 142254972 | GCTTTT AT | G | - | 3790_3796 | I1264fs*24 | COSM20627 |
| | | | | | del ATAAAAG | | |
| PIK3CA | 178936091 | G | A | + | 1633 G>A | E545K | COSM763 |
| PIK3CA | 178952085 | A | G | + | 3140 A>G | H1047R | COSM775 |
| PIK3CA | 178952149 | C | CA | + | 3204_3205 | N1068fs*4 | COSM12464 |
| | | | | | ins A | | |
| FGFR3 | 1803568 | C | G | + | 746 C>G | S249C | COSM715 |
| PDGFRA | 55141048 | T | TA | + | 1694_1695 | S566fs*6 | COSM28053 |
| | | | | | ins A | | |
| PDGFRA | 55152093 | A | T | + | 2525 A>T | D842V | COSM736 |
| KIT | 55599321 | A | T | + | 2447 A>T | D816V | COSM1314 |
| FBXW7 | 153249384 | C | T | - | 1394 G>A | R465H | COSM22965 |
| APC | 112175538 | GC | G | + | 4248 del C | I1417fs*2 | COSM18584 |
| APC | 112175639 | C | T | + | 4348 C>T | R1450* | COSM13127 |
| APC | 112175957 | A | AA | + | 4666_4667 | T1556fs*3 | COSM18561 |
| | | | | | ins A | | |
| GABRA6 | 161117296 | G | C | + | 763 G>C | V255L | COSM70853 |
| GABRG2 | 161580301 | A | G | + | 1355 A>G | Y452C | COSM74722 |
| NPM1 | 170837547 | G | GTCTG | + | 863_864 | W288fs* 12 | COSM17559 |
| | | | | | ins TCTG | | |
| EGFR | 55242465 | GGAATT | G | + | 2236_2250 | E746_A750 | COSM6225 |
| | | AAGAGA AGCA (SEQ ID NO: 16) | | | del 15 | del ELREA (SEQ ID NO: 17) | |
| EGFR | 55249012 | C | CGGT | + | 2310_2311 | D770 N771 | COSM12378 |
| | | | | | ins GGT | ins G | |
| EGFR | 55249071 | C | T | + | 2369 C>T | T790M | COSM6240 |
| EGFR | 55259515 | T | G | + | 2573 T>G | L858R | COSM6224 |
| MET | 116423428 | T | G | + | 3757 T>G | Y1253D | COSM700 |
| BRAF | 140453136 | A | T | - | 1799 T>A | V600E | COSM476 |
| EZH2 | 148508727 | T | A | - | 1937 A>T | Y646F | COSM37028 |
| JAK2 | 5073770 | G | T | + | 1849 G>T | V617F | COSM12600 |
| GNAQ | 80409488 | T | G | - | 626 A>C | Q209P | COSM28758 |
| RET | 43617416 | T | C | + | 2753 T>C | M918T | COSM965 |
| PTEN | 89692904 | C | T | + | 388 C>T | R130* | COSM5152 |
| PTEN | 89717716 | A | AA | + | 741_742 | P248fs*5 | COSM4986 |
| | | | | | ins A | | |
| PTEN | 89717774 | AA | A | + | 800 del A | K267fs*9 | COSM5809 |
| ATM | 108117846 | TGT | T | + | 1058_1059 | C353fs*5 | COSM21924 |
| | | | | | del GT | | |
| ATM | 108175462 | G | A | + | 5557 G>A | D1853N | COSM41596 |
| KRAS | 25398284 | C | T | - | 35 G>A | G12D | COSM521 |
| PTPN11 | 112888210 | G | A | + | 226 G>A | E76K | COSM13000 |
| FLT3 | 28592642 | C | A | - | 2503 G>T | D835Y | COSM783 |
| RB 1 | 48941648 | C | T | + | 958 C>T | R320* | COSM891 |
| PARP2 | 20820412 | A | C | + | 398 A>C | D133A | COSM75849 |
| ARHGAP5 | 32561739 | G | A | + | 1864 G>A | E622K | COSM88502 |
| AKT1 | 105246455 | C | T | - | 145 G>A | E49K | COSM36918 |
| AKT1 | 105246551 | C | T | - | 49 G>A | E17K | COSM33765 |
| RAD51 | 41001312 | C | T | + | 433 C>T | Q145* | COSM117943 |
| IDH2 | 90631838 | C | T | - | 515 G>A | R172K | COSM33733 |
| IDH2 | 90631934 | C | T | - | 419 G>A | R140Q | COSM41590 |
| TP53 | 7577120 | C | T | - | 818 G>A | R273H | COSM10660 |
| TP53 | 7577538 | C | T | - | 743 G>A | R248Q | COSM10662 |
| TP53 | 7577557 | AG | A | - | 723 del C | C242fs*5 | COSM6530 |
| TP53 | 7578406 | C | T | - | 524 G>A | R175H | COSM10648 |
| TP53 | 7579423 | GG | G | - | 263 del C | S90fs*33 | COSM18610 |
| NF1 | 29556989 | T | TAC | + | 2987_2988 | R997fs*16 | COSM41820 |
| | | | | | ins AC | | |
| NF1 | 29576111 | C | T | + | 4084 C>T | R1362* | COSM24443 |
| NF1 | 29679317 | TG | T | + | 7501 del G | E2501fs*22 | COSM24468 |
| SMAD4 | 48603093 | T | TT | + | 1394_1395 | A466fs*28 | COSM14105 |
| | | | | | ins T | | |
| AKT2 | 40761084 | C | A | - | 268 G>T | V90L | COSM93894 |
| ERCC1 | 45924470 | G | T | - | 287 C>A | A96E | COSM140843 |
| GNAS | 57484420 | C | T | + | 601 C>T | R201C | COSM27887 |

A nucleotide sequence may comprise a genotype selected from the group consisting of mutation c.145G>A to gene AKT1, mutation c.49G>A to gene AKT1, mutation c.268G>T to gene AKT2, mutation c.371A>T to gene AKT3, mutation c.4248delC to gene APC, mutation c.4348C>T to gene APC, mutation c.4666_4667insA to gene APC, mutation c.1864G>A to gene ARHGAP5, mutation c.1058_1059delGT to gene ATM, mutation c.5557G>A to gene ATM, mutation c.3790_3796delATAAAAG to gene ATR, mutation c. 1799T>A to gene BRAF, mutation c.121A>G to gene CTNNB1, mutation c.2644C>T to gene DNMT3A, mutation c.2236_2250del15 to gene EGFR, mutation c.2310_2311insGGT to gene EGFR, mutation c.2369C>T to gene EGFR, mutation c.2573T>G to gene EGFR, mutation c.2324_2325ins12 to gene ERBB2, mutation c.287C>A to gene ERCC1, mutation c.1937A>T to gene EZH2, mutation c.1394G>A to gene FBXW7, mutation c.746C>G to gene FGFR3, mutation c.2503G>T to gene FLT3, mutation c.763G>C to gene GABRA6, mutation c. 1355A>G to gene GABRG2, mutation c.626A>C to gene GNAQ, mutation c.601C>T to gene GNAS, mutation c.394C>T to gene IDH1, mutation c.515G>A to gene IDH2, mutation c.419G>A to gene IDH2, mutation c.1849G>T to gene JAK2, mutation c.2447A>T to gene KIT, mutation c.1679T>A to gene KIT, mutation c.35G>A to gene KRAS, mutation c.3757T>G to gene MET, mutation c.1151T>A to gene MLH1, mutation c.1544G>T to gene MPL, mutation c.2250delG to gene MSH2, mutation c.2359_2360delCT to gene MSH2, mutation c.2664A>T to gene MTOR, mutation c.794T>C to gene MYD88, mutation c.2987_2988insAC to gene NF1, mutation c.4084C>T to gene NF1, mutation c.7501delGto gene NF1, mutation c.863_864insTCTGto gene NPM1, mutation c.182A>G to gene NRAS, mutation c.2738delGto gene PARP1, mutation c.398A>C to gene PARP2, mutation c.1694_1695insA to gene PDGFRA, mutation c.2525A>T to gene PDGFRA, mutation c.1633G>A to gene PIK3CA, mutation c.3140A>Gto gene PIK3CA, mutation c.3204_3205insA to gene PIK3CA, mutation c.388C>T to gene PTEN, mutation c.741_742insA to gene PTEN, mutation c.800delA to gene PTEN, mutation c.226G>A to gene PTPN11, mutation c.433C>T to gene RAD51, mutation c.958C>T to gene RB1, mutation c.2753T>C to gene RET, mutation c.1394_1395insT to gene SMAD4, mutation c.818G>A to gene TP53, mutation c.743G>A to gene TP53, mutation c.723delC to gene TP53, mutation c.524G>A to gene TP53, mutation c.263delC to gene TP53, and mutation c.426_429delTGAC to gene VHL. At least 1, 2, 3, 4, 5, 6, 7, 8, 9, or 10 nucleotide sequences of a plurality of nucleotide sequences may comprise a genotype selected from the foregoing mutations. In some embodiments, each nucleotide sequence of a plurality of nucleotide sequences comprises a genotype selected from the foregoing mutations.

A nucleotide sequence may comprise a genotype selected from the group consisting of mutation c.942+3A>T to gene MSH2 (mutS homolog 2), mutation c.1662-12 1677del to gene MSH2, mutation c.2056 _2060delinsCTTCTACCTCAAAAA (SEQ ID NO: 13) to gene MSH6 (mutS homolog 6), mutation c.2308_2312delGGTAAinsT to gene MSH6, mutation c.2641delGinsAAAA to gene MSH6, mutation c.3163_3164insG to gene MSH6, mutation c.232_243delinsATGTAAGGto gene MLH1 (mutL homolog 1), mutation c.1852_1854delAAG to gene MI,H1, mutation c.2445+ 1 G>C to gene PMS2 (PMS1 homolog 2, mismatch repair system component), mutation c.2243_2246delAGAA to gene PMS2, mutation c.2253T>C to gene PMS2, mutation c.861_864delACAG to gene PMS2, mutation c.9_32dup24 to gene CDKN2A (cyclin dependent kinase inhibitor 2A), mutation c.8975_9100del126 to gene BRCA2 (breast cancer 2), mutation c.9203del126 to gene BRCA2, mutation c.1310_1313delAAGA to gene BRCA2, mutation c.1813dupA to gene BRCA2, mutation c.9342_9343insAluY to gene BRCA2, mutation c.5266_5267insC to gene BRCA1 (breast cancer 1), mutation c.5177_5180delGAAA to gene BRCA1, mutation c.3756_3759delGTCT to gene BRCA1, mutation c.3481_3491delGAAGATACTAG (SEQ ID NO: 14) to gene BRCA1, mutation c.3113A>G to gene BRCA1, mutation c.3084_3094delTAATAACATTA (SEQ ID NO: 15) to gene BRCA1, mutation c.2834_2836delinsC to gene BRCA1, and mutation c.68_69delAGto gene BRCA1. At least 1, 2, 3, 4, 5, 6, 7, 8, 9, or 10 nucleotide sequences of a plurality of nucleotide sequences may comprise a genotype selected from the foregoing mutations. In some embodiments, each nucleotide sequence of a plurality of nucleotide sequences comprises a genotype selected from the foregoing mutations.

**Table 2. Selected inheritable mutations.**

| **Gene** | **Position** | **REF** | **ALT** | **HGVS ID** | **dbSNP ID** |
|---|---|---|---|---|---|
| BMPR1A | 88635779 | C | A | | rs11528010 |
| BMPR1A | 88672141 | G | C | c.675+1G>C | rs12267107 |
| BMPR1A | 88677031 | C | T | c.817C>T | rs587782400 |
| BRCA1 | 41209079 | | G | c.5266_5267insC | rs397507246 |
| BRCA1 | 41215362 | TTTTC | T | c.5177_5180delGAAA | rs80357867 |
| BRCA1 | 41215946 | C | T | c.5096G>A | rs41293459 |
| BRCA1 | 41222948 | TTCTTCTG GGGTCAG GCCAG (SEQ ID NO: 18) | T | c.4964_4982delCTGG CCTGACCCCAGAA GA (SEQ ID NO: 19) | rs803 59876 |
| BRCA1 | 41243788 | AGAC | | c.3756_3759delGTCT | rs80357868 |
| BRCA1 | 41244056 | ACTAGTAT CTTC (SEQ ID NO: 20) | A | c.3481_3491delGAAG ATACTAG (SEQ ID NO: 14) | rs80357877 |
| BRCA1 | 41244434 | T | C | c.3113A>G | rs16941 |
| BRCA1 | 41244453 | TAATGTTA TTA (SEQ ID NO: 21) | | c.3084_3094delTAAT AACATTA (SEQ ID NO: 15) | rs80357647 |
| BRCA1 | 41244710 | ATAC | AG | c.2834_2836delinsC | rs386134270 |
| BRCA1 | 41245591 | TTTTC | T | c.1953_1956delGAAA | rs80357526 |
| BRCA1 | 41246157 | TTTT | CTTTC | c.1387_1390delinsGA AAG | rs397508866 |
| BRCA1 | 41246333 | TGATTCAG ACTCCCCA TCATGTGA GTCATCA GAACCTA ACA (SEQ ID NO: 22) | T | c.1175_1214delTGTT AGGTTCTGATGAC TCACATGATGGGG AGTCTGAATC (SEQ ID NO: 23) | rs80359874 |
| BRCA1 | 41246531 | C | CT | c.1016dupA | rs80357618 |
| BRCA1 | 41246723 | G | GCCACATGGCT **(SEQ ID NO: 24)** | c.815_824dupAGCCA TGTGG (SEQ ID NO: 25) | rs387906563 |
| BRCA1 | 41256173 | | T | c.406_407insA | rs80357709 |
| BRCA1 | 41258503 | A | C | c.181T>G | rs28897672 |
| BRCA1 | 41276044 | CT | | c.68_69delAG | rs386833395 |
| BRCA1 | 41215363 | GAAA | | c.5177_5180delGAAA | rs80357867 |
| BRCA2 | | | | c.9203del126 | rs80359736 |
| BRCA2 | 32893302 | T | TGCCGGGCGCGGTGGC TCACGCCTGTAATCCC AGCACTTTGGGAGGCC GAGGCGGGCGGATCAC GAGGTCAGGAGATCGA CACCATCCCGGCTGAA ACGGTGAAACCCCGTC TCTACTAAAAATACAA AAAATTAGCCGGGCGT AGTGGCGGGCGCCTGT AGTCCCAGCTACTTGG GAGGCTGAGGCAGGAG AATGGCGTGAACCCGG GAGGCGGAGCTTGCAG TG (SEQ ID NO: 26) | c.156_157insAluYa5 | |
| BRCA2 | 32906915 | AAAAG | A | c.1310_1313delAAGA | rs80359277 |
| BRCA2 | 32907420 | G | GA | c.1813dupA | rs397507277 |
| BRCA2 | 32911073 | C | CA | c.2588dupA | rs606231399 |
| BRCA2 | 32911297 | AAAC | | c.2808_2811delACAA | rs80359352 |
| BRCA2 | 32911888 | A | G | | |
| BRCA2 | 32912528 | CTGTTTG | T | c.4037_4043delinsT | rs276174841 |
| BRCA2 | 32913055 | A | G | | |
| BRCA2 | 32913558 | C | CA | c.5073dupA | rs80359480 |
| BRCA2 | 32913837 | AA | | c.5350_5351delAA | |
| BRCA2 | 32914236 | C | T | | |
| BRCA2 | 32914437 | T | | c.5946delT | |
| BRCA2 | 32914766 | TT | | c.6275_6276delTT | |
| BRCA2 | 32915005 | G | C | | |
| BRCA2 | 32929232 | A | G | | |
| BRCA2 | 32929387 | T | C | | |
| BRCA2 | 32932022 | ATA | TT | c.7762_7764delinsTT | rs483353072 |
| BRCA2 | 32936646 | T | C | | |
| BRCA2 | 32945137 | AG | | c.8537_8538delAG | rs80359715 |
| BRCA2 | 32953600 | ACCGTGT GGAAG | TCCC | c.8902_8913delinsTC CC | rs864622735 |
| | | (SEQ ID NO: 27) | | | |
| BRCA2 | 32954272 | G | GA | c.9253dupA | rs80359752 |
| BRCA2 | 32968896 | T | TAATGAGGACATTATTTT TTTTTTTTTTTTTTTTT TTTTTTTTTTTTTTTTT TTTGAGACGGAGTCTC GCTCTGTCGCCCAGGC CGGACTGTGGACTGCA GTGGCGCAATCTCGGC TCACTGCAAGCTCCGC TTCCCGGGTTCACGCC ATTCTCCTGCCTCAGCC TCCCGAGTAGCTGGGA CTACAGGCGCCCGCCA CCGCGCCCGGCTAATT TTTTGTATTTTTAGTAG AGACGGGGTTTCACCT TGTTAGCCAGGATGGT CTCGATCTCCTGACCTC ATGATCCACCCGCCTC GGCCTCCCAAAGTGCT GGGATTACAGGCGTGA GCCACCGCGCCCGGCC (SEQ ID NO: 28) | c.9342_9343insAluY | |
| BRCA2 | 32972744 | C | GAATTATATCT (SEQ ID NO: 29) | c.10095delCinsGAAT TATATCT (SEQ ID NO: 29) | rs276174803 |
| BRCA2 | 32906925 | AAGA | | c.1310_1313delAAGA | rs80359277 |
| CDH1 | 68771372 | C | T | | |
| CDH1 | 68855983 | C | T | c.1792C>T | rs121964877 |
| CDH1 | 68857441 | T | C | | |
| CDKN2A | 21974794 | A | AGGCTCCATGCTGCTC CCCGCCGCC (SEQ ID NO: 30) | c.9_32dup24 | rs587780668 |
| EPCAM | 47601029 | G | C | | rs146480420 |
| EPCAM | 47601106 | T | C | | rs1126497 |
| EPCAM | 47602375 | G | A | c.429G>A | |
| EPCAM | 47604183 | C | T | c.523C>T | |
| EPCAM | 47606078 | A | G | c.556-14A>G | rs376155665 |
| MLH1 | 37042468 | TGAAAGG TTCACT (SEQ ID NO: 31) | TATGTAAGG | c.232_243delinsATGT AAGG | rs63750437 |
| MLH1 | 37048482 | G | | c.382delG | |
| MLH1 | 37053568 | A | G | | rs1799977 |
| MLH1 | 37055948 | GATAAAA CCCTAGCC TTCAAA (SEQ ID NO: 32) | G | c.704_723delATAAA ACCCTAGCCTTCA AA (SEQ ID NO: 33) | rs863224480 |
| MLH1 | 37089122 | TGAA | T | c.1852_1854delAAG | rs121912962 |
| MLH1 | 37047639 | AAG | | c.1852_1854delAAG | rs121912962 |
| MSH2 | 47630550 | C | G | | rs2303426 |
| MSH2 | 47641560 | A | T | c.942+3A>T | rs193922376 |
| MSH2 | 47643567 | G | C | c.1076G>C | rs587779070 |
| MSH2 | 47698091 | TTTCGATT TGCAGCA AATTGACT TCTTTA (SEQ ID NO: 34) | T | c.1662-12_1677del | |
| MSH2 | 47703500 | T | C | | rs2303428 |
| MSH2 | 47414421 | A | T | c.942+3A>T | rs193922376 |
| MSH6 | 48010488 | G | A | | rs1042821 |
| MSH6 | 48025856 | TA | T | c.741delA | |
| MSH6 | 48027178 | GGTTG | CTTCTACCTCAAAAA (SEQ ID NO: 13) | c.2056_2060delinsCT TCTACCTCAAAAA (SEQ ID NO: 13) | |
| MSH6 | 48027429 | GGTAA | T | c.2308_2312delGGTA AinsT | rs864622585 |
| MSH6 | 48027762 | G | AAAA | c.2641delGinsAAAA | rs63751408 |
| MSH6 | 48028284 | | G | c.3163_3164insG | rs149605979 |
| MSH6 | 48030639 | AC | A | c.3261delC | rs267608078 |
| MSH6 | 48033727 | T | TTCAAAAGGACATAG AAAA (SEQ ID NO: 35) | c.3939_3957dupTCA | rs267608126 |
| | | | | AAAGGGACATAGA AAA (SEQ ID NO: 36) | |
| MUTYH | 45796894 | C | A | c.1435G>T | |
| MUTYH | 45797227 | C | T | c.1187G>A | rs36053993 |
| MUTYH | 45797371 | G | | c.1147delC | |
| MUTYH | 45797505 | C | G | | rs3219489 |
| MUTYH | 45797834 | T | G | c.933+3A>C | |
| MUTYH | 45798474 | T | C | c.536A>G | rs34612342 |
| PALB2 | 23619235 | A | C | | |
| PALB2 | 23632682 | C | T | c.3113G>A | rs180177132 |
| PALB2 | 23634293 | C | T | | |
| PALB2 | 23641089 | C | A | c.2386G>T | |
| PALB2 | 23641461 | C | G | | |
| PALB2 | 23646191 | T | C | | |
| PMS2 | 6017218 | C | G | c.2445+1G>C | |
| PMS2 | 6018255 | TTCT | | c.2243_2246delAGAA ; c.2253T>C | rs267608173 |
| PMS2 | 6022511 | CT | C | c.2117de1A | rs587782704 |
| PMS2 | 6026775 | T | C | | rs2228006 |
| PMS2 | 6027134 | G | A | c.1261C>T | |
| PMS2 | 6035203 | ACTGT | A | c.861_864delACAG | rs267608154 |
| PMS2 | 6036980 | G | C | | rs1805319 |
| PMS2 | 6045548 | C | A | c.137G>T | rs121434629 |
| PMS2 | 6035204 | ACAG | | c.861_864delACAG | rs267608154 |
| PTEN | 89711892 | C | T | c.511C>T | rs121909221 |
| PTEN | 89717729 | | AT | c.758_759msAT | |
| PTEN | 89720831 | G | GCAAATAAAGA (SEQ ID NO: 37) | c.987_996dupTAAAG ACAAA (SEQ ID NO: 38) | rs864622387 |
| SMAD4 | 48593453 | | T | c.1206_1207insT | |
| SMAD4 | 48603052 | G | GGCTACTGCACAAGCT GCAGCAGCTGCCC (SEQ ID NO: 39) | c.1353_1354insGCTA CTGCACAAGCTGC AGCAGCTGCCC (SEQ ID NO: 40) | rs786204125 |
| SMAD4 | 48604703 | G | | c.1529delG | rs377767371 |
| STK11 | 1221313 | | C | c.842_843insC | rs121913321 |
| STK11 | 1219317 | C | | | |
| TP53 | 7577021 | G | A | c.916C>T | rs121913344 |
| TP53 | 7578211 | G | A | c.637C>T | rs397516436 |
| TP53 | 7579472 | G | C | | rs1042522 |

A nucleotide sequence may comprise a genotype selected from the group consisting of mutation c.675+1G>C to gene BMPR1A, mutation c.817C>T to gene BMPR1A, mutation c.1016dupA to gene BRCA1, mutation c.1175_1214delTGTTAGGTTCTGATGACTCACATGATGGGGAGTCTGAATC (SEQ ID NO: 23) to gene BRCA1, mutation c.1387_1390delinsGAAAG to gene BRCA1, mutation c.181 T>G to gene BRCA1, mutation c.1953_1956delGAAA to gene BRCA1, mutation c.2834 _2836delinsC to gene BRCA1, mutation c.3084_3094delTAATAACATTA (SEQ ID NO: 15) to gene BRCA1, mutation c.3113A>G to gene BRCA1, mutation c.3481_3491delGAAGATACTAG (SEQ ID NO: 14) to gene BRCA1, mutation c.3756_3759delGTCT to gene BRCA1, mutation c.406_407insA to gene BRCA1, mutation c.4964_4982delCTGGCCTGACCCCAGAAGA (SEQ ID NO: 19) to gene BRCA1, mutation c.5096G>A to gene BRCA1, mutation c.5177_5180delGAAA to gene BRCA1, mutation c.5177_5180delGAAA to gene BRCA1, mutation c.5266_5267insC to gene BRCA1, mutation c.68_69delAGto gene BRCA1, mutation c.815_824dupAGCCATGTGG (SEQ ID NO: 25) to gene BRCA1, mutation c. 10095delCinsGAATTATATCT (SEQ ID NO: 29) to gene BRCA2, mutation c.1310_1313delAAGA to gene BRCA2, mutation c.1310_1313delAAGA to gene BRCA2, mutation c.156_157insAluYa5 to gene BRCA2, mutation c.1813dupA to gene BRCA2, mutation c.2588dupA to gene BRCA2, mutation c.2808_2811delACAA to gene BRCA2, mutation c.4037_4043delinsT to gene BRCA2, mutation c.5073dupA to gene BRCA2, mutation c.5350_5351delAA to gene BRCA2, mutation c.5946delT to gene BRCA2, mutation c.6275_6276delTT to gene BRCA2, mutation c.7762_7764delinsTT to gene BRCA2, mutation c.8537_8538delAG to gene BRCA2, mutation c.8902_8913delinsTCCC to gene BRCA2, mutation c.9203del126 to gene BRCA2, mutation c.9253dupA to gene BRCA2, mutation c.9342_9343insAluY to gene BRCA2, mutation c.1792C>T to gene CDH1, mutation c.9_32dup24 to gene CDKN2A, mutation c.429G>A to gene EPCAM, mutation c.523C>T to gene EPCAM, mutation c.556-14A>G to gene EPCAM, mutation c.1852_1854delAAG to gene MI,H1, mutation c. 1852_1854delAAGto gene MI,H1, mutation c.232_243delinsATGTAAGG to gene MI,H1, mutation c.382delG to gene MLH1, mutation c.704_723delATAAAACCCTAGCCTTCAAA (SEQ ID NO: 33) to gene MI,H1, mutation c.1076G>C to gene MSH2, mutation c.1662-12_1677del to gene MSH2, mutation c.942+3A>T to gene MSH2, mutation c.942+3A>T to gene MSH2, mutation c.2056_2060delinsCTTCTACCTCAAAAA (SEQ ID NO: 13) to gene MSH6, mutation c.2308_2312delGGTAAinsT to gene MSH6, mutation c.2641delGinsAAAA to gene MSH6, mutation c.3163_3164insGto gene MSH6, mutation c.3261delC to gene MSH6, mutation c.3939_3957dupTCAAAAGGGACATAGAAAA (SEQ ID NO: 36) to gene MSH6, mutation c.741delA to gene MSH6, mutation c.1147delC to gene MUTYH, mutation c.1187G>A to gene MUTYH, mutation c.1435G>T to gene MUTYH, mutation c.536A>G to gene MUTYH, mutation c.933+3A>C to gene MUTYH, mutation c.2386G>T to gene PALB2, mutation c.3113G>A to gene PALB2, mutation c.1261C>T to gene PMS2, mutation c.137G>T to gene PMS2, mutation c.2117delA to gene PMS2, mutation c.2243_2246delAGAA to gene PMS2, c.2253T>C to gene PMS2, mutation c.2445+1G>C to gene PMS2, mutation c.861_864delACAG to gene PMS2, mutation c.861_864delACAGto gene PMS2, mutation c.511C>T to gene PTEN, mutation c.758_759insAT to gene PTEN, mutation c.987_996dupTAAAGACAAA (SEQ ID NO: 38) to gene PTEN, mutation c.1206_1207insT to gene SMAD4, mutation c.1353_1354insGCTACTGCACAAGCTGCAGCAGCTGCCC (SEQ ID NO: 40) to gene SMAD4, mutation c.1529delG to gene SMAD4, mutation c.842_843insC to gene STK11, mutation c.637C>T to gene TP53, and mutation c.916C>T to gene TP53. At least 1, 2, 3, 4, 5, 6, 7, 8, 9, or 10 nucleotide sequences of a plurality of nucleotide sequences may comprise a genotype selected from the foregoing mutations. In some embodiments, each nucleotide sequence of a plurality of nucleotide sequences comprises a genotype selected from the foregoing mutations.

A nucleotide sequence of a plurality of nucleotide sequences may be a subsequence of a gene selected from the group consisting of BRAF, CTNNB1, EGFR, ERBB2, IDH1, KIT, KRAS, NRAS/CSDE1, PDGFRA, PIK3CA, PTEN, RET, and TP53, and/or the respective regulatory regions of any one of the foregoing. At least 1, 2, 3, 4, 5, 6, 7, 8, 9, or 10 nucleotide sequences of a plurality of nucleotide sequences may be subsequences of genes selected from the group consisting of BRAF, CTNNB1, EGFR, ERBB2, IDH1, KIT, KRAS, NRAS/CSDE1, PDGFRA, PIK3CA, PTEN, RET, and TP53, and/or the respective regulatory regions of any of the foregoing. Each nucleotide sequence of a plurality of nucleotide sequences may comprise a subsequence of a gene selected from the group consisting of BRAF, CTNNB1, EGFR, ERBB2, IDH1, KIT, KRAS, NRAS/CSDE1, PDGFRA, PIK3CA, PTEN, RET, and TP53, and/or the respective regulatory regions of any of the foregoing.

A nucleotide sequence may comprise a genotype selected from the group consisting of mutation c.1799T>A to gene BRAF, mutation c.121A>G to gene CTNNB1, mutation c.2236_2250del15 to gene EGFR, mutation c.2369C>T to gene EGFR, mutation c.2573T>G to gene EGFR, mutation c.2324_2325ins12 to gene ERBB2, mutation c.394C>T to gene IDH1, mutation c.1679T>A to gene KIT, mutation c.35G>A to gene KRAS, mutation c. 182A>G to gene NRAS/CSDE1, mutation c.2525A>T to gene PDGFRA, mutation c. 1633G>A to gene PIK3CA, mutation c.3140A>G to gene PIK3CA, mutation c.800delA to gene PTEN, mutation c.2753T>C to gene RET, and mutation c.524G>A to gene TP53. At least 1, 2, 3, 4, 5, 6, 7, 8, 9, or 10 nucleotide sequences of a plurality of nucleotide sequences may comprise a genotype selected from the foregoing mutations. In some embodiments, each nucleotide sequence of a plurality of nucleotide sequences comprises a genotype selected from the foregoing mutations. For example, a plurality of nucleotide sequences may consist of 3, 4, 5, 6, 7, 8, 9, 10, 11, 12, 13, 14, 15, or 16 nucleotide sequences; each nucleotide sequence may be a subsequence of a human gene selected from BRAF, CTNNB1, EGFR, ERBB2, IDH1, KIT, KRAS, NRAS/CSDE1, PDGFRA, PIK3CA, PTEN, RET, and TP53; and each genotype of a nucleotide sequence may be selected from mutation c.1799T>A to gene BRAF, mutation c.121A>G to gene CTNNB1, mutation c.2236_2250del15 to gene EGFR, mutation c.2369C>T to gene EGFR, mutation c.2573T>G to gene EGFR, mutation c.2324_2325ins12 to gene ERBB2, mutation c.394C>T to gene IDH1, mutation c.1679T>A to gene KIT, mutation c.35G>A to gene KRAS, mutation c.182A>G to gene NRAS/CSDE1, mutation c.2525A>T to gene PDGFRA, mutation c.1633G>A to gene PIK3CA, mutation c.3140A>Gto gene PIK3CA, mutation c.800delA to gene PTEN, mutation c.2753T>C to gene RET, and mutation c.524G>A to gene TP53, wherein each nucleotide sequence comprises a different genotype.

Each nucleotide sequence of a plurality of nucleotide sequences comprises a different genotype. Each nucleotide sequence of a plurality of nucleotide sequences may or may not comprise a subsequence of different genes. For example, a plurality of nucleotide sequences may comprise a first nucleotide sequence that comprises the genotype mutation c.2369C>T to gene EGFR and a second nucleotide sequence that comprises the genotype mutation c.2573T>G to gene EGFR, and such a plurality would comprise two nucleotide sequences that comprise subsequences of the same gene EGFR. These genotypes are proximal in the EGFR gene-separated by 204 nucleotides - and the subsequences may overlap either partially, completely, or not at all. In comparison, a plurality of nucleotide sequences may comprise a first nucleotide sequence that comprises the mutation c.2369C>T to gene EGFR and a second nucleotide sequence that comprises the same mutation c.2369C>T to gene EGFR only if at least one of the first or second nucleotide sequences also comprises a different genotype (*e.g*., c.2573T>G to gene EGFR) because each nucleotide sequence of a plurality of nucleotide sequences comprises a different genotype.

A nucleic acid may comprise the nucleotide sequence set forth in SEQ ID NO:1. A nucleic acid may consist of the nucleotide sequence set forth in SEQ ID NO:1. A nucleic acid may have at least about 80%, about 85%, about 95%, about 96%, about 97%, about 98%, or about 99% sequence homology with the nucleotide sequence set forth in SEQ ID NO:1. A nucleotide sequence of a plurality of nucleotide sequences may have the nucleotide sequence spanning nucleotides 8545 to 9153 of SEQ ID NO:1 (mutation c.800delA to gene PTEN), nucleotides 7937 to 8544 of SEQ ID NO:1 (mutation c.1799T>A to gene BRAF), nucleotides 7327 to 7936 of SEQ ID NO:1 (mutation c.121A>G to gene CTNNB1), nucleotides 6712 to 7326 of SEQ ID NO:1 (mutation c.2573T>G to gene EGFR), nucleotides 6115 to 6711 of SEQ ID NO:1 (mutation c.2236_2250del15 to gene EGFR), nucleotides 5503 to 6114 of SEQ ID NO:1 (mutation c.2369C>T to gene EGFR), nucleotides 4879 to 5502 of SEQ ID NO:1 (mutation c.2324_2325ins12 to gene ERBB2), nucleotides 4271 to 4878 of SEQ ID NO:1 (mutation c.35G>A to gene KRAS), nucleotides 3661 to 4270 of SEQ ID NO:1 (mutation c.182A>G to gene NRAS), nucleotides 3051 to 3660 of SEQ ID NO:1 (mutation c.2525A>T to gene PDGFRA), nucleotides 2445 to 3050 of SEQ ID NO:1 (mutation c.1633G>A to gene PIK3CA), nucleotides 1839 to 2444 of SEQ ID NO:1 (mutation c.3140A>G to gene PIK3CA), nucleotides 1227 to 1838 of SEQ ID NO:1 (mutation c.2753T>C to gene RET), nucleotides 1 to 610 of SEQ ID NO:1 (mutation c.524G>A to gene TP53), nucleotides 611 to 1226 of SEQ ID NO:1 (mutation c.1679T>A to gene KIT), or nucleotides 9154 to 9854 of SEQ ID NO:1 (mutation c.394C>T to gene IDH1). A nucleotide sequence of a plurality of nucleotide sequences may have at least about 95%, about 96%, about 97%, about 98%, or about 99% sequence homology with the nucleotide sequence spanning nucleotides 8545 to 9153 of SEQ ID NO:1 (mutation c.800delA to gene PTEN), nucleotides 7937 to 8544 of SEQ ID NO:1 (mutation c.1799T>A to gene BRAF), nucleotides 7327 to 7936 of SEQ ID NO:1 (mutation c.121A>G to gene CTNNB1), nucleotides 6712 to 7326 of SEQ ID NO:1 (mutation c.2573T>G to gene EGFR), nucleotides 6115 to 6711 of SEQ ID NO:1 (mutation c.2236_2250del15 to gene EGFR), nucleotides 5503 to 6114 of SEQ ID NO:1 (mutation c.2369C>T to gene EGFR), nucleotides 4879 to 5502 of SEQ ID NO:1 (mutation c.2324_2325ins12 to gene ERBB2), nucleotides 4271 to 4878 of SEQ ID NO:1 (mutation c.35G>A to gene KRAS), nucleotides 3661 to 4270 of SEQ ID NO:1 (mutation c.182A>G to gene NRAS), nucleotides 3051 to 3660 of SEQ ID NO:1 (mutation c.2525A>T to gene PDGFRA), nucleotides 2445 to 3050 of SEQ ID NO:1 (mutation c.1633G>A to gene PIK3CA), nucleotides 1839 to 2444 of SEQ ID NO:1 (mutation c.3140A>G to gene PIK3CA), nucleotides 1227 to 1838 of SEQ ID NO:1 (mutation c.2753T>C to gene RET), nucleotides 1 to 610 of SEQ ID NO:1 (mutation c.524G>A to gene TP53), nucleotides 611 to 1226 of SEQ ID NO:1 (mutation c.1679T>A to gene KIT), or nucleotides 9154 to 9854 of SEQ ID NO: 1 (mutation c.394C>T to gene IDH1). A nucleotide sequence of a plurality might not have 100% sequence homology with a nucleotide sequence spanning the above-referenced regions of SEQ ID NO:1, for example, if the nucleotide sequence of the plurality has a different length than the spanning nucleotide sequence or if the nucleotide sequence of the plurality has been modified to include restriction sites.

A nucleic acid may comprise the nucleotide sequence set forth in SEQ ID NO:2 or SEQ ID NO:3. A nucleic acid may consist of the nucleotide sequence set forth in SEQ ID NO:2 or SEQ ID NO:3. A nucleic acid may have at least about 80%, about 85%, about 95%, about 96%, about 97%, about 98%, or about 99% sequence homology with the nucleotide sequence set forth in SEQ ID NO:2 or SEQ ID NO:3.

### III. EMBODIMENTS RELATED TO HEART CONDITIONS

In some aspects, a disease or condition may be a heart condition, such as cardiomyopathy. In some embodiments, each nucleotide sequence of the plurality is associated with a heart condition, such as cardiomyopathy. Selected single nucleotide polymorphisms, variants, and mutations that are associated with cardiomyopathy are listed in Table 3.

**Table 3. Selected Genetic Variants Associated with Cardiomyopathy**

| Gene | Reference SNP | Genetic Variation | Protein Mutation | Genome Build DNA |
|---|---|---|---|---|
| MYBPC3 | rs375882485 | c.1504C>T | p.Arg502Trp | g.47364249G>A |
| MYBPC3 | rs397515963 | c.2373_2374insG | p.Trp792ValfsX41 | g.47359280_47359281 insC |
| MYBPC3 | rs36212066 | c.3628-41_3628-17del | Intron variant | g.47353826_47353850 del |
| MYH7 | rs371898076 | c.1988G>A | p.Arg663His | g.23896042C>T |
| MYH7 | rs121913625 | c.1357C>T | p.Arg453Cys | g.23898214G>A |
| MYH7 | rs121913626 | c.1750G>C | p.Gly584Arg | g.23896932C>G |
| TNNI3 | rs397516351 | c.532_534delAAG | p.Lys178del | g.55665413_55665415 delCTT |
| TNNI3 | rs104894729 | c.575G>A | p.Arg192His | g.55663260C>T |
| TNNT2 | rs397516470 | c.487 489delGAG | p.Glu163del | g.201332505 201332507 delCTC |
| TPM1 | rs 199476315 | c.574G>A | p.Glu192Lys | g.63353922G>A |

A nucleotide sequence of a plurality of nucleotide sequences may be a subsequence of a gene selected from the group consisting of cardiac myosin-binding protein C (MYBPC3); myosin, heavy chain 7, cardiac muscle, beta (MYH7); troponin I type 3 (TNNI3); cardiac troponin T (TNNT2); and tropomyosin alpha-1 chain (TPM1), and/or the respective regulatory regions of any one of the foregoing. At least 1, 2, 3, 4, 5, 6, 7, 8, 9, or 10 nucleotide sequences of a plurality of nucleotide sequences may be subsequences of genes selected from the group consisting of MYBPC3, MYH7, TNNI3, TNNT2, and TPM1, and/or the respective regulatory regions of any of the foregoing. Each nucleotide sequence of a plurality of nucleotide sequences may comprise a subsequence of a gene selected from the group consisting of MYBPC3, MYH7, TNNI3, TNNT2, and TPM1, and/or the respective regulatory regions of any of the foregoing.

Each subsequence may comprise a mutation that is associated with the disease or condition (*e.g.,* cardiomyopathy) or a healthy genotype at a position wherein such mutations are known to occur.

A nucleotide sequence may comprise a genotype selected from the group consisting of mutation 1504C>T to gene MYBPC3, mutation 2373_2374insGto gene MYBPC3, mutation 3628-41_3628-17del to gene MYBPC3, mutation 1988G>A to gene MYH7, mutation 1357C>T to gene MYH7, mutation 1750G>C to gene MYH7, mutation 532_534delAAG to gene TNNI3, mutation 575G>A to gene TNNI3, mutation 487_489delGAG to gene TNNT2, and mutation 574G>A to gene TPM1. At least 1, 2, 3, 4, 5, 6, 7, 8, 9, or 10 nucleotide sequences of a plurality of nucleotide sequences may comprise genotypes selected from mutation 1504C>T to gene MYBPC3, mutation 2373_2374insGto gene MYBPC3, mutation 3628-41_3628-17del to gene MYBPC3, mutation 1988G>A to gene MYH7, mutation 1357C>T to gene MYH7, mutation 1750G>C to gene MYH7, mutation 532_534delAAGto gene TNNI3, mutation 575G>A to gene TNNI3, mutation 487_489delGAG to gene TNNT2, and mutation 574G>A to gene TPM1. In some embodiments, each nucleotide sequence of a plurality of nucleotide sequences comprises a genotype selected from mutation 1504C>T to gene MYBPC3, mutation 2373_2374insG to gene MYBPC3, mutation 3628-41_3628-17del to gene MYBPC3, mutation 1988G>A to gene MYH7, mutation 1357C>T to gene MYH7, mutation 1750G>C to gene MYH7, mutation 532_534delAAG to gene TNNI3, mutation 575G>A to gene TNNI3, mutation 487_489delGAG to gene TNNT2, and mutation 574G>A to gene TPM1.

At least 1, 2, 3, 4, 5, 6, 7, 8, 9, 10, 11, 12, 13, 14, 15, 16, 17, 18, 19, 20, 1, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotide sequences of a plurality of nucleotide sequences may be subsequences of genes selected from the group consisting of ABCC9, ACTC1, ACTN2, ANKRD1, BAG3, CASQ2, CAV3, CHRM2, CRYAB, CSRP3, DES, DMD, DOLK, DSC2, DSG2, DSP, DTNA, EMD, FHL2, GATAD1, GLA, ILK, JPH2, JUP, LAMA4, LAMP2, LDB3, LMNA, MURC, MYBPC3, MYH6, MYH7, MYL2, MYL3, MYLK2, MYOM1, MYOZ2, MYPN, NEBL, NEXN, PDLIM3, PKP2, PLN, PRDM16, PRKAG2, PTPN11, RAF1, RBM20, RYR2, SCN5A, SGCD, TAZ, TCAP, TMEM43, TNNC1, TNNI3, TNNT2, TPM1, TRDN, TTN, TTR, and VCL, and/or the respective regulatory regions of any of the foregoing. Each nucleotide sequence of a plurality of nucleotide sequences may comprise a subsequence of a gene selected from the group consisting of ABCC9, ACTC1, ACTN2, ANKRD1, BAG3, CASQ2, CAV3, CHRM2, CRYAB, CSRP3, DES, DMD, DOLK, DSC2, DSG2, DSP, DTNA, EMD, FHL2, GATAD1, GLA, ILK, JPH2, JUP, LAMA4, LAMP2, LDB3, LMNA, MURC, MYBPC3, MYH6, MYH7, MYL2, MYL3, MYLK2, MYOM1, MYOZ2, MYPN, NEBL, NEXN, PDLIM3, PKP2, PLN, PRDM16, PRKAG2, PTPN11, RAF1, RBM20, RYR2, SCN5A, SGCD, TAZ, TCAP, TMEM43, TNNC1, TNNI3, TNNT2, TPM1, TRDN, TTN, TTR, and VCL and/or the respective regulatory regions of any of the foregoing.

At least 1, 2, 3, 4, 5, 6, 7, 8, 9, 10, 11, 12, 13, 14, 15, 16, 17, 18, 19, 20, 1, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotide sequences of a plurality of nucleotide sequences may be subsequences of genes selected from the group consisting of ABCC9, ACTC1, ACTN2, AKAP9, ANK2, ANKRD1, BAG3, BRAF, CACNA1C, CACNB2, CASQ2, CAV3, CRYAB, CSRP3, DES, DMD, DSC2, DSG2, DSP, DTNA, EMD, FKTN, GATAD1, GLA, GPD1L, HCN4, HRAS, ILK, JPH2, JUP, KCNE1, KCNE2, KCNE3, KCNH2, KCNJ2, KCNJ5, KCNJ8, KCNQ1, KRAS, LAMA4, LAMP2, LDB3, LMNA, MAP2K1, MAP2K2, MTND1, MTND5, MTND6, MTTD, MTTG, MTTH, MTTI, MTTK, MTTL1, MTTL2, MTTM, MTTQ, MTTS1, MTTS2, MYBPC3, MYH7, MYL2, MYL3, MYLK2, MYOZ2, MYPN, NEBL, NEXN, NKX2.5, NRAS, PDLIM3, PKP2, PLN, PRKAG2, PTPN11, RAF1, RANGRF, RBM20, RYR2, SCN1B, SCN3B, SCN4B, SCN5A, SGCD, SNTA1, SOS1, TAZ, TCAP, TMEM43, TMPO, TNNC1, TNNI3, TNNT2, TPM1, TTN, TTR, and VCL, and/or the respective regulatory regions of any of the foregoing. Each nucleotide sequence of a plurality of nucleotide sequences may comprise a subsequence of a gene selected from the group consisting of ABCC9, ACTC1, ACTN2, AKAP9, ANK2, ANKRD1, BAG3, BRAF, CACNA1C, CACNB2, CASQ2, CAV3, CRYAB, CSRP3, DES, DMD, DSC2, DSG2, DSP, DTNA, EMD, FKTN, GATAD1, GLA, GPD1L, HCN4, HRAS, ILK, JPH2, JUP, KCNE1, KCNE2, KCNE3, KCNH2, KCNJ2, KCNJ5, KCNJ8, KCNQ1, KRAS, LAMA4, LAMP2, LDB3, LMNA, MAP2K1, MAP2K2, MTND1, MTND5, MTND6, MTTD, MTTG, MTTH, MTTI, MTTK, MTTL1, MTTL2, MTTM, MTTQ, MTTS1, MTTS2, MYBPC3, MYH7, MYL2, MYL3, MYLK2, MYOZ2, MYPN, NEBL, NEXN, NKX2.5, NRAS, PDLIM3, PKP2, PLN, PRKAG2, PTPN11, RAF1, RANGRF, RBM20, RYR2, SCN1B, SCN3B, SCN4B, SCN5A, SGCD, SNTA1, SOS1, TAZ, TCAP, TMEM43, TMPO, TNNC1, TNNI3, TNNT2, TPM1, TTN, TTR, and VCL, and/or the respective regulatory regions of any of the foregoing.

A nucleic acid may comprise the nucleotide sequence set forth in SEQ ID NO:4. A nucleic acid may consist of the nucleotide sequence set forth in SEQ ID NO:4. A nucleic acid may have at least about 95%, about 96%, about 97%, about 98%, or about 99% sequence homology with the nucleotide sequence set forth in SEQ ID NO:4.

### IV. COMPOSITIONS COMPRISING A PLURALITY OF NUCLEIC ACID FRAGMENTS

A single, multiplexed nucleic acid, however, may fragment and/or degrade during manufacturing, storage, and/or processing. A multiplexed nucleic acid comprising multiple different nucleotide sequences presents many advantages for preparing reference materials. Fragmentation and/or degradation does not necessarily affect the performance of a reference material, however, because next generation sequencing strategies assemble relatively long nucleotide sequences from relatively short nucleic acids. Further, the fragmentation and/or degradation of a single, multiplexed nucleic acid may be desirable, for example, because shorter nucleic acids more closely replicate the mRNAs of a transcriptome after it has been extracted from a cell.

Accordingly, also described herein is a composition comprising a plurality of nucleic acid fragments. Sequence assembly of the nucleotide sequences of the plurality of nucleic acid fragments may result in the complete nucleotide sequence of a full-length nucleic acid as described in sections I-III, *supra.* The term "sequence assembly" refers to the alignment and merging of the nucleotide sequences of a plurality of nucleic acid fragments into longer nucleotide sequences in order to reconstruct the original nucleotide sequence (*see, e.g.,* El-Metwally, S. et al., PLoS Computational Biology 9(12): e1003345 (2013); Nagarajan, N. and M. Pop, Nature Reviews Genetics 14(3): 157 (2013); Paszkiewicz, K. and D.J. Studholme, Briefings Bioinformatics 11(5):457 (2010)). Sequence assembly of the nucleotide sequences of a plurality of nucleic acid fragments may result in less than the complete nucleotide sequence of a full-length nucleic acid so long as each nucleotide sequence of the plurality of nucleotide sequences of the full-length nucleic acid *(e.g.,* as described in sections I-III) is encoded by at least one nucleic acid fragment of the plurality of nucleic acids. For example, sequence assembly of the nucleotide sequences of the nucleic acid fragments of the plurality may result in assembled sequences that align with at least 50%, 60%, 70%, 80%, 90%, 95%, 96%, 97%, 98%, or 99% of the nucleotide sequence of the full-length nucleic acid. Omitted nucleotide sequences may include, for example, unstable nucleotide sequences and/or specific nucleotide sequences that are intentionally depleted or otherwise selected against (*e.g*., during a hybridization or amplification step).

A plurality of nucleic acid fragments may be produced from a full-length nucleic acid as described in sections I-III, *supra* (*e.g.,* the plurality of nucleic acid fragments may be produced from a number of copies of the same full-length nucleic acid). The plurality of nucleic acid fragments may consist of fragments or degradation products of a full-length nucleic acid as described in sections I-III, *supra* (*e.g.,* the plurality of nucleic acid fragments may consist of fragments or degradation products from a number of copies of the same full-length nucleic acid).

Each nucleotide sequence of a plurality of nucleotide sequences of a full-length nucleic acid as described in sections I-III, *supra,* may be encoded by at least one nucleic acid fragment of a plurality of nucleic acid fragments.

Different copies of the same nucleic acid may be fragmented/degraded in many different ways, and thus, a plurality of nucleic acid fragments may or may not comprise identical nucleic acid fragments. Further, portions of individual nucleic acids may be lost, for example, during a purification step, or degraded to a length that lacks sequenceable content. Nevertheless, next generation sequencing can reassemble the nucleotide sequence of the original, unfragmented, full-length nucleic acid from the plurality of nucleic acid fragments so long as the plurality of nucleic acid fragments contains sufficient redundancy. For example, the plurality of nucleic acid fragments may comprise about 2x to about 1,000,000x coverage of the nucleotide sequence of an original, unfragmented, full-length nucleic acid, such as about 10x to about 100,000x, about 20x to about 50,000x, about 100x to about 10,000x, or about 100x to about 1000x coverage. Thus, the nucleotide sequence of the original, unfragmented, full-length nucleic acid may be identified by sequencing the plurality of nucleic acid fragments by next generation sequencing.

The plurality of nucleic acid fragments may comprise about 2x to about 1,000,000x coverage of each nucleotide sequence of the plurality of nucleotide sequences of an original, unfragmented, full-length nucleic acid, such as about 10x to about 100,000x, about 20x to about 50,000x, about 100x to about 10,000x, or about 100x to about 1000x coverage. Thus, each nucleotide sequence of the plurality of nucleotide sequences of the original, unfragmented, full-length nucleic acid may be identified by sequencing the plurality of nucleic acid fragments by next generation sequencing.

A composition comprising a plurality of nucleic acid fragments may further comprise substantially all of the genome of a cell. The ratio of the nucleotide sequence of the original, unfragmented, full-length nucleic acid *(e.g.,* the nucleic acid from which the plurality of nucleic acid fragments originated) to a single copy of the genome of the cell may be about 1:10 to about 1000: 1, such as about 1:5 to about 500:1, about 1:3 to about 300:1, about 1:2 to about 200:1, or about 1:1 to about 100:1 in the composition. The ratio of each copy of a nucleotide sequence of a plurality of nucleotide sequences of the original, unfragmented, full-length nucleic acid (*e.g.,* the nucleic acid from which the plurality of nucleic acid fragments originated) to a single copy of the genome of the cell may be about 1:10 to about 1000: 1, such as about 1:5 to about 500:1, about 1:3 to about 300: 1, about 1:2 to about 200:1, or about 1:1 to about 100:1 in the composition.

A composition comprising a plurality of nucleic acid fragments may further comprise a cell. The cell may be the cell of the genome, *supra, i.e.,* the composition may comprise substantially all of a genome of a cell because the composition comprises a cell. The cell may be a human cell. The cell may be a fibroblast or a lymphocyte, such as an immortalized B lymphocyte. The cell may be GM24385. The cell may be any of the cells described herein, *infra.*

The composition may comprise a plurality of cells. The plurality of cells may comprise the cell, *supra, e.g.,* wherein the genome of the composition is the genome of the cell. Each cell of a plurality of cells may comprise substantially the same genome. "Substantially the same genome" refers to genomes from the same individual (*e.g*., person), from the same parent cell, or from the same cell line, which may contain slight differences, such as epigenetic differences, spontaneous mutations, and mutations arising from processing, such as transfection and cell-fixation (*e.g.*, which may affect the integrity of cellular DNA).

The plurality of nucleic acid fragments of a composition may be intracellular nucleic acid fragments, *e.g.,* the plurality of nucleic acid fragments may exist intracellularly, for example, in the cytoplasm and/or nucleus of a cell. The plurality of cells may comprise the plurality of nucleic acid fragments of the composition. The plurality of nucleic acid fragments may have been introduced into cells of the composition (*e.g.*, a plurality of cells) by transfection. "Transfection" refers to the introduction of exogenous material into a cell, and the term includes the introduction of exogenous nucleic acids by transformation, transfection, infection (*e.g.*, with a recombinant virus), and electroporation, as well as other known methods. A full-length nucleic acid as described in sections I-III, *supra,* may be introduced into cells of the composition by transfection, and the full-length nucleic acid may be fragmented and/or degraded into the plurality of nucleic acid fragments during transfection or after transfection, thereby generating the plurality of nucleic acid fragments.

In some instances described herein, each cell of the plurality of cells is fixed. Methods for fixing cells are described herein, *infra,* and include formalin-fixation. In some instances described herein, the cells of the composition are embedded in paraffin.

In some instances described herein, the composition does not comprise cells. For example, the composition may simply comprise a plurality of nucleic acid fragments generated from a full-length nucleic acid described in sections I-III, *supra.* The composition may comprise nucleic acids extracted from cells described in the preceding paragraphs, *e.g.,* the plurality of nucleic acid fragments may be extracted from a plurality of cells as described in the preceding paragraphs, *e.g.,* along with the genomes of the plurality of cells. Thus, the plurality of nucleic acid fragments may have been extracted from a cell or from a plurality of cells.

The composition may further comprise urea *(e.g.,* 100 mM to 8 M urea), guanidine (*e.g.,* 100 mM to 6 M guanidine), a DNAse inhibitor, a metal chelator (*e.g.,* ethylenediaminetetraacetate), a protease *(e.g.,* proteinase K), an RNAse, ethanol *(e.g.,* 10-99% ethanol), isopropanol *(e.g.,* 10-99% isopropanol), and/or a DNA polymerase *(e.g.,* Taq polymerase). Methods of extracting and purifying DNA from cells using the foregoing reagents are well known. The plurality of nucleic acid fragments may be associated with a solid support, such as beads (*e.g.*, magnetic beads), to assist in purification.

### V. CELLS

In some aspects, the disclosure relates to a cell comprising a nucleic acid as described herein. A nucleic acid of the disclosure may be integrated into the genome of a cell, or it may be present on a plasmid or as a linear nucleic acid, such as a linear plasmid. A nucleic acid may be present in a cell but not integrated into the genome of the cell.

A cell may comprise at least two nucleic acids as described herein, *e.g.,* wherein at least two of the nucleic acids comprise different pluralities of nucleotide sequences. For example, a cell may comprise a plurality of nucleic acid fragments as described herein, wherein 2 to 50, 2 to 40, 2 to 30, 2 to 20, 2 to 10, 2 to 9, 2 to 8, 2 to 7, 2 to 6, 2 to 5, or 2 to 4 nucleic acid fragments of the plurality each comprise different pluralities of nucleotide sequences.

A cell may comprise more than one copy of the same nucleic acid. For example, a cell may comprise 1, 2, 3, 4, 5, 6, 7, 8, 9, 10, 15, 20, 25, 30, 35, 40, 45, 50, 60, 70, 80, 90, 100, 120, 150, or 200 copies of the same nucleic acid. A cell may comprise at least 1, 2, 3, 4, 5, 6, 7, 8, 9, 10, 15, 20, 25, 30, 35, 40, 45, 50, 60, 70, 80, 90, 100, 120, 150, or 200 copies of the same nucleic acid. A cell may comprise 1 to 1000, 2 to 1000, 5 to 1000, 10 to 1000, 20 to 1000, 50 to 1000, 100 to 1000, 150 to 1000, 200 to 1000, 250 to 1000, 1 to 500, 2 to 500, 5 to 500, 10 to 500, 20 to 1000, 50 to 500, 100 to 500, 150 to 500, or 200 to 500, 250 to 500, 1 to 400, 2 to 400, 5 to 400, 10 to 400, 20 to 400, 50 to 400, 100 to 400, 150 to 400, 200 to 400, or 250 to 400 copies of the same nucleic acid.

A nucleic acid may become fragmented or otherwise degrade before, during, or after transfection of the nucleic acid into a cell. Accordingly, in some embodiments, a cell may comprise a plurality of nucleic acid fragments *(*e.g., that are either fragments of a single, full-length nucleic acid as described herein, *supra,* or fragments of multiple copies of a single, full-length nucleic acid as described herein, *supra*). The plurality of nucleic acid fragments may be admixed with the nucleic acids of the cell, *e.g.,* cytosolic and/or nuclear nucleic acids. The cell may comprise multiple copies of each nucleotide sequence of the plurality of nucleotide sequences, such as 1 to 1000, 2 to 1000, 5 to 1000, 10 to 1000, 20 to 1000, 50 to 1000, 100 to 1000, 150 to 1000, 200 to 1000, 250 to 1000, 1 to 500, 2 to 500, 5 to 500, 10 to 500, 20 to 1000, 50 to 500, 100 to 500, 150 to 500, or 200 to 500, 250 to 500, 1 to 400, 2 to 400, 5 to 400, 10 to 400, 20 to 400, 50 to 400, 100 to 400, 150 to 400, 200 to 400, or 250 to 400 copies of each nucleotide sequence. A cell may comprise 1, 2, 3, 4, 5, 6, 7, 8, 9, 10, 15, 20, 25, 30, 35, 40, 45, 50, 60, 70, 80, 90, 100, 120, 150, or 200 copies of each nucleotide sequence of a plurality of nucleotide sequences as described herein, *supra.* A cell may comprise at least 1, 2, 3, 4, 5, 6, 7, 8, 9, 10, 15, 20, 25, 30, 35, 40, 45, 50, 60, 70, 80, 90, 100, 120, 150, or 200 copies of each nucleotide sequence of a plurality of nucleotide sequences as described herein, *supra.* Each nucleotide sequence of a plurality of nucleotide sequences that originates from the same full-length nucleic acid may be present in a plurality of nucleic acid fragments at approximately the same copy number. Some nucleotide sequences are more or less stable than other nucleotide sequences, however, and thus, a cell may contain different nucleotide sequences of a plurality of nucleotide sequences at different copy numbers. A copy of a nucleotide sequence may occur, for example, on a single nucleic acid fragment of the plurality of nucleic acid fragments.

A cell may be a human cell. A cell may be a fibroblast or lymphocyte. A cell may be the cell of a cell line. A cell may be an adherent cell or a suspension cell.

A cell may be selected from the group consisting of 721, 293T, 721, A172, A253, A2780, A2780ADR, A2780cis, A431, A-549, BCP-1 cells, BEAS-2B, BR 293, BxPC3, Cal-27, CML T1, COR-L23, COR-L23/5010, COR-L23/CPR, COR-L23/R23, COV-434, DU145, DuCaP, EM2, EM3, FM3, H1299, H69, HCA2, HEK-293, HeLa, HL-60, HMEpC, HT-29, HUVEC, Jurkat, JY cells, K562 cells, KBM-7 cells, KCL22, KG1, Ku812, KYO1, LNCap, Ma-Mel, MCF-10A, MCF-7, MDA-MB-157, MDA-MB-231, MDA-MB-361, MG63, MONO-MAC 6, MOR/0.2R, MRC5, NCI-H69/CPR, NCI-H69/LX10, NCI-H69/LX20, NCI-H69/LX4, Peer, Raji, Saos-2 cells, SiHa, SKBR3, SKOV-3, T2, T-47D, T84, U373, U87, U937, VCaP, WM39, WT-49, and YAR cells.

A cell may be any cell available from the ATCC (*e.g.*, http://www.atcc.org). In certain embodiments, the cell is a mammalian cell, such as a human cell (*e.g.*, available from the ATCC). The cell may be a cell from any of the National Institute of General Medical Sciences (NIGMS) Human Genetic Cell Repository cell lines available from the Coriell Institute for Medical Research (https://catalog.coriell.org/1/NIGMS), such as a cell line from the "Apparently Healthy" collection. The cell may be may be a fibroblast, lymphoblast, or lymphocyte. The cell may be transformed, e.g., with Epstein-Barr virus. The cell may be an immortalized cell. For example, the cell may be an immortalized lymphocyte, such as an immortalized B lymphocyte. The cell may be an Epstein-Barr virus-transformed lymphocyte, such as an Epstein-Barr virus-transformed B lymphocyte. The cell may be GM12878 (*see* Zook, J.M. et al., Nature Biotechnology 32:246 (2014)). The cell may be GM12878, GM24149, GM24143, GM24385, GM24631, GM24694, or GM24695 (*see* Zook, J.M. et al., Scientific Data 3:160025 (2016)). In certain embodiments, the cell is GM24385.

A cell may be a bacterial, yeast, insect, mouse, rat, hamster, dog, or monkey cell, *e.g.,* for cloning or validating a construct. For example, the cell may be *E. coli* or *Saccharomyces cerevisiae, e.g.,* for cloning a nucleic acid of the disclosure.

In some aspects, the disclosure relates to composition comprising a first plurality of cells and a second plurality of cells (referred to as a "composition comprising cells"). The first plurality of cells consist of cells that comprise 5 to 500 copies of the nucleic acid. The second plurality of cells consist of cells that do not comprise the nucleic acid. The first plurality of cells and second plurality of cells are human cells, such as immortalized lymphocytes, such as GM24385 cells. The cells of the first plurality and the second plurality are be admixed in the composition. The ratio of the number of cells of the first plurality to the number of cells of the second plurality is about 1:1 to about 1:10,000, such as about 1:2 to about 1:2000, or about 1:10 to about 1:1000 in the composition. The ratio may depend in part on either the average copy number of the nucleic acid in the first plurality of cells or the average copy number of the nucleotide sequences of the plurality of nucleotide sequences in the first plurality of cells. The ratio of the number of cells of the first plurality of cells to the number of cells of the second plurality of cells may be adjusted, for example, such that the composition comprises about 0.01 copies of the nucleic acid (or about 0.01 copies of each nucleotide sequence of the plurality of nucleotide sequences) to about 100 copies of the nucleic acid (or about 100 copies of each nucleotide sequence of the plurality of nucleotide sequences) per cell of the composition. The ratio may be adjusted such that the composition comprises about 0.1 to about 50 copies, about 0.5 to about 20 copies, or about 1 to about 10 copies of the nucleic acid per cell of the composition (or about 0.1 to about 50 copies, about 0.5 to about 20 copies, or about 1 to about 10 copies of each nucleotide sequence of the plurality of nucleotide sequences per cell of the composition).

A cell, plurality of cells, or composition comprising cells may be fixed.
In certain embodiments, a cell, plurality of cells, or composition comprising cells is fixed with formalin. A cell, plurality of cells, or composition comprising cells may be fixed with glutaraldehyde, ethanol, methanol, acetone, methyl benzoate, xylene, acetic acid, picrate, HOPE fixative, osmium tetroxide, and/or uranyl acetate.

A cell, plurality of cells, or composition comprising cells may be dehydrated, e.g., using ethanol or an organic solvent.

A cell, plurality of cells, or composition comprising cells may be embedded in paraffin. For example, a cell, plurality of cells, or composition comprising cells may be fixed in formalin and embedded in paraffin. A cell, plurality of cells, or composition comprising cells may be embedded in paraffin and sectioned. A cell, plurality of cells, or composition comprising cells may be mounted on a slide.

In some aspects, the disclosure relates to a paraffin section comprising a plurality of cells or composition comprising cells. The paraffin section may comprise 1 to about 1,000,000 cells, such as about 10 to about 100,000 cells, about 50 to about 50,000 cells, about 100 to about 10,000 cells, about 500 to about 5,000 cells, about 200 to about 2000 cells, about 100 to about 1000 cells, or about 50 to about 1000 cells. The paraffin section may be about 1 µm to about 50 µm thick, such as about 2 µm to about 25 µm thick, or about 5 µm to about 20 µm thick. The paraffin section may be about 2, 3, 4, 5, 6, 7, 8, 9, 10, 11, 12, 13, 14, 15, 16, 17, 18, 19, or 20 µm thick. The paraffin section may be about 1 mm to about 100 mm in length, width, or diameter, such as about 5 mm to about 50 mm, or about 10 mm to about 40 mm. For example, a paraffin section may be about 5 mm to about 50 mm in length, about 5 mm to about 50 mm in width, and about 5 µm to about 20 µm thick. A paraffin section may be about 5 mm to about 50 mm in diameter and about 5 µm to about 20 µm thick.

A cell, plurality of cells, or composition comprising cells may be present in a cell pellet. A cell, plurality of cells, or composition comprising cells may be suspended in blood plasma, such as a mammalian blood plasma. In certain embodiments, a cell, plurality of cells, or composition comprising cells may be suspended in human blood plasma or a solution designed to replicate human blood plasma.

In some aspects, the disclosure relates to a method for making a biological reference material, comprising transfecting a plurality of cells with a nucleic acid described herein.

A method may comprise fixing a plurality of cells or composition comprising cells. For example, the method may comprise fixing a plurality of cells or composition comprising cells with formalin. A method may comprise fixing a plurality of cells or composition comprising cells with glutaraldehyde, ethanol, methanol, acetone, methyl benzoate, xylene, acetic acid, picrate, HOPE fixative, osmium tetroxide, and/or uranyl acetate.

A method may comprise embedding a plurality of cells or composition comprising cells in paraffin. A method may comprise sectioning paraffin-embedded cells. A method may comprise mounting a plurality of cells on a slide, e.g., paraffin-embedded cells or cells that are not embedded in paraffin.

A method may comprise mounting a plurality of cells or composition comprising cells on a slide.

In some aspects, the disclosure relates to a biological reference material comprising a plurality of cells as described herein and paraffin, wherein the plurality of cells are fixed and embedded in the paraffin. The biological reference material may be a section of cells embedded in paraffin, *e.g.,* wherein the section is about 1 µm to about 50 µm thick, such as about 5 µm to about 20 µm thick.

A biological reference material may further comprise untransfected cells, e.g., wherein the untransfected cells do not comprise the nucleic acid. In certain embodiments, the untransfected cells are the same species as the cells of the plurality, *e.g.,* the untransfected cells may be from the same source *(e.g.,* cell line) as the cells of the plurality. The ratio of cells of the plurality of cells to untransfected cells may be about 4:1 to about 1:10,000, such as about 1:1 to about 1:5,000, about 1:1 to about 1:1000, about 1:10 to about 1:1000, or about 1:50 to about 1:500. The ratio of cells of the plurality of cells to untransfected cells may be about 45:55, about 50:50, about 55:45, about 1:1, about 1:2, about 1:3, about 1:4, about 1:5, about 1:6, about 1:7, about 1:8, about 1:9, about 1:10, about 1:20, about 1:25, about 1:50, about 1:100, about 1:200, about 1:250, about 1:500, or about 1:1000.

In some embodiments, the ratio of the copy number of the nucleic acid to the copy number of cell genomes in the biological reference material is about 10:1 to about 1:10,000, such as about 5:1 to about 1:1000, about 2:1 to about 1:100, about 1:1 to about 1:50, or about 1:2 to about 1:20. In general, each genome contains two copies of a gene *(e.g.,* for genes occurring on diploid chromosomes, such as autosomes). The copy number of a nucleic acid to the copy number of a gene in the cell genome in the biological reference material may be about 10:1 to about 1:10,000, such as about 5:1 to about 1:1000, about 1:1 to about 1:100, about 1:2 to about 1:50, or about 1:4 to about 1:40. Thus, the ratio of a genotype of a nucleic acid to the copy number of a gene in the cell genome that is associated with the genotype *(e.g.,* the wild type allele) in the biological reference material may be about 10:1 to about 1:10,000, such as about 5:1 to about 1:1000, about 1:1 to about 1:100, about 1:2 to about 1:50, or about 1:4 to about 1:40.

In some aspects, the disclosure relates to a biological reference material comprising a plurality of cells as described herein and a liquid, such as saline, phosphate-buffered saline, or blood plasma, such as a mammalian blood plasma. A cell, plurality of cells, or composition comprising cells of a biological reference material may be suspended in plasma, such as human blood plasma or a solution designed to replicate human blood plasma.

A biological reference material may be a cell pellet, *e.g.,* made by centrifuging a plurality of cells or composition comprising cells as described herein.

In some aspects, the disclosure relates to a composition comprising a purified nucleic acid and an aqueous buffer, wherein the purified nucleic acid is isolated from a biological reference material as described herein. The composition may comprise a buffer, such as tris buffer (*i.e.*, tris(hydroxymethyl)aminomethane or a salt thereof). The composition may comprise a chelating agent, such as ethylenediaminetetraacetic acid, or a salt thereof. The composition may comprise trace amounts of formaldehyde and/or paraffin, although the composition may be free of formaldehyde and paraffin.

### EXEMPLIFICATION

### Example 1. Nucleic acid design for oncology targets

A nucleic acid (DNA) was designed comprising 16 nucleotide sequences, wherein each nucleotide sequence comprises one of the 16 genotypes listed in Table 4. Each of these genotypes is associated with a neoplastic solid tumor. Each of the nucleotide sequences is a subsequence of a human gene that contains the genotype. Each nucleotide sequence is about 600 nucleotides long. Each genotype occurs near the middle of a subsequence, *i.e.,* each genotype occurs about 300 nucleotides from both the 5' end and the 3' end of a nucleotide sequence.

**Table 4**

| Genes and genotypes of the nucleic acid comprising the sequence set forth in SEQ ID NO: 1. | | | | |
|---|---|---|---|---|
| **Gene ID** | **COSMIC Identifier** | **Mutation Type** | **Genotype (HGVS Nomenclature)** | **Location in SEQ ID NO:1** |
| TP53 | COSM10648 | SNV | c.524G>A | 1 to 610 |
| KIT | COSM1257 | SNV | c.1679T>A | 611 to 1226 |
| RET | COSM965 | SNV | c.2753T>C | 1227 to 1838 |
| PIK3CA | COSM775 | SNV | c.3140A>G | 1839 to 2444 |
| PIK3CA | COSM763 | SNV | c.1633G>A | 2445 to 3050 |
| PDGFR A | COSM736 | SNV | c.2525A>T | 3051 to 3660 |
| NRAS | COSM584 | SNV | c.182A>G | 3661 to 4270 |
| KRAS | COSM521 | SNV | c.35G>A | 4271 to 4878 |
| ERBB2 | COSM682/20959 | Large Insertion | c.2324_2325ins12 | 4879 to 5502 |
| EGFR | COSM6240 | SNV | c.2369C>T | 5503 to 6114 |
| EGFR | COSM6225 | Large Deletion | c.2236_2250de115 | 6115 to 6711 |
| EGFR | COSM6224 | SNV | c.2573T>G | 6712 to 7326 |
| CTNNB1 | COSM5664 | SNV | c.121A>G | 7327 to 7936 |
| BRAF | COSM476 | SNV | c.1799T>A | 7937 to 8544 |
| PTEN | COSM5809 | Deletion in homopolymer | c. 800delA | 8545 to 9153 |
| IDH1 | COSM28747 | SNV | c.394C>T | 9154 to 9854 |

The sequence of the nucleic acid is set forth in SEQ ID NO: 1. Nucleotides 8545 to 9153 of SEQ ID NO: 1 are a subsequence of human gene PTEN and correspond to the genotype mutation c.800delA in gene PTEN. Nucleotides 7937 to 8544 of SEQ ID NO: 1 are a subsequence of human gene BRAF and correspond to the genotype mutation c. 1799T>A in gene BRAF. Nucleotides 7327 to 7936 of SEQ ID NO: 1 are a subsequence of human gene CTNNB1 and correspond to the genotype mutation c.121A>G in gene CTNNB1. Nucleotides 6712 to 7326 of SEQ ID NO: 1 are a subsequence of human gene EGFR and correspond to the genotype mutation c.2573T>G in gene EGFR. Nucleotides 6115 to 6711 of SEQ ID NO:1 are a subsequence of human gene EGFR and correspond to the genotype mutation c.2236_2250del15 in gene EGFR. Nucleotides 5503 to 6114 of SEQ ID NO:1 are a subsequence of human gene EGFR and correspond to the genotype mutation c.2369C>T in gene EGFR. Nucleotides 4879 to 5502 of SEQ ID NO:1 are a subsequence of human gene ERBB2 and correspond to the genotype mutation c.2324_2325ins12 in gene ERBB2. Nucleotides 4271 to 4878 of SEQ ID NO:1 are a subsequence of human gene KRAS and correspond to the genotype mutation c.35G>A in gene KRAS. Nucleotides 3661 to 4270 of SEQ ID NO:1 are a subsequence of human gene NRAS and correspond to the genotype mutation c.182A>G in gene NRAS. Nucleotides 3051 to 3660 of SEQ ID NO: 1 are a subsequence of human gene PDGFRA and correspond to the genotype mutation c.2525A>T in gene PDGFRA. Nucleotides 2445 to 3050 of SEQ ID NO:1 are a subsequence of human gene PIK3CA and correspond to the genotype mutation c.1633G>A in gene PIK3CA. Nucleotides 1839 to 2444 of SEQ ID NO:1 are a subsequence of human gene PIK3CA and correspond to the genotype mutation c.3140A>G in gene PIK3CA. Nucleotides 1227 to 1838 of SEQ ID NO:1 are a subsequence of human gene RET and correspond to the genotype mutation c.2753T>C in gene RET. Nucleotides 1 to 610 of SEQ ID NO:1 are a subsequence of human gene TP53and correspond to the genotype mutation c.524G>A in gene TP53. Nucleotides 611 to 1226 of SEQ ID NO:1 are a subsequence of human gene KIT and correspond to the genotype mutation c.1679T>A in gene KIT. Nucleotides 9154 to 9854 of SEQ ID NO:1 are a subsequence of human gene IDH1 and correspond to the genotype mutation c.394C>T in gene IDH1.

The nucleic acid was used to transfect GM24385 cells, which is a well-characterized human cell line. The copy number of the nucleic acid in the transfected GM24382 cells was measured, and the transfected cells were diluted with non-transfected GM24382 cells to arrive at compositions comprising an allele frequency of about 15%, about 7%, or about 4%. The allele frequency is defined as the frequency of a genotype on the nucleic acid to the frequency of the wild type allele in the genomes of the GM24382 cells in the composition. Thus, the allele frequency of an EGFR genotype is the same as the frequency of a TP53 genotype in the composition, even though the nucleic acid comprises three EGFR nucleotide sequences and one TP53 nucleotide sequence.

Three compositions of cells comprising allele frequencies of either about 15%, about 7%, or about 4% were fixed with formalin, embedded in paraffin, and sectioned into a 10 µm curl (Figure 1). DNA was extracted from sections corresponding to different allele frequencies and the allele frequencies were verified by digital PCR. Figure 2 shows that eight genotypes were detected by digital PCR with observed allele frequencies corresponding to the actual allele frequencies. DNA was also extracted from formalin-fixed, paraffin-embedded sections, amplified using Ion AmpliSeq^{™} Cancer Hotspot Panel v2, and sequenced using an Ion Torrent. Figure 3 shows that fifteen genotypes were detected by next-generation sequencing with observed allele frequencies corresponding to actual allele frequencies.

### Example 2, Nucleic acid design for inheritable oncology targets

A number of different inheritable genetic polymorphisms correlate with an increased incidence of cancer, including the mutations listed in Table 5.

**Table 5. Selected mutations that correlate with cancer.**

| **Gene** | **Mutation Type** | **HGVS Nomenclature** | **Variant Length** |
|---|---|---|---|
| MSH2 | SNV | NM 000251.2:c.942+3A>T | 1 |
| | indel | NM_000251.2:c.1662-12_1677del | 28 |
| MSH6 | delins | NM_000179.2:c.2056_2060delinsCTTCTACCTCAAAAA (SEQ ID NO: 13) | 15 |
| | delins | NM_000179.2:c.2308_2312delGGTAAinsT | 6 |
| | delins | NM_000179.2:c.2641delGinsAAAA | 5 |
| | indel | NM_000179.2:c.3163_3164insG | 1 |
| MI,H1 | Indel | NM_000249.3:c.1852_1854delAAG | 4 |
| | delins | NM_000249.3:c.232_243delinsATGTAAGG | 12 |
| PMS2 | SNV | NM_000535.5:c.2445+1G>C | 1 |
| | | NM_000535.5:c.2243_2246delAGAA; | |
| | indel | NM_000535.5:c.2253T>C | 4 |
| | Indel | NM_000535.5:c.861_864delACAG | 5 |
| CDKN2 | Indel | NM_000077.4:c.9_32dup24 | 25 |
| BRCA2 | Indel | NM_000059.3:c.9203del126 | 126 |
| | Indel | NM_000059.3:c.1310_1313delAAGA | 5 |
| | Indel | NM_000059.3:c.1813dupA | 2 |
| | Indel | NM_000059.3:c.9342_9343insAluY | 343 |
| BRCA1 | indel | NM_007294.3:c.5266_5267insC | 1 |
| | Indel | NM_007294.3:c.5177_5180delGAAA | 5 |
| | indel | NM_007294.3:c.3756_3759delGTCT | 4 |
| | Indel | NM_007294.3:c.3481_3491delGAAGATACTAG (SEQ ID NO: 14) | 12 |
| | SNV | NM_007294.3:c.3113A>G | 1 |
| | indel | NM_007294.3:c.3084_3094delTAATAACATTA (SEQ ID NO: 15) | 11 |
| | delins | NM_007294.3:c.2834_2836delinsC | 6 |
| | indel | NM_007294.3:c.68_69delAG | 2 |

Two plasmids were designed for use in manufacturing reference materials that may serve as controls. A first plasmid comprises mutation c.942+3A>T to gene MSH2 (mutS homolog 2); mutations c.2056 _2060delinsCTTCTACCTCAAAAA (SEQ ID NO: 13), c.2308_2312delGGTAAinsT, c.2641delGinsAAAA, and c.3163_3164insG to gene MSH6 (mutS homolog 6), which each occur in the same nucleotide sequence; mutation c,1852_1854delAAG to gene MLH1 (mutL homolog 1); mutations c.2445+1G>C, c.2243_2246delAGAA to gene PMS2(PMS1 homolog 2, mismatch repair system component), and c.861_864delACAG to gene PMS2, which each occur in different nucleotide sequences; and mutation c.9_32dup24 to gene CDKN2A (cyclin dependent kinase inhibitor 2A) (Figure 4). A second plasmid comprises mutations c.8975_9100del126, c.1310_1313delAAGA, c.1813dupA, and c.9342_9343insAluY to gene BRCA2 (breast cancer 2) and mutations c.5266_5267insC, c.5177_5180delGAAA, c.3756_3759delGTCT, c.3481_3491delGAAGATACTAG (SEQ ID NO: 14), c.3113A>G, c.3084_3094delTAATAACATTA (SEQ ID NO: 15), c.2834_2836delinsC, and c.68_69delAG to gene BRCA1 (breast cancer 1) (Figure 5).

Slightly different plasmids were designed for use in manufacturing reference materials that may serve as controls. A first plasmid comprises mutation c.942+3A>T to gene MSH2 (mutS homolog 2); mutation c.1662-12 1677del to gene MSH2; mutations c.2056 _2060delinsCTTCTACCTCAAAAA (SEQ ID NO: 13), c.2308_2312delGGTAAinsT, c.2641delGinsAAAA, and c.3163_3164insG to gene MSH6 (mutS homolog 6), which each occur in the same nucleotide sequence; mutation c.232_243delinsATGTAAGGto gene MI,H1 (mutL homolog 1), mutation c.1852_1854delAAG to gene MI,H1; and mutations c.2445+1G>C, c.2243_2246delAGAA to gene PMS2 (PMS1 homolog 2, mismatch repair system component); and c.861_864delACAG to gene PMS2 (Figure 6; SEQ ID NO:2). A second plasmid comprises mutation c.9_32dup24 to gene CDKN2A (cyclin dependent kinase inhibitor 2A); mutations c.8975_9100del126, c.1310_1313delAAGA, c.1813dupA, and c.9342_9343insAluY to gene BRCA2 (breast cancer 2); and mutations c.5266_5267insC, c.5177_5180delGAAA, c.3756_3759delGTCT, c.3481_3491delGAAGATACTAG (SEQ ID NO: 14), c.3113A>G, c.3084_3094delTAATAACATTA (SEQ ID NO: 15), c.2834_2836delinsC, and c.68_69delAG to gene BRCA1 (breast cancer 1) (Figure 7; SEQ ID NO:3).

### Example 3. Nucleic acid design for cardiomyopathy targets

A multiplex nucleic acid was designed for detecting ten mutations associated with cardiomyopathy (Table 6; Figure 8). Approximately 1000 base pairs of each gene target were selected so that the mutation resided near the middle of each sequence. These ~1000 base pair segments were synthesized sequentially in a generic cloning vector. NotI restriction sites were placed at both the 5' end and 3' end of the construct to separate the cardiomyopathy gene construct from the vector backbone. The sequence used as the insert is shown in SEQ ID NO:4. Each gene target was also synthesized in a separate plasmid as a control (10 individual plasmids, each plasmid containing a ~1000 base pair insert with a single cardiomyopathy variant).

**Table 6. Gene targets for cardiomyopathy multiplex reference materials**

| **Name** | **Mutation Type** | **Gene** |
|---|---|---|
| MYBPC3 c.1504C>T plasmid | SNV | myosin binding protein C, cardiac |
| MYBPC3 c.2373_2374insG plasmid | Small insertion | |
| MYBPC3 c.3628-41_3628-17del plasmid | Large Deletion (in repetitive region) | |
| MYH7 c.1988G>A plasmid | SNV | myosin, heavy chain 7, cardiac muscle, beta |
| MYH7 c.1357C>T plasmid | SNV | |
| MYH7 c.1750G>C plasmid | SNV | |
| TNNI3 c.532_534delAAG plasmid | Small Deletion | troponin I type 3 (cardiac) |
| TNNI3 c.575G>A plasmid | SNV | |
| TNNT2 c.487_489delGAG plasmid | Deletion in highly repetitive region | troponin T type 2 (cardiac) |
| TPM1 c.574G>A plasmid | SNV | tropomyosin 1 (alpha) |

For a pilot experiment, the constructs were not incorporated into cells, but instead the DNA was assayed to determine if the synthetic, multiplex design was compatible with the NGS testing methods.

Plasmids were linearized and quantified by droplet digital PCR, both for the single plasmid containing 10 gene targets as well as the 10 plasmids, each containing a single gene target. The 10 individual plasmids were pooled together in an equimolar plasmid pool. Genomic DNA from the GM24385 reference cell line was extracted using the Qiagen Puregene manual method and quantitated using multiple droplet digital PCR assays. The linearized plasmid (or plasmid pool) was mixed together with genomic DNA at three allelic frequencies: 45%, 50% and 55%. Since the plasmid contained mutant sequences and the genomic DNA contained wild type sequences, the resulting mix should have allele frequency similar to a target dilution. (50% is heterozygote allele frequency.) Table 7 summarizes the lot numbers that were prepared.

**Table 7. Summary of pilot lots**

| **Lot Number** | **Description** | **Target AF** |
|---|---|---|
| 102304 | Mixture of 10 different plasmids with GM24385 gDNA | 45% |
| 102305 | Mixture of 10 different plasmids with GM24385 gDNA | 50% |
| 102306 | Mixture of 10 different plasmids with GM24385 gDNA | 55% |
| 102315 | Multiplex plasmid (1 construct x 10 mutations) and GM24385 gDNA | 50% |

Two TaqMan-based real time PCR assays were used to assess the nucleic acid mixtures on a BioRad QX-200 droplet digital PCR system. The assay targets as well as primer and probe sequences are shown in Table 8. Droplet digital PCR was performed in triplicate. Results for lot 102315, which contains the single plasmid with 10 targets, are shown in Figure 9 and 10.

**Table 8: Primer and probe sequences for two cardiomyopathy targets**

| | | | |
|---|---|---|---|
| Assay Name rs36212006 | MYBPC3 c.3628-41_3628-17del | Forward Primer: AACCAGGAAATCTTGGGCTATA | SEQ ID NO:5 |
| | | Reverse Primer: ACACAGATGTGTCTCCCTG | SEQ ID NO:6 |
| | | Mutant Probe: (AGGTCCCCTCTCT+T+T+ACC): | SEQ ID NO:7 |
| | | Wild Type Probe: TGGCTTCCC+TCCC+TCTC | SEQ ID NO:8 |
| rs397516351 | TNNI3 c.532_534delAAG | Forward Primer: TAAGGAGTCCCTGGACCTG | SEQ ID NO:9 |
| | | Reverse Primer: GCCTTAGCCCACACTCAC | SEQ ID NO:10 |
| | | Mutant Probe: (AGG TGA +AG+G +A+GG): | SEQ ID NO:11 |
| | | Wild Type Probe (AG+GT+GAA+G+A+A+GG): | SEQ ID NO:12 |

The multiplexed plasmid (10 variants in one plasmid) performed similarly to the pool of individual plasmids for both ddPCR (data not shown) and for next generation sequencing (Figures 11 and 12). Sequencing was performed in a whole exome sequencing assay using the SureSelectXT Target Enrichment System for the Illumina Paired-End Multiplexed Sequencing Library and performed on an MiSeq Instrument. Substitution mutations were identified at the expected frequency in the multiplex sample (Figures 11 and 12). Indel mutations were identified at a lower-than-expected frequency in each sample (Figures 11 and 12), consistent with the current limitations of next generation sequencing technology. In particular, the 3628-41_3628-17del mutation displayed a considerably lower allele frequency than the other variants. Large deletions are known to be difficult for next-generation sequencing alignment and detection, and multiplexed reference materials may be particularly useful for addressing this problem.

## Claims

1. A nucleic acid, comprising a plurality of nucleotide sequences, wherein each nucleotide sequence of the plurality comprises a genotype that is associated with a disease or condition; each nucleotide sequence is 600-10,000 base pairs long; and the plurality of nucleotide sequences comprises nucleotide sequences from at least 2 different chromosomes.

2. The nucleic acid of claim 1, wherein:
(a) the genotype of each nucleotide sequence of the plurality is a somatic mutation or an inheritable mutation; and/or
(b) the genotype of each nucleotide sequence of the plurality is a single nucleotide polymorphism, point mutation, premature stop codon, trinucleotide repeat, translocation, somatic rearrangement, allelomorph, single nucleotide variant, insertion or deletion ("indel"), regulatory variant, or gene fusion; and/or
(c) the plurality of nucleotide sequences comprises nucleotide sequences from at least 3 different chromosomes; and/or
(d) the nucleic acid is DNA; and/or
(e) the plurality of nucleotide sequences comprises nucleotide sequences from at least 3 or at least 5 different chromosomes; and/or
(f) each nucleotide sequence of the plurality is a subsequence of a gene or regulatory region thereof,
optionally wherein each nucleotide sequence of the plurality is a subsequence of a gene, optionally wherein each nucleotide sequence of the plurality is a subsequence of an exon, optionally wherein the genotype of each nucleotide sequence of the plurality causes a mutation, deletion, or insertion of at least one amino acid to the amino acid sequence encoded by the exon; and/or
(g) the nucleic acid is as set out in (e) or (f) and
each gene is a human gene; and/or
(h) each nucleotide sequence of the plurality is a human nucleotide sequence.

3. The nucleic acid of claim 1 or 2, wherein:
(a) the genotype of each nucleotide sequence of the plurality of nucleotide sequences
is associated with a neoplasm, optionally a solid tumour; and/or
(b) the genotype of each nucleotide sequence of the plurality of nucleotide sequences is associated with a neoplasm which is lung cancer, lymphoid cancer, acute lymphoid leukemia, acute myeloid leukemia, chronic myelogenous leukemia, thyroid cancer, stomach cancer, large intestine cancer, urinary tract cancer, central nervous system cancer, kidney cancer, breast cancer, and/or soft tissue cancer, optionally a solid tumour; and/or
(c) the genotype of each nucleotide sequence of the plurality of nucleotide sequences is associated with a neoplasm in (a) or (b) and each nucleotide sequence of the plurality of nucleotide sequences is a subsequence of a gene selected from the group consisting of ABL1, AKT1, AKT2, AKT3, ALK, APC, AR, AR1D1A, ARAF, ARHGAP5, ATM, ATR, BCL2, BCR, BRAF, BRC42, BRCA1, BRCA2, BRIP1, CBFB, CCND1, CCND2, CCNE1, CDH1, CDK4, CDK6, CDKN2A, CDKN2B, CSF 1R, CTNNB1, DDR2, DNMT3A, EGFR, ERBB2 ("HER2"), ERBB3, ERBB4 ("HER4"), ERCC1, ESR1, ETV1, ETV4, ETV6, EWSR1, EZH2, FANCA, FANCC, FANCD2, FANCE, FANCF, FANCG, FANCL, FBXW7, FGFR1, FGFR2, FGFR3, FLT3, FOXL2, GABRA6, GABRG2, GATA3, GNA11, GNAQ, GNAS, HNF1A, HPAS, HRAS, IDH1, IDH2, IHD2, JAK2, JAK3, KDR, KIT, KMT2A, KRAS, MAP2K1, MAP2K2, MECOM, MET, MKL1, MLH1, MLL, MPL, MSH2, MSH6, MTOR, MYC, MYCN, MYD88, NF1, NF2, NFE2L2, NOTCH1, NPM1, NRAS/CSDE1, NTRK1, NUP214, PALB2, PARP1, PARP2, PDGFRA, PDGFRB, PICALM, PIK3CA, PMS2, PTCH1, PTEN, PTPN11, RAD51, RAFT, RARA, RB1, RET, RHEB, RHOA, RIT1, ROS1, RUNX1, RUNX1T1, SMAD4, SMARCB1, SMO, SRC, STK11, TAL1, TCF3, TERT, TMPRSS2, TP53, TSC1, TSC2, and VHL; and/or
(d) the genotype of each nucleotide sequence of the plurality of nucleotide sequences is associated with a neoplasm as in (a) or (b)
and each nucleotide sequence of the plurality of nucleotide sequences is a subsequence of a gene selected from the group consisting of AKT1, AKT2, AKT3, APC, ARHGAP5, ATM, ATR, BRAF, CTNNB1, DNMT3A, EGFR, ERBB2, ERCC1, EZH2, FBXW7, FGFR3, FLT3, GABRA6, GABRG2, GNAQ, GNAS, IDH1, IDH2, JAK2, KIT, KRAS, MET, MLH1, MPL, MSH2, MTOR, MYD88, NF1, NPM1, NRAS/CSDE1, PARP1, PARP2, PDGFRA, PIK3CA, PTEN, PTPN11, RAD51, RB1, RET, SMAD4, TP53, and VHL, optionally wherein the genotype of each nucleotide sequence of the plurality of nucleotide sequences is selected from the group consisting of mutation 145G>A to gene AKT1, mutation c.49G>A to gene AKT1, mutation c.268G>T to gene AKT2, mutation c.371A>T to gene AKT3, mutation c.4248delC to gene APC, mutation c.4348C>T to gene APC, mutation c.4666_4667insA to gene APC, mutation c. 1864G>A to gene ARHGAP5, mutation c.1058_1059delGT to gene ATM, mutation c.5557G>A to gene ATM, mutation c.3790_3796delATAAAAG to gene ATR, mutation c.1799T>A to gene BRAF, mutation c.121A>G to gene CTNNB 1, mutation c.2644C>T to gene DNMT3A, mutation c.2236_2250del15 to gene EGFR, mutation c.2310_2311insGGT to gene EGFR, mutation c.2369C>T to gene EGFR, mutation c.2573T>G to gene EGFR, mutation c.2324_2325ins12 to gene ERBB2, mutation c.287C>A to gene ERCC1, mutation c.1937A>T to gene EZH2, mutation c.1394G>A to gene FBXW7, mutation c.746C>G to gene FGFR3, mutation c.2503G>T to gene FLT3, mutation c.763G>C to gene GABRA6, mutation c.1355A>G to gene GABRG2, mutation c.626A>C to gene GNAQ, mutation c.601C>T to gene GNAS, mutation c.394C>T to gene IDH1, mutation c.515G>A to gene IDH2, mutation c.419G>A to gene IDH2, mutation c.1849G>T to gene JAK2, mutation c.2447A>T to gene KIT, mutation c.1679T>A to gene KIT, mutation c.35G>A to gene KRAS, mutation c.3757T>G to gene MET, mutation c.1151T>A to gene MLH1, mutation c.1544G>T to gene MPL, mutation c.2250delG to gene MSH2, mutation c.2359_2360delCT to gene MSH2, mutation c.2664A>T to gene MTOR, mutation c.794T>C to gene MYD88, mutation c.2987_2988insAC to gene NF1, mutation c.4084C>T to gene NF1, mutation c.7501delG to gene NF1, mutation c.863_864insTCTG to gene NPM1, mutation c.182A>G to gene NRAS, mutation c.2738delG to gene PARP1, mutation c.398A>C to gene PARP2, mutation c.1694_1695insA to gene PDGFRA, mutation c.2525A>T to gene PDGFRA, mutation c.1633G>A to gene PIK3CA, mutation c.3140A>G to gene PIK3CA, mutation c.3204_3205insA to gene PIK3CA, mutation c.388C>T to gene PTEN, mutation c.741_742insA to gene PTEN, mutation c.800delA to gene PTEN, mutation c.226G>A to gene PTPN11, mutation c.433C>T to gene RAD51, mutation c.958C>T to gene RB1, mutation c.2753T>C to gene RET, mutation c.1394_1395insT to gene SMAD4, mutation c.818G>A to gene TP53, mutation c.743G>A to gene TP53, mutation c.723delC to gene TP53, mutation c.524G>A to gene TP53, mutation c.263delC to gene TP53, and mutation c.426_429delTGAC to gene VHL; and/or
(e) the nucleic acid is as set out in part (d) and
each nucleotide sequence of the plurality of nucleotide sequences is a subsequence from a gene is selected from the group consisting of BRAF, CTNNB1, EGFR, ERBB2, IDH1, KIT, KRAS, NRAS/CSDE1, PDGFRA, PIK3CA, PTEN, RET, and TP53; and/or
(f) the nucleic acid is as set out in part (d) or (e) and
the plurality of nucleotide sequences comprises at least 5 nucleotide sequences;
each nucleotide sequence of the plurality of nucleotide sequences is a subsequence of a human gene; and
each gene is selected from the group consisting of BRAF, CTNNB1, EGFR, ERBB2, IDH1, KIT, KRAS, NRAS/CSDE1, PDGFRA, PIK3CA, PTEN, RET, and TP53; and/or
(g) the nucleic acid is as set out in part (e) or (f) and
the genotype of each nucleotide sequence of the plurality of nucleotide sequences is selected from the group consisting of mutation c.1799T>A to gene BRAF, mutation c.121A>G to gene CTNNB1, mutation c.2236_2250deI15 to gene EGFR, mutation c.2369C>T to gene EGFR, mutation c.2573T>G to gene EGFR, mutation c.2324_2325ins12 to gene ERBB2, mutation c.394C>T to gene IDH1, mutation c.1679T>A to gene KIT, mutation c.35G>A to gene KRAS, mutation c.182A>G to gene NRAS/CSDE1, mutation c.2525A>T to gene PDGFRA, mutation c.1633G>A to gene PIK3CA, mutation c.3140A>G to gene PIK3CA, mutation c.800delA to gene PTEN, mutation c.2753T>C to gene RET, and mutation c.524G>A to gene TP53; and/or
(h) the nucleic acid is as set out in part (g) and each nucleotide sequence of the plurality of nucleotide sequences has at least 95% sequence homology with the nucleotide sequence spanning nucleotides 8545 to 9153 of SEQ ID NO:1 (mutation c.800delA to gene PTEN), nucleotides 7937 to 8544 of SEQ ID NO:1 (mutation c.1799T>A to gene BRAF), nucleotides 7327 to 7936 of SEQ ID NO:1 (mutation c.121A>G to gene CTNNB 1), nucleotides 6712 to 7326 of SEQ ID NO:1 (mutation c.2573T>G to gene EGFR), nucleotides 6115 to 6711 of SEQ ID NO:1 (mutation c.2236_2250deI15 to gene EGFR), nucleotides 5503 to 6114 of SEQ ID NO:1 (mutation c.2369C>T to gene EGFR), nucleotides 4879 to 5502 of SEQ ID NO:1 (mutation c.2324_2325ins12 to gene ERBB2), nucleotides 4271 to 4878 of SEQ ID NO:1 (mutation c.35G>A to gene KRAS), nucleotides 3661 to 4270 of SEQ ID NO:1 (mutation c.182A>G to gene NRAS), nucleotides 3051 to 3660 of SEQ ID NO:1 (mutation c.2525A>T to gene PDGFRA), nucleotides 2445 to 3050 of SEQ ID NO:1 (mutation c.1633G>A to gene PIK3CA), nucleotides 1839 to 2444 of SEQ ID NO:1 (mutation c.3140A>G to gene PIK3CA), nucleotides 1227 to 1838 of SEQ ID NO:1 (mutation c.2753T>C to gene RET), nucleotides 1 to 610 of SEQ ID NO:1 (mutation c.524G>A to gene TP53), nucleotides 611 to 1226 of SEQ ID NO:1 (mutation c.1679T>A to gene KIT), or nucleotides 9154 to 9854 of SEQ ID NO:1 (mutation c.394C>T to gene IDH1), optionally wherein each nucleotide sequence of the plurality of nucleotide sequences has the nucleotide sequence spanning nucleotides 8545 to 9153 of SEQ ID NO:1 (mutation c.800delA to gene PTEN), nucleotides 7937 to 8544 of SEQ ID NO:1 (mutation c.1799T>A to gene BRAF), nucleotides 7327 to 7936 of SEQ ID NO:1 (mutation c.121A>G to gene CTNNB 1), nucleotides 6712 to 7326 of SEQ ID NO:1 (mutation c.2573T>G to gene EGFR), nucleotides 6115 to 6711 of SEQ ID NO:1 (mutation c.2236_2250deI15 to gene EGFR), nucleotides 5503 to 6114 of SEQ ID NO:1 (mutation c.2369C>T to gene EGFR), nucleotides 4879 to 5502 of SEQ ID NO:1 (mutation c.2324_2325ins12 to gene ERBB2), nucleotides 4271 to 4878 of SEQ ID NO:1 (mutation c.35G>A to gene KRAS), nucleotides 3661 to 4270 of SEQ ID NO:1 (mutation c. 182A>G to gene NRAS), nucleotides 3051 to 3660 of SEO ID NO:1 (mutation c.2525A>T to gene PDGFRA), nucleotides 2445 to 3050 of SEQ ID NO:1 (mutation c.1633G>A to gene PIK3CA), nucleotides 1839 to 2444 of SEQ ID NO:1 (mutation c.3140A>G to gene PIK3CA), nucleotides 1227 to 1838 of SEQ ID NO:1 (mutation c.2753T>C to gene RET), nucleotides 1 to 610 of SEQ ID NO:1 (mutation c.524G>A to gene TP53), nucleotides 611 to 1226 of SEQ ID NO:1 (mutation c.1679T>A to gene KIT), or nucleotides 9154 to 9854 of SEQ ID NO:1 (mutation c.394C>T to gene DH1).

4. The nucleic acid of any one of claims 1-3 part (g), wherein:
(a) the plurality of nucleotide sequences comprises at least 5 nucleotide sequences;
each nucleotide sequence of the plurality of nucleotide sequences is a subsequence of a human gene;
each gene is selected from the group consisting of BRAF, CTNNB1, EGFR, EERBB2, IDH1, KIT, KRAS, NRAS/CSDE1, PDGFRA, PIK3CA, PTEN, RET, and TP53; and
the genotype of each nucleotide sequence of the plurality is selected from the group consisting of mutation c.1799T>A to gene BRAF, mutation c.121A>G to gene CTNNB1, mutation c.2236_2250deI15 to gene EGFR, mutation c.2369C>T to gene EGFR, mutation c.2573T>G to gene EGFR, mutation c.2324_2325ins12 to gene ERBB2, mutation c.394C>T to gene IDH1, mutation c.1679T>A to gene KIT, mutation c.35G>A to gene KRAS, mutation c.182A>G to gene NRAS/CSDE1, mutation c.2525A>T to gene PDGFRA, mutation c.1633G>A to gene PIK3CA, mutation c.3140A>G to gene PIK3CA, mutation c.800delA to gene PTEN, mutation c.2753T>C to gene RET, and mutation c.524G>A to gene TP53; and/or
(b) the nucleic acid is as set out in part (a) and
each nucleotide sequence of the plurality of nucleotide sequences has at least 95% sequence homology with the nucleotide sequence spanning nucleotides 8545 to 9153 of SEQ ID NO:1 (mutation c.800delA to gene PTEN), nucleotides 7937 to 8544 of SEQ ID NO:1 (mutation c.1799T>A to gene BRAF), nucleotides 7327 to 7936 of SEQ ID NO:1 (mutation c.121A>G to gene CTNNB1), nucleotides 6712 to 7326 of SEQ ID NO:1 (mutation c.2573T>G to gene EGFR), nucleotides 6115 to 6711 of SEQ ID NO:1 (mutation c.2236_2250deI15 to gene EGFR), nucleotides 5503 to 6114 of SEQ ID NO:1 (mutation c.2369C>T to gene EGFR), nucleotides 4879 to 5502 of SEQ ID NO:1 (mutation c.2324_2325ins12 to gene ERBB2), nucleotides 4271 to 4878 of SEQ ID NO:1 (mutation c.35G>A to gene KRAS), nucleotides 3661 to 4270 of SEQ ID NO:1 (mutation c.182A>G to gene NRAS), nucleotides 3051 to 3660 of SEQ ID NO:1 (mutation c.2525A>T to gene PDGFRA), nucleotides 2445 to 3050 of SEQ ID NO:1 (mutation c.1633G>A to gene PIK3CA), nucleotides 1839 to 2444 of SEQ ID NO:1 (mutation c.3140A>G to gene PIK3CA), nucleotides 1227 to 1838 of SEQ ID NO:1 (mutation c.2753T>C to gene RET), nucleotides 1 to 610 of SEQ ID NO:1 (mutation c.524G>A to gene TP53), nucleotides 611 to 1226 of SEQ ID NO:1 (mutation c.1679T>A to gene KIT), or nucleotides 9154 to 9854 of SEQ ID NO:1 (mutation c.394C>T to gene IDH1), optionally wherein each nucleotide sequence of the plurality of nucleotide sequences has the nucleotide sequence spanning nucleotides 8545 to 9153 of SEQ ID NO:1 (mutation c.800delA to gene PTEN), nucleotides 7937 to 8544 of SEQ ID NO:1 (mutation c.1799T>A to gene BRAF), nucleotides 7327 to 7936 of SEQ ID NO:1 (mutation c.121A>G to gene CTNNB 1), nucleotides 6712 to 7326 of SEQ ID NO:1 (mutation c.2573T>G to gene EGFR), nucleotides 6115 to 6711 of SEQ ID NO:1 (mutation c.2236_2250deI15 to gene EGFR), nucleotides 5503 to 6114 of SEQ ID NO:1 (mutation c.2369C>T to gene EGFR), nucleotides 4879 to 5502 of SEQ ID NO:1 (mutation c.2324_2325ins12 to gene ERBB2), nucleotides 4271 to 4878 of SEQ ID NO:1 (mutation c.35G>A to gene KRAS), nucleotides 3661 to 4270 of SEQ ID NO:1 (mutation c.182A>G to gene NRAS), nucleotides 3051 to 3660 of SEQ ID NO:1 (mutation c.2525A>T to gene PDGFRA), nucleotides 2445 to 3050 of SEQ ID NO:1 (mutation c.1633G>A to gene PIK3CA), nucleotides 1839 to 2444 of SEQ ID NO:1 (mutation c.3140A>G to gene PIK3CA), nucleotides 1227 to 1838 of SEQ ID NO:1 (mutation c.2753T>C to gene RET), nucleotides 1 to 610 of SEQ ID NO:1 (mutation c.524G>A to gene TP53), nucleotides 611 to 1226 of SEQ ID NO:1 (mutation c.1679T>A to gene KIT), or nucleotides 9154 to 9854 of SEQ ID NO:1 (mutation c.394C>T to gene IDH1); and/or
(c) the nucleic acid has at least 80% sequence homology with the nucleotide sequence set forth in SEQ ID NO:1 or
has the nucleotide sequence set forth in SEQ ID NO:1.

5. The nucleic acid of any one of claims 1, 2 or 3 parts (a)-(b), wherein:
(a) each nucleotide sequence of the plurality of nucleotide sequences is a subsequence of a gene selected from ABI1, ABL1, ABL2, ACSL3, ACUR1, AF15Q14, AF1Q, AF3P21, AF5Q31, AKAP9, AKT1, AKT2, AKT3, AL017, ALDH2, ALK, AMER1, APC, APEX1, AR, AR1D1A, ARAF, ARHGAP5, ARHGEF12, ARHH, ARID1A, ARID2, ARNT, ASPSCR1, ASXL1, ATF1, ATIC, ATM, ATP11B, ATP1A1, ATP2B3, ATR, ATRX, AXIN1, BAP1, BCL10, BCL11A, BCL11B, BCL2, BCL2L1, BCL3, BCL5, BCL6, BCL7A, BCL9, BCOR, BCR, BHD, BIRC2, BIRC3, BLM, BMPR1A, BRAF, BRC42, BRCA1, BRCA2, BRD3, BRD4, BRIP1, BTG1, BUB1B, C12ORF9, C15ORF21, C15ORF55, C16ORF75, C2ORF44, CACNA1D, CALR, CAMTA1, CANT1, CARD11, CARS, CASP8, CBFA2T1, CBFA2T3, CBFB, CBL, CBLB, CBLC, CCDC6, CCNB1IP1, CCND1, CCND2, CCND3, CCNE1, CD273, CD274, CD44, CD74, CD79A, CD79B, CDC73, CDH1, CDH11, CDK12, CDK4, CDK6, CDKN2A, CDKN2A(PL4), CDKN2B, CDKN2C, CDX2, CEBPA, CEP1, CEP89, CHCHD7, CHEK2, CHIC2, CHN1, CIC, CIITA, CLIP1, CLTC, CLTCL1, CMKOR1, CNOT3, COL1A1, COL2A1, COPEB, COX6C, CREB1, CREB3L1, CREB3L2, CREBBP, CRLF2, CRTC3, CSF1R, CSF3R, CSNK2A1, CTNNB1, CUX1, CYLD, D10S170, DAXX, DCTN1, DDB2, DDIT3, DDR2, DDX10, DDX5, DDX6, DEK, DICERL, DNM2, DNMT3A, DUX4, EBFL, ECT2L, EGFR, EIF3E, EIF4A2, ELF4, ELK4, ELKS, ELL, ELN, EML4, EP300, EPS 15, ERBB2, ERBB2 ("HER2"), ERBB3, ERBB4 ("HER4"), ERC1, ERCC1, ERCC2, ERCC3, ERCC4, ERCC5, ERG, ESR1, ETV1, ETV4, ETV5, ETV6, EVI1, EWSR1, EXT1, EXT2, EZH2, EZR, FACL6, FAM46C, FANCA, FANCC, FANCD2, FANCE, FANCF, FANCG, FANCL, FAS, FBXOI1, FBXW7, FCGR2B, FEV, FGFR1, FGFR10P, FGFR2, FGFR3, FGFR4, FH, FHIT, FIP1L1, FLJ27352, FLT3, FLU, FNBP1, FOX03A, FOX04, FOXA1, FOXL2, FOXOIA, FOXP1, FSTL3, FUBP1, FUS, FVT1, GABRA6, GABRG2, GAS6, GAS7, GATA1, GATA2, GATA3, GMPS, GNA11, GNAQ, GNAS, GOLGA5, GOPC, GPC3, GPHN, GRAF, H3F3A, H3F3B, HCMOGT-1, HEAB, HERPUD1, HEY1, HIP1, HIST1H3B, HIST1H4L, HLA-A, HLF, HLXB9, HMGA1, HMGA2, HNF1A, HNRNPA2B1, HOOK3, HOXA11, HOXA13, HOXA9, HOXC11, HOXC13, HOXD11, HOXD13, HPAS, HRAS, HSPCA, HSPCB, IDH1, IDH2, IGF1R, IGH, IGK, IGL, IHD2, IKZF1, IL2, IL21R, IL6, IL6ST, IL7R, IRF4, IRTA1, ITK, JAK1, JAK2, JAK3, JAZF1, JUN, KCNJ5, KDM5A, KDM5C, KDM6A, KDR, KIAA1549, KIAA1598, KIF5B, KIT, KLF4, KLK2, KMT2A, KMT2D, KRAS, KTN1, LAF4, LASP1, LCK, LCP1, LCX, LHFP, LIFR, LM02, LMNA, LMOL, LPP, LRIG3, LSM14A, LYL1, MAF, MAFB, MALAT1, MALT1, MAML2, MAP2K1, MAP2K2, MAP2K4, MAX, MCL1, MDM2, MDM4, MDS1, MDS2, MECOM, MECT1, MED 12, MEN1, MET, MITF, MKL1, MLF1, MLH1, MLL, MLL3, MLLT1, MLLT10, MLLT2, MLLT3, MLLT4, MLLT6, MLLT7, MN1, MPL, MSF, MSH2, MSH6, MSI2, MSN, MTCP1, MTOR, MUC1, MUTYH, MY018A, MY05A, MYB, MYC, MYC1, MYCL1, MYCN, MYD88, MYH11, MYH9, MYST4, NAB2, NACA, NBSL, NCOA1, NCOA2, NCOA4, NDRG1, NF1, NF2, NFATC2, NFE2L2, NFIB, NFKB2, NIN, NKX2- 1, NKX2-1, NKX2-8, NONO, NOTCH1, NOTCH2, NPM1, NR4A3, NRAS, NRAS/CSDE1, NRG1, NSD1, NT5C2, NTRK1, NTRK3, NUMA1, NUP214, NUP98, NUTM2A, NUTM2B, OLIG2, OMD, P2RY8, PAFAH1B2, PALB2, PARP1, PARP2, PAX3, PAX5, PAX7, PAX8, PBRM1, PBX1, PCM1, PCSK7, PDCD1LG2, PDE4DIP, PDGFB, PDGFRA, PDGFRB, PERI, PHF6, PHOX2B, PICALM, PIK3CA, PIK3R1, PIM1, PLAG1, PLCG1, PML, PMS1, PMS2, PMX1, PNP, PNUTL1, POT1, POU2AF1, POU5F1, PPARG, PPFIBP1, PPP2R1A, PRCC, PRDM1, PRDM16, PRF1, PRKAR1A, PSIP1, PTCH1, PTEN, PTPN11, PTPRB, PTPRC, PTPRK, PWWP2A, RAB5EP, RAC1, RAD21, RAD51, RAD51L1, RAF1, RAFT, RALGDS, RANBP17, RAP1GDS1, RARA, RB1, RBI, RBM15, RECQL4, REL, RET, RHEB, RHOA, RIT1, RNF43, ROS1, RPL10, RPL22, RPL5, RPN1, RPS6KB1, RSP02, RSP03, RUNDC2A, RUNX1, RUNX1T1, RUNXBP2, SBDS, SDC4, SDH5, SDHB, SDHC, SDHD, SET, SETBP1, SETD2, SF3B1, SFPQ, SFRS3, SH2B3, SH3GL1, SIL, SLC34A2, SLC45A3, SMAD4, SMARCA4, SMARCB1, SMARCE1, SMO, SOCS1, SOX2, SRC, SRGAP3, SRSF2, SS18, SS18L1, SSX1, SSX2, SSX4, STAG2, STAT3, STAT5B, STAT6, STK11, STL, SUFU, SUZ12, SYK, TAF15, TAL1, TAL2, TALI, TBL1XR1, TCEA1, TCF1, TCF12, TCF3, TCF7L2, TCL1A, TCL6, TERT, TET2, TFE3, TFEB, TFG, TFPT, TFRC, THRAP3, TIAF1, TIF1, TLX1, TLX3, TMPRSS2, TNFAIP3, TNFRSF14, TNFRSF17, TOPI, TP53, TPM3, TPM4, TPR, TRA, TRAF7, TRB, TRD, TRIM27, TRIM33, TRIP11, TRRAP, TSC1, TSC2, TSHR, TTL, U2AF1, UBR5, USP6, VHL, VT11A, WAS, WHSC1, WHSC1L1, WIF1, WRN, WT1, WWTR1, XPA, XPC, XPO1, YWHAE, ZCCHC8, ZNF145, ZNF198, ZNF217, ZNF278, ZNF331, ZNF384, ZNF521, ZNF9, ZRSR2, and ZRSR2;
(b) each nucleotide sequence of the plurality of nucleotide sequences is a subsequence of a gene selected from BMPR1A (bone morphogenetic protein receptor, type IA), BRCA1 (breast cancer 1), BRCA2 (breast cancer 2), CDH1 (cadherin-1), CDKN2A (cyclin dependent kinase inhibitor 2A), EPCAM (epithelial cell adhesion molecule), MI,H1 (mutL homolog 1), MSH2 (mutS homolog 2), MSH6 (mutS homolog 6), MUTYH (mutY DNA glycosylase), PALB2 (partner and localizer of BRCA2), PMS2 (PMS1 homolog 2, mismatch repair system component), PTEN (phosphatase and tensin homolog), SMAD4 (SMAD family member 4; mothers against decapentaplegic homolog 4), STK11 (serine/threonine kinase 11), and TP53 (tumor protein p53);
(c) the nucleic acid is as set out in part (b) and
each genotype of a nucleotide sequence of the plurality of nucleotide sequences is selected from the group consisting of mutation c.942+3A>T to gene MSH2, mutation c.1662-12 1677del to gene MSH2, mutation c.2056_2060delinsCTTCTACCTCAAAAA (SEQ ID NO: 13) to gene MSH6, mutation c.2308_2312delGGTAAinsT to gene MSH6, mutation c.2641delGinsAAAA to gene MSH6, mutation c.3163_3164insG to gene MSH6, mutation c.232_243delinsATGTAAGGto gene MI,H1, mutation c.1852_1854delAAG to gene MLH1, mutation c.2445+1G>C to gene PMS2, mutation c.2243_2246delAGAA to gene PMS2, mutation c.2253T>C to gene PMS2, mutation c.861_864delACAGto gene PMS2, mutation c.9_32dup24 to gene CDKN2A, mutation c.8975_9100del126 to gene BRCA2, mutation c.9203del126 to gene BRCA2, mutation c.1310_1313delAAGA to gene BRCA2, mutation c.1813dupA to gene BRCA2, mutation c.9342_9343insAluY to gene BRCA2, mutation c.5266_5267insC to gene BRCA1, mutation c.5177_5180delGAAA to gene BRCA1, mutation c.3756_3759delGTCT to gene BRCA1, mutation c.3481_3491delGAAGATACTAG (SEQ ID NO: 14) to gene BRCA1, mutation c.3113A>G to gene BRCA1, mutation c.3084_3094delTAATAACATTA (SEQ ID NO: 15) to gene BRCA1, mutation c.2834_2836delinsC to gene BRCA1, and mutation c.68_69delAG to gene BRCA1;
(d) the nucleic acid is as set out in part (b) or (c) and
the nucleic acid has at least 80% sequence homology with the nucleotide sequence set forth in SEQ ID NO:2 or SEQ ID NO:3, or
has the nucleotide sequence set forth in SEQ ID NO:2 or SEQ ID NO:3;
(e) the nucleic acid is as set out in part (b) and
each genotype of a nucleotide sequence of the plurality of nucleotide sequences is selected from the group consisting of mutation c.675+1G>C to gene BMPR1A, mutation c.817C>T to gene BMPR1A, mutation c.1016dupA to gene BRCA1, mutation c.1175_1214delTGTTAGGTTCTGATGACTCACATGATGGGGAGTCTGAATC (SEQ ID NO: 23) to gene BRCA1, mutation c.1387_1390delinsGAAAG to gene BRCA1, mutation c.181T>G to gene BRCA1, mutation c.1953_1956delGAAA to gene BRCA1, mutation c.2834_2836delinsC to gene BRCA1, mutation c.3084_3094delTAATAACATTA (SEQ ID NO: 15) to gene BRCA1, mutation c.3113A>G to gene BRCA1, mutation c.3481_3491delGAAGATACTAG (SEQ ID NO: 14) to gene BRCA1, mutation c.3756_3759delGTCT to gene BRCA1, mutation c.406_407insA to gene BRCA1, mutation c.4964_4982delCTGGCCTGACCCCAGAAGA (SEQ ID NO: 19) to gene BRCA1, mutation c.5096G>A to gene BRCA1, mutation c.5177_5180delGAAA to gene BRCA1, mutation c.5177_5180delGAAA to gene BRCA1, mutation c.5266_5267insC to gene BRCA1, mutation c.68_69delAG to gene BRCA1, mutation c.815_824dupAGCCATGTGG (SEQ ID NO: 25) to gene BRCA1, mutation c.10095delCinsGAATTATATCT (SEQ ID NO: 29) to gene BRCA2, mutation c.1310_1313delAAGA to gene BRCA2, mutation c.1310_1313delAAGA to gene BRCA2, mutation c.156_157insAluYa5 to gene BRCA2, mutation c.1813dupA to gene BRCA2, mutation c.2588dupA to gene BRCA2, mutation c.2808_2811delACAA to gene BRCA2, mutation c.4037_4043delinsT to gene BRCA2, mutation c.5073dupA to gene BRCA2, mutation c.5350_5351delAA to gene BRCA2, mutation c.5946delT to gene BRCA2, mutation c.6275_6276delTT to gene BRCA2, mutation c.7762_7764delinsTT to gene BRCA2, mutation c.8537_8538delAG to gene BRCA2, mutation c.8902_8913delinsTCCC to gene BRCA2, mutation c.9203del126 to gene BRCA2, mutation c.9253dupA to gene BRCA2, mutation c.9342_9343insAluY to gene BRCA2, mutation c.1792C>T to gene CDH1, mutation c.9_32dup24 to gene CDKN2A, mutation c.429G>A to gene EPCAM, mutation c.523C>T to gene EPCAM, mutation c.556-14A>G to gene EPCAM, mutation c.1852_1854delAAG to gene MLH1, mutation c.1852_1854delAAG to gene MLH1, mutation c.232_243delinsATGTAAGG to gene MLH1, mutation c.382delG to gene MLH1, mutation c.704_723delATAAAACCCTAGCCTTCAAA (SEQ ID NO: 33) to gene MLH1, mutation c.1076G>C to gene MSH2, mutation c.1662-12_1677del to gene MSH2, mutation c.942+3A>T to gene MSH2, mutation c.942+3A>T to gene MSH2, mutation c.2056_2060delinsCTTCTACCTCAAAAA (SEQ ID NO: 13) to gene MSH6, mutation c.2308_2312delGGTAAinsT to gene MSH6, mutation c.2641delGinsAAAA to gene MSH6, mutation c.3163_3164insG to gene MSH6, mutation c.3261delC to gene MSH6, mutation c.3939_3957dupTCAAAAGGGACATAGAAAA (SEQ ID NO: 36) to gene MSH6, mutation c.741delA to gene MSH6, mutation c.1147delC to gene MUTYH, mutation c.1187G>A to gene MUTYH, mutation c.1435G>T to gene MUTYH, mutation c.536A>G to gene MUTYH, mutation c.933+3A>C to gene MUTYH, mutation c.2386G>T to gene PALB2, mutation c.3113G>A to gene PALB2, mutation c.1261C>T to gene PMS2, mutation c.137G>T to gene PMS2, mutation c.2117delA to gene PMS2, mutation c.2243_2246delAGAA, c.2253T>C to gene PMS2, mutation c.2445+1G>C to gene PMS2, mutation c.861_864delACAG to gene PMS2, mutation c.861_864delACAG to gene PMS2, mutation c.511C>T to gene PTEN, mutation c.758_759insAT to gene PTEN, mutation c.987_996dupTAAAGACAAA (SEQ ID NO: 38) to gene PTEN, mutation c.1206_1207insT to gene SMAD4, mutation c.1353_1354insGCTACTGCACAAGCTGCAGCAGCTGCCC (SEQ ID NO: 40) to gene SMAD4, mutation c.1529delG to gene SMAD4, mutation c.842_843insC to gene STK11, mutation c.637C>T to gene TP53, and mutation c.916C>T to gene TP53.

6. The nucleic acid of claim 1 or 2, wherein:
(a) the genotype of each nucleotide sequence of the plurality of nucleotide sequences is associated with a heart condition, optionally
Cardiomyopathy;
(b) the genotype of each nucleotide sequence of the plurality of nucleotide sequences is
associated with a heart condition, optionally cardiomyopathy, and
each nucleotide sequence of the plurality of nucleotide sequences comprises a subsequence of a gene selected from the group consisting of cardiac myosin-binding protein C (MYBPC3); myosin, heavy chain 7, cardiac muscle, beta (MYH7); troponin I type 3 (TNNI3); cardiac troponin T (TNNT2); and tropomyosin alpha-1 chain (TPM1); and
each subsequence comprises a mutation that is associated with cardiomyopathy;
optionally
wherein each mutation is selected from the group consisting of:
a mutation to the gene for cardiac myosin-binding protein C (MYBPC3) selected from the group consisting of 1504C>T, 2373_2374insG, and 3628-41_3628-17del;
a mutation to the gene for myosin, heavy chain 7, cardiac muscle, beta (MYH7) selected from the group consisting of 1988G>A, 1357C>T, and 1750G>C;
a mutation to the gene for troponin I type 3 (TNNI3) selected from the group consisting of 532_534delAAG and 575G>A;
a mutation to the gene for cardiac troponin T (TNNT2), wherein the mutation is 487_489delGAG; and
a mutation to the gene for tropomyosin alpha-1 chain (TPM1), wherein the mutation is 574G>A;
(c) the genotype of each nucleotide sequence of the plurality of nucleotide sequences is associated with a heart condition, optionally cardiomyopathy, and
each nucleotide sequence of the plurality of nucleotide sequences is a subsequence of a gene selected from MYBPC3, MYH7, TNNI3, TNNT2, and TPM1, optionally wherein each genotype of a nucleotide sequence of the plurality of nucleotide sequences is selected from the group consisting of mutation 1504C>T to gene MYBPC3, mutation 2373_2374insG to gene MYBPC3, mutation 3628-41_3628-17del to gene MYBPC3, mutation 1988G>A to gene MYH7, mutation 1357C>T to gene MYH7, mutation 1750G>C to gene MYH7, mutation 532_534delAAG to gene TNNI3, mutation 575G>A to gene TNNI3, mutation 487_489delGAG to gene TNNT2, and mutation 574G>A to gene TPM1;
(d) the genotype of each nucleotide sequence of the plurality of nucleotide sequences is associated with a heart condition, optionally cardiomyopathy, and
each nucleotide sequence of the plurality of nucleotide sequences is a subsequence of a gene selected from ABCC9, ACTC1, ACTN2, AKAP9, ANK2, ANKRD1, BAG3, BRAF, CACNA1C, CACNB2, CASQ2, CAV3, CRYAB, CSRP3, DES, DMD, DSC2, DSG2, DSP, DTNA, EMD, FKTN, GATAD1, GLA, GPD1L, HCN4, HRAS, ILK, JPH2, JUP, KCNE1, KCNE2, KCNE3, KCNH2, KCNJ2, KCNJ5, KCNJ8, KCNQ1, KRAS, LAMA4, LAMP2, LDB3, LMNA, MAP2K1, MAP2K2, MTND1, MTND5, MTND6, MTTD, MTTG, MTTH, MTTI, MTTK, MTTL1, MTTL2, MTTM, MTTQ, MTTS1, MTTS2, MYBPC3, MYH7, MYL2, MYL3, MYLK2, MYOZ2, MYPN, NEBL, NEXN, NKX2.5, NRAS, PDLIM3, PKP2, PLN, PRKAG2, PTPN11, RAF1, RANGRF, RBM20, RYR2, SCN1B, SCN3B, SCN4B, SCN5A, SGCD, SNTA1, SOS1, TAZ, TCAP, TMEM43, TMPO, TNNC1, TNNI3, TNNT2, TPM1, TTN, TTR, and VCL; and/or
(e) the nucleic acid has at least 80% sequence homology with the nucleotide sequence set forth in SEQ ID NO:4 or
has the nucleotide sequence set forth in SEQ ID NO:4.

7. The nucleic acid of any one of claims 1-6, wherein:
(a) each nucleotide sequence of the plurality is at least 600 nucleotides long,
about 600 nucleotides to about 2000 nucleotides long,
about 600 nucleotides to about 1200 nucleotides long,
about 1000 nucleotides to about 10,000 nucleotides long; and/or
(b) The nucleic acid further comprises an origin of replication, optionally
wherein the origin of replication is an origin of replication from *Escherichia coli* or *Saccharomyces cerevisiae* and/or
(c) the nucleic acid further comprises at least one methylated nucleoside or nucleotide; and/or
(d) the nucleic acid further comprises a promoter,
optionally wherein the promoter is an SP6 promoter; and/or
(e) the nucleic acid further comprises a promoter,
wherein the promoter is from a different species than the nucleotide sequences of the plurality, optionally
wherein the promoter is an SP6 promoter; and/or
the nucleic acid is a plasmid.

8. A cell comprising the nucleic acid of any one of claims 1-7.

9. The cell of claim 8, wherein:
(a) the cell is a human cell; and/or
(b) wherein the cell is a fibroblast or a lymphocyte, optionally an immortalized B lymphocyte; and/or
(c) the cell is GM12878, GM24149, GM24143, GM24385, GM24631, GM24694, or GM24695, optionally GM24385; and/or
(d) the cell comprises 1 to 1000 copies or 5 to 500 copies of the nucleic acid; or
(e) the cell is *E. coli.*

10. A composition, comprising a first plurality of cells and a second plurality of cells, wherein:
the first plurality of cells consists of cells according to claim 9 part (d) which cells comprise 5 to 500 copies of the nucleic acid;
the second plurality of cells consists of cells that do not comprise the nucleic acid;
the first plurality of cells and the second plurality of cells are human cells;
the first plurality of cells and the second plurality of cells are admixed in the composition; and
the ratio of the number of cells of the first plurality to the number of cells of the second plurality is about 1:1 to about 1:10,000 in the composition.

11. A method for making a biological reference material, comprising transfecting a plurality of cells with the nucleic acid of any one of claims 1-7; optionally further comprising:
(a) fixing the cells of the plurality, optionally
wherein fixing the cells comprises fixing the cells with formalin; and/or
(b) embedding the cells in paraffin; and/or
(c) diluting the plurality of cells with untransfected cells, optionally
wherein diluting the plurality of cells with untransfected cells comprises diluting at a ratio of transfected cells to untransfected cells of about 1:1 to about 1:10,000.

12. A biological reference material, comprising
(a) a plurality of cells of any one of claims 8 or 9 parts (a)-(d) and paraffin, wherein the plurality of cells are fixed and embedded in the paraffin,
optionally further comprising untransfected cells, wherein the untransfected cells do not comprise the nucleic acid, optionally wherein the ratio of cells comprising the nucleic acid to untransfected cells is about 1:1 to about 1:10,000, about 1 :5 to about 1:5,000 or about 1:10 to about 1:1,000; or
(b) a plurality of cells of any one of claims 8 or 9 parts (a)-(d); and a liquid, optionally wherein the liquid is plasma.

13. A composition comprising a nucleic acid and an aqueous buffer, wherein the nucleic acid is a nucleic acid of any one of claims 1-7 that has been extracted from the reference material of claim 12, optionally
wherein the buffer comprises tris(hydroxymethyl)aminomethane and ethylenediaminetetraacetic acid, or a salt of any one of the foregoing.

## Patentansprüche

1. Nukleinsäure, die eine Vielzahl von Nukleotidsequenzen umfasst, wobei jede Nukleotidsequenz der Vielzahl einen Genotyp umfasst, der mit einer Krankheit oder einem Zustand verbunden ist; jede Nukleotidsequenz 600-10.000 Basenpaare lang ist; und die Vielzahl von Nukleotidsequenzen Nukleotidsequenzen von mindestens zwei verschiedenen Chromosomen umfasst.

2. Nukleinsäure nach Anspruch 1, wobei:
(a) der Genotyp jeder Nukleotidsequenz der Vielzahl eine somatische Mutation oder eine vererbbare Mutation ist; und/oder
(b) der Genotyp jeder Nukleotidsequenz der Vielzahl ein einzelner Nukleotidpolymorphismus, eine PunktMutation, ein vorzeitiges Stoppcodon, eine Trinukleotidwiederholung, eine Translokation, eine somatische Umlagerung, ein Allelomorph, eine einzelne Nukleotidvariante, eine Insertion oder Deletion ("Indel"), regulatorisch Variante oder Genfusion ist; und/oder
(c) die Vielzahl von Nukleotidsequenzen Nukleotidsequenzen von mindestens drei verschiedenen Chromosomen umfasst; und/oder
(d) die Nukleinsäure DNA ist; und/oder
(e) die Vielzahl von Nukleotidsequenzen Nukleotidsequenzen von mindestens 3 oder mindestens 5 verschiedenen Chromosomen umfasst; und/oder
(f) jede Nukleotidsequenz der Vielzahl eine Teilsequenz eines Gens oder einer regulatorischen Region davon ist, optional wobei jede Nukleotidsequenz der Vielzahl eine Teilsequenz eines Gens ist,
optional wobei jede Nukleotidsequenz der Vielzahl eine Teilsequenz eines Exons ist,
optional wobei der Genotyp jeder Nukleotidsequenz der Vielzahl eine Mutation, Deletion oder Insertion von mindestens einer Aminosäure in die vom Exon kodierte Aminosäuresequenz verursacht; und/oder
(g) die Nukleinsäure wie in (e) oder (f) dargelegt ist und jedes Gen ein menschliches Gen ist; und/oder
(h) jede Nukleotidsequenz der Vielzahl eine menschliche Nukleotidsequenz ist.

3. Nukleinsäure nach Anspruch 1 oder 2, wobei:
(a) der Genotyp jeder Nukleotidsequenz der Vielzahl von Nukleotidsequenzen mit einem Neoplasma, gegebenenfalls einem soliden Tumor, verbunden ist; und/oder
(b) der Genotyp jeder Nukleotidsequenz der Vielzahl von Nukleotidsequenzen mit einem Neoplasma assoziiert ist, das Lungenkrebs, Lymphkrebs, akute lymphatische Leukämie, akute myeloische Leukämie, chronische myeloische Leukämie, Schilddrüsenkrebs, Magenkrebs, Dickdarmkrebs, Harnwegskrebs, Krebs des Zentralnervensystems, Nierenkrebs, Brustkrebs und/oder Weichteilkrebs, optional ein solider Tumor ist; und/oder
(c) der Genotyp jeder Nukleotidsequenz der Vielzahl von Nukleotidsequenzen mit einem Neoplasma in (a) oder (b) assoziiert ist und jede Nukleotidsequenz der Vielzahl von Nukleotidsequenzen eine Teilsequenz eines Gens ist, ausgewählt aus der Gruppe bestehend aus ABL1, AKT1, AKT2, AKT3, ALK, APC, AR, AR1D1A, ARAF, ARHGAP5, ATM, ATR, BCL2, BCR, BRAF, BRC42, BRCA1, BRCA2, BRIP1, CBFB, CCND1, CCND2, CCNE1, CDH1, CDK4, CDK6, CDKN2A, CDKN2B, CSF1R, CTNNB1, DDR2, DNMT3A, EGFR, ERBB2 ("HER2"), ERBB3, ERBB4 ("HER4"), ERCC1, ESR1, ETV1, ETV4, ETV6, EWSR1, EZH2, FANCA, FANCC, FANCD2, FANCE, FANCF, FANCG, FANCL, FBXW7, FGFR1, FGFR2, FGFR3, FLT3, FOXL2, GABRA6, GABRG2, GATA3, GNA11, GNAQ, GNAS, HNF1A, HPAS, HRAS, IDH1, IDH2, IHD2, JAK2, JAK3, KDR, KIT, KMT2A, KRAS, MAP2K1, MAP2K2, MECOM, MET, MKL1, MLH1, MLL, MPL, MSH2, MSH6, MTOR, MYC, MYCN, MYD88, NF1, NF2, NFE2L2, NOTCH1, NPM1, NRAS/CSDE1, NTRK1, NUP214, PALB2, PARP1, PARP2, PDGFRA, PDGFRB, PICALM, PIK3CA, PMS2, PTCH1, PTEN, PTPN11, RAD51, RAFT, RARA, RB1, RET, RHEB, RHOA, RIT1, ROS1, RUNX1, RUNX1T1, SMAD4, SMARCB1, SMO, SRC, STK11, TAL1, TCF3, TERT, TMPRSS2, TP53, TSC1, TSC2 und VHL; und/oder
(d) der Genotyp jeder Nukleotidsequenz der Vielzahl von Nukleotidsequenzen mit einem Neoplasma wie in (a) oder (b) verbunden ist,
und jede Nukleotidsequenz der Vielzahl von Nukleotidsequenzen eine Teilsequenz eines Gens ist, ausgewählt aus der Gruppe bestehend aus AKT1, AKT2, AKT3, APC, ARHGAP5, ATM, ATR, BRAF, CTNNB1, DNMT3A, EGFR, ERBB2, ERCC1, EZH2, FBXW7, FGFR3, FLT3, GABRA6, GABRG2, GNAQ, GNAS, IDH1, IDH2, JAK2, KIT, KRAS, MET, MLH1, MPL, MSH2, MTOR, MYD88, NF1, NPM1, NRAS/CSDE1, PARP1, PARP2, PDGFRA, PIK3CA, PTEN, PTPN11, RAD51, RB1, RET, SMAD4, TP53, und VHL, wobei optional der Genotyp jeder Nukleotidsequenz der Vielzahl von Nukleotidsequenzen aus der Gruppe bestehend aus Mutation 145G>A zum Gen AKT1, Mutation c.49G>A zum Gen AKT1, Mutation c.268G>T zum Gen AKT2, Mutation c.371A>T zum Gen AKT3, Mutation c.4248delC zum Gen APC, Mutation c.4348C>T zum Gen APC, Mutation c.4666_4667insA zum Gen APC, Mutation c.1864G>A zum Gen ARHGAP5, Mutation c.1058_1059delGT zum Gen ATM, Mutation c.5557G>A zum Gen ATM, Mutation c.3790_3796delATAAAAG zum Gen ATR, Mutation c.1799T>A zum Gen BRAF, Mutation c.121A>G zum Gen CTNNB1, Mutation c.2644C>T zum Gen DNMT3A, Mutation c.2236_2250del15 zum Gen EGFR, Mutation c.2310_2311insGGT zum Gen EGFR, Mutation c.2369C>T zum Gen EGFR, Mutation c.2573T>G zum Gen EGFR, Mutation c.2324_2325ins12 zum Gen ERBB2, Mutation c.287C>A zum Gen ERCC1, Mutation c.1937A>T zum Gen EZH2, Mutation c.1394G>A zum Gen FBXW7, Mutation c.746C>G zum Gen FGFR3, Mutation c.2503G>T zum Gen FLT3, Mutation c.763G>C zum Gen GABRA6, Mutation c.1355A>G zum Gen GABRG2, Mutation c.626A>C zum Gen GNAQ, Mutation c.601C>T zum Gen GNAS, Mutation c.394C>T zum Gen IDH1, Mutation c.515G>A zum Gen IDH2, Mutation c.419G>A zum Gen IDH2, Mutation c.1849G>T zum Gen JAK2, Mutation c.2447A>T zum Gen KIT, Mutation c.1679T>A zum Gen KIT, Mutation c.35G>A zum Gen KRAS, Mutation c.3757T>G zum Gen MET, Mutation c.1151T>A zum Gen MLH1, Mutation c.1544G>T zum Gen MPL, Mutation c.2250delG zum Gen MSH2, Mutation c.2359_2360delCT zum Gen MSH2, Mutation c.2664A>T zum Gen MTOR, Mutation c.794T>C zum Gen MYD88, Mutation c.2987_2988insAC zum Gen NF1, Mutation c.4084C>T zum Gen NF1, Mutation c.7501delG zum Gen NF1, Mutation c.863_864insTCTG zum Gen NPM1, Mutation c.182A>G zum Gen NRAS, Mutation c.2738delG zum Gen PARP1, Mutation c.398A>C zum Gen PARP2, Mutation c.1694 1695insA zum Gen PDGFRA, Mutation c.2525A>T zum Gen PDGFRA, Mutation c.1633G>A zum Gen PIK3CA, Mutation c.3140A>G zum Gen PIK3CA, Mutation c.3204_3205insA zum Gen PIK3CA, Mutation c.388C>T zum Gen PTEN, Mutation c.741_742insA zum Gen PTEN, Mutation c.800delA zum Gen PTEN, Mutation c.226G>A zum Gen PTPN11, Mutation c.433C>T zum Gen RAD51, Mutation c.958C>T zum Gen RB1, Mutation c.2753T>C zum Gen RET, Mutation c.1394_1395insT zum Gen SMAD4, Mutation c.818G>A zum Gen TP53, Mutation c.743G>A zum Gen TP53, Mutation c.723delC zum Gen TP53, Mutation c.524G>A zum Gen TP53, Mutation c.263delC zum Gen TP53, and Mutation c.426_429delTGAC zum Gen VHL ausgewählt ist; und/oder
(e) die Nukleinsäure wie in Teil (d) dargelegt ist und jede Nukleotidsequenz der Vielzahl von Nukleotidsequenzen eine Teilsequenz eines Gens ist, ausgewählt aus der Gruppe bestehend aus BRAF, CTNNB1, EGFR, ERBB2, IDH1, KIT, KRAS, NRAS/CSDE1, PDGFRA, PIK3CA, PTEN, RET und TP53; und/oder
(f) die Nukleinsäure wie in Teil (d) oder (e) dargelegt ist und
die Vielzahl von Nukleotidsequenzen mindestens 5 Nukleotidsequenzen umfasst;
jede Nukleotidsequenz der Vielzahl von Nukleotidsequenzen eine Teilsequenz eines menschlichen Gens ist; und
jedes Gen aus der Gruppe bestehend aus BRAF, CTNNB1, EGFR, ERBB2, IDH1, KIT, KRAS, NRAS/CSDE1, PDGFRA, PIK3CA, PTEN, RET und TP53 ausgewählt ist; und/oder
(g) die Nukleinsäure wie in Teil (e) oder (f) dargelegt ist und
der Genotyp jeder Nukleotidsequenz der Vielzahl von Nukleotidsequenzen ausgewählt ist aus der Gruppe bestehend aus Mutation c.1799T>A zum Gen BRAF, Mutation c.121A>G zum Gen CTNNB1, Mutation c.2236_2250del15 zum Gen EGFR, Mutation c.2369C>T zum Gen EGFR, Mutation c.2573T>G zum Gen EGFR, Mutation c.2324_2325ins12 zum Gen ERBB2, Mutation c.394C>T zum Gen IDH1, Mutation c.1679T>A zum Gen KIT, Mutation c.35G>A zum Gen KRAS, Mutation c.182A>G zum Gen NRAS/CSDE1, Mutation c.2525A>T zum Gen PDGFRA, Mutation c.1633G>A zum Gen PIK3CA, Mutation c.3140A>G zum Gen PIK3CA, Mutation c.800delA zum Gen PTEN, Mutation c.2753T>C zum Gen RET, and Mutation c.524G>A zum Gen TP53; und/oder
(h) die Nukleinsäure wie in Teil (g) dargelegt ist und jede Nukleotidsequenz der Vielzahl von Nukleotidsequenzen mindestens 95 % Sequenzhomologie mit der Nukleotidsequenz aufweist, die die Nukleotide 8545 bis 9153 von SEQ ID Nr.1 (Mutation c.800delA zum Gen PTEN), Nukleotide 7937 bis 8544 von SEQ ID Nr.1 (Mutation c.1799T>A zum Gen BRAF), Nukleotide 7327 bis 7936 von SEQ ID Nr.1 (Mutation c.121A>G zum Gen CTNNB1), Nukleotide 6712 bis 7326 von SEQ ID Nr.1 (Mutation c.2573T>G zum Gen EGFR), Nukleotide 6115 bis 6711 von SEQ ID Nr.1 (Mutation c.2236_2250del15 zum Gen EGFR), Nukleotide 5503 bis 6114 von SEQ ID Nr.1 (Mutation c.2369C>T zum Gen EGFR), Nukleotide 4879 bis 5502 von SEQ ID Nr.1 (Mutation c.2324_2325ins12 zum Gen ERBB2), Nukleotide 4271 bis 4878 von SEQ ID Nr.1 (Mutation c.35G>A zum Gen KRAS), Nukleotide 3661 bis 4270 von SEQ ID Nr.1 (Mutation c.182A>G zum Gen NRAS), Nukleotide 3051 bis 3660 von SEQ ID Nr.1 (Mutation c.2525A>T zum Gen PDGFRA), Nukleotide 2445 bis 3050 von SEQ ID Nr.1 (Mutation c.1633G>A zum Gen PIK3CA), Nukleotide 1839 bis 2444 von SEQ ID Nr.1 (Mutation c.3140A>G zum Gen PIK3CA), Nukleotide 1227 bis 1838 von SEQ ID Nr.1 (Mutation c.2753T>C zum Gen RET), Nukleotide 1 bis 610 von SEQ ID Nr.1 (Mutation c.524G>A zum Gen TP53), Nukleotide 611 bis 1226 von SEQ ID Nr.1 (Mutation c.1679T> A zum Gen KIT) oder Nukleotide 9154 bis 9854 von SEQ ID Nr.1 (Mutation 394C>T zum Gen IDH1) umfasst, optional wobei jede Nukleotidsequenz der Vielzahl von Nukleotidsequenzen die Nukleotidsequenz aufweist, die die Nukleotide 8545 bis 9153 von SEQ ID Nr.1 (Mutation c.800delA zum Gen PTEN), Nukleotide 7937 bis 8544 von SEQ ID Nr.1 (Mutation c.1799T>A zum Gen BRAF), Nukleotide 7327 bis 7936 von SEQ ID Nr.1 (Mutation c.121A>G zum Gen CTNNB1), Nukleotide 6712 bis 7326 von SEQ ID Nr.1 (Mutation c.2573T>G zum Gen EGFR), Nukleotide 6115 bis 6711 von SEQ ID Nr.1 (Mutation c.2236_2250del15 zum Gen EGFR), Nukleotide 5503 bis 6114 von SEQ ID Nr.1 (Mutation c.2369C>T zum Gen EGFR), Nukleotide 4879 bis 5502 von SEQ ID Nr.1 (Mutation c.2324 2325ins12 zum Gen ERBB2), Nukleotide 4271 bis 4878 von SEQ ID Nr.1 (Mutation c.35G>A zum Gen KRAS), Nukleotide 3661 bis 4270 von SEQ ID Nr.1 (Mutation c.182A>G zum Gen NRAS), Nukleotide 3051 bis 3660 von SEO ID Nr.1 (Mutation c.2525A>T zum Gen PDGFRA), Nukleotide 2445 bis 3050 von SEQ ID Nr.1 (Mutation c.1633G>A zum Gen PIK3CA), Nukleotide 1839 bis 2444 von SEQ ID Nr.1 (Mutation c.3140A>G zum Gen PIK3CA), Nukleotide 1227 bis 1838 von SEQ ID Nr.1 (Mutation c.2753T>C zum Gen RET), Nukleotide 1 bis 610 von SEQ ID Nr.1 (Mutation c.524G>A zum Gen TP53), Nukleotide 611 bis 1226 von SEQ ID Nr.1 (Mutation c.1679T> A zum Gen KIT) oder Nukleotide 9154 bis 9854 von SEQ ID Nr.1 (Mutation c.394C>T zum Gen DH1) umfasst.

4. Nukleinsäure nach einem der Ansprüche 1-3 Teil (g), wobei:
(a) die Vielzahl von Nukleotidsequenzen mindestens 5 Nukleotidsequenzen umfasst;
jede Nukleotidsequenz der Vielzahl von Nukleotidsequenzen eine Teilsequenz eines menschlichen Gens ist;
jedes Gen aus der Gruppe bestehend aus BRAF, CTNNB1, EGFR, EERBB2, IDH1, KIT, KRAS, NRAS/CSDE1, PDGFRA, PIK3CA, PTEN, RET und TP53 ausgewählt ist; und
der Genotyp jeder Nukleotidsequenz der Vielzahl aus der Gruppe bestehend aus Mutation c.1799T>A zum Gen BRAF, Mutation c.121A>G zum Gen CTNNBI1, Mutation c.2236 2250del15 zum Gen EGFR, Mutation c.2369C>T zum Gen EGFR, Mutation c.2573T>G zum Gen EGFR, Mutation c.2324 2325ins12 zum Gen ERBB2, Mutation c.394C>T zum Gen IDH1, Mutation c.1679T>A zum Gen KIT, Mutation c.35G>A zum Gen KRAS, Mutation c.182A>G zum Gen NRAS/CSDE1, Mutation c.2525A>T zum Gen PDGFRA, Mutation c.1633G>A zum Gen PIK3CA, Mutation c.3140A>G zum Gen PIK3CA, Mutation c.800delA zum Gen PTEN, Mutation c.2753T>C zum Gen RET und Mutation c.524G>A zum Gen TP53 ausgewählt ist; und/oder
(b) die Nukleinsäure ist wie in Teil (a) dargelegt ist und
jede Nukleotidsequenz der Vielzahl von Nukleotidsequenzen mindestens 95 % Sequenzhomologie mit der Nukleotidsequenz aufweist, die die Nukleotide 8545 bis 9153 von SEQ ID Nr.1 (Mutation c.800delA zum Gen PTEN), Nukleotide 7937 bis 8544 von SEQ ID Nr.1 (Mutation c.1799T>A zum Gen BRAF), Nukleotide 7327 bis 7936 von SEQ ID Nr.1 (Mutation c.121A>G zum Gen CTNNB1), Nukleotide 6712 bis 7326 von SEQ ID Nr.1 (Mutation c.2573T>G zum Gen EGFR), Nukleotide 6115 bis 6711 von SEQ ID Nr.1 (Mutation c.2236 2250del15 zum Gen EGFR), Nukleotide 5503 bis 6114 von SEQ ID Nr.1 (Mutation c.2369C>T zum Gen EGFR), Nukleotide 4879 bis 5502 von SEQ ID Nr.1 (Mutation cc.2324 2325ins12 zum Gen ERBB2), Nukleotide 4271 bis 4878 von SEQ ID Nr.1 (Mutation c.35G>A zum Gen KRAS), Nukleotide 3661 bis 4270 von SEQ ID Nr.1 (Mutation c.182A>G zum Gen NRAS), Nukleotide 3051 bis 3660 von SEQ ID Nr.1 (Mutation c.2525A>T zum Gen PDGFRA), Nukleotide 2445 bis 3050 von SEQ ID Nr.1 (Mutation c.1633G>A zum Gen PIK3CA), Nukleotide 1839 bis 2444 von SEQ ID Nr.1 (Mutation c.3140A>G zum Gen PIK3CA), Nukleotide 1227 bis 1838 von SEQ ID Nr.1 (Mutation c.2753T>C zum Gen RET), Nukleotide 1 bis 610 von SEQ ID Nr.1 (Mutation c.524G>A zum Gen TP53), Nukleotide 611 bis 1226 von SEQ ID Nr.1 (Mutation c.1679T> A zum Gen KIT) oder Nukleotide 9154 bis 9854 von SEQ ID Nr.1 (Mutation c.394C>T zum Gen IDH1) umfasst,
optional wobei jede Nukleotidsequenz der Vielzahl von Nukleotidsequenzen die Nukleotidsequenz aufweist, die die Nukleotide 8545 bis 9153 von SEQ ID Nr.1 (Mutation c.800delA zum Gen PTEN), die Nukleotide 7937 bis 8544 von SEQ ID Nr.1 (Mutation c. 1799T>A zum Gen BRAF), Nukleotide 7327 bis 7936 von SEQ ID Nr.1 (Mutation c.121A>G zum Gen CTNNB1), Nukleotide 6712 bis 7326 von SEQ ID Nr.1 (Mutation c.2573T>G zum Gen EGFR), Nukleotide 6115 bis 6711 von SEQ ID Nr.1 (Mutation c.2236 2250del15 zum Gen EGFR), Nukleotide 5503 bis 6114 von SEQ ID Nr.1 (Mutation c.2369C>T zum Gen EGFR), Nukleotide 4879 bis 5502 von SEQ ID Nr.1 (Mutation c.2324 2325ins12 zum Gen ERBB2), Nukleotide 4271 bis 4878 von SEQ ID Nr.1 (Mutation c.35G>A zum Gen KRAS), Nukleotide 3661 bis 4270 von SEQ ID Nr.1 (Mutation c.182A>G zum Gen NRAS), Nukleotide 3051 bis 3660 von SEQ ID Nr.1 (Mutation c.2525A>T zum Gen PDGFRA), Nukleotide 2445 bis 3050 von SEQ ID Nr.1 (Mutation c.1633G>A zum Gen PIK3CA), Nukleotide 1839 bis 2444 von SEQ ID Nr.1 (Mutation c.3140A>G zum Gen PIK3CA), Nukleotide 1227 bis 1838 von SEQ ID Nr.1 (Mutation c.2753T>C zum Gen RET), Nukleotide 1 bis 610 von SEQ ID Nr.1 (Mutation c.524G>A zum Gen TP53), Nukleotide 611 bis 1226 von SEQ ID Nr.1 (Mutation c.1679T>A zum Gen KIT) oder Nukleotide 9154 bis 9854 von SEQ ID Nr.1 (Mutation c.394C>T zum Gen IDH1) umfasst; und/oder (c) die Nukleinsäure mindestens 80 % Sequenzhomologie mit der in SEQ ID Nr.1 dargelegten Nukleotidsequenz aufweist oder
die in SEQ ID Nr.1 dargelegte Nukleotidsequenz aufweist.

5. Die Nukleinsäure nach einem der Ansprüche 1, 2 oder 3 Teile (a)-(b), wobei:
(a) jede Nukleotidsequenz der Vielzahl von Nukleotidsequenzen eine Teilsequenz eines Gens ist, ausgewählt aus ABI1, ABL1, ABL2, ACSL3, ACUR1, AF15Q14, AF1Q, AF3P21, AF5Q31, AKAP9, AKT1, AKT2, AKT3, AL017, ALDH2, ALK, AMER1, APC, APEX1, AR, AR1D1A, ARAF, ARHGAP5, ARHGEF12, ARHH, ARID1A, ARID2, ARNT, ASPSCR1, ASXL1, ATF1, ATIC, ATM, ATP11B, ATP1A1, ATP2B3, ATR, ATRX, AXIN1, BAP1, BCL10, BCL11A, BCL11B, BCL2, BCL2L1, BCL3, BCL5, BCL6, BCL7A, BCL9, BCOR, BCR, BHD, BIRC2, BIRC3, BLM, BMPR1A, BRAF, BRC42, BRCA1, BRCA2, BRD3, BRD4, BRIP1, BTG1, BUB1B, C12ORF9, C15ORF21, C15ORF55, C160RF75, C2ORF44, CACNA1D, CALR, CAMTA1, CANT1, CARD11, CARS, CASP8, CBFA2T1, CBFA2T3, CBFB, CBL, CBLB, CBLC, CCDC6, CCNB1IP1, CCND1, CCND2, CCND3, CCNE1, CD273, CD274, CD44, CD74, CD79A, CD79B, CDC73, CDH1, CDH11, CDK12, CDK4, CDK6, CDKN2A, CDKN2A(PL4), CDKN2B, CDKN2C, CDX2, CEBPA, CEP1, CEP89, CHCHD7, CHEK2, CHIC2, CHN1, CIC, CIITA, CLIP1, CLTC, CLTCL1, CMKOR1, CNOT3, COL1A1, COL2A1, COPEB, COX6C, CREB1, CREB3L1, CREB3L2, CREBBP, CRLF2, CRTC3, CSF1R, CSF3R, CSNK2A1, CTNNB1, CUX1, CYLD, D10S170, DAXX, DCTN1, DDB2, DDIT3, DDR2, DDX10, DDX5, DDX6, DEK, DICERL, DNM2, DNMT3A, DUX4, EBFL, ECT2L, EGFR, EIF3E, EIF4A2, ELF4, ELK4, ELKS, ELL, ELN, EML4, EP300, EPS 15, ERBB2, ERBB2 ("HER2"), ERBB3, ERBB4 ("HER4"), ERC1, ERCC1, ERCC2, ERCC3, ERCC4, ERCC5, ERG, ESR1, ETV1, ETV4, ETV5, ETV6, EVI1, EWSR1, EXT1, EXT2, EZH2, EZR, FACL6, FAM46C, FANCA, FANCC, FANCD2, FANCE, FANCF, FANCG, FANCL, FAS, FBXOI1, FBXW7, FCGR2B, FEV, FGFR1, FGFR10P, FGFR2, FGFR3, FGFR4, FH, FHIT, FIP1L1, FLJ27352, FLT3, FLU, FNBP1, FOX03A, FOX04, FOXA1, FOXL2, FOXOIA, FOXP1, FSTL3, FUBP1, FUS, FVT1, GABRA6, GABRG2, GAS6, GAS7, GATA1, GATA2, GATA3, GMPS, GNA11, GNAQ, GNAS, GOLGA5, GOPC, GPC3, GPHN, GRAF, H3F3A, H3F3B, HCMOGT-1, HEAB, HERPUD1, HEY1, HIP1, HIST1H3B, HIST1H4L, HLA-A, HLF, HLXB9, HMGA1, HMGA2, HNF1A, HNRNPA2B1, HOOK3, HOXA11, HOXA13, HOXA9, HOXC11, HOXC13, HOXD11, HOXD13, HPAS, HRAS, HSPCA, HSPCB, IDH1, IDH2, IGF1R, IGH, IGK, IGL, IHD2, IKZF1, IL2, IL21R, IL6, IL6ST, IL7R, IRF4, IRTA1, ITK, JAK1, JAK2, JAK3, JAZF1, JUN, KCNJ5, KDM5A, KDM5C, KDM6A, KDR, KIAA1549, KIAA1598, KIF5B, KIT, KLF4, KLK2, KMT2A, KMT2D, KRAS, KTN1, LAF4, LASP1, LCK, LCP1, LCX, LHFP, LIFR, LM02, LMNA, LMOL, LPP, LRIG3, LSM14A, LYL1, MAF, MAFB, MALAT1, MALT1, MAML2, MAP2K1, MAP2K2, MAP2K4, MAX, MCL1, MDM2, MDM4, MDS1, MDS2, MECOM, MECT1, MED 12, MEN1, MET, MITF, MKL1, MLF1, MLH1, MLL, MLL3, MLLT1, MLLT10, MLLT2, MLLT3, MLLT4, MLLT6, MLLT7, MN1, MPL, MSF, MSH2, MSH6, MSI2, MSN, MTCP1, MTOR, MUC1, MUTYH, MY018A, MY05A, MYB, MYC, MYC1, MYCL1, MYCN, MYD88, MYH11, MYH9, MYST4, NAB2, NACA, NBSL, NCOA1, NCOA2, NCOA4, NDRG1, NF1, NF2, NFATC2, NFE2L2, NFIB, NFKB2, NIN, NKX2-1, NKX2-1, NKX2-8, NONO, NOTCH1, NOTCH2, NPM1, NR4A3, NRAS, NRAS/CSDE1, NRG1, NSD1, NT5C2, NTRK1, NTRK3, NUMA1, NUP214, NUP98, NUTM2A, NUTM2B, OLIG2, OMD, P2RY8, PAFAH1B2, PALB2, PARP1, PARP2, PAX3, PAX5, PAX7, PAX8, PBRM1, PBX1, PCM1, PCSK7, PDCD1LG2, PDE4DIP, PDGFB, PDGFRA, PDGFRB, PERI, PHF6, PHOX2B, PICALM, PIK3CA, PIK3R1, PIM1, PLAG1, PLCG1, PML, PMS1, PMS2, PMX1, PNP, PNUTL1, POT1, POU2AF1, POU5F1, PPARG, PPFIBP1, PPP2R1A, PRCC, PRDM1, PRDM16, PRF1, PRKAR1A, PSIP1, PTCH1, PTEN, PTPN11, PTPRB, PTPRC, PTPRK, PWWP2A, RAB5EP, RAC1, RAD21, RAD51, RAD51L1, RAF1, RAFT, RALGDS, RANBP17, RAP1GDS1, RARA, RB1, RBI, RBM15, RECQL4, REL, RET, RHEB, RHOA, RIT1, RNF43, ROS1, RPL10, RPL22, RPL5, RPN1, RPS6KB1, RSP02, RSP03, RUNDC2A, RUNX1, RUNX1T1, RUNXBP2, SBDS, SDC4, SDH5, SDHB, SDHC, SDHD, SET, SETBP1, SETD2, SF3B1, SFPQ, SFRS3, SH2B3, SH3GL1, SIL, SLC34A2, SLC45A3, SMAD4, SMARCA4, SMARCB1, SMARCE1, SMO, SOCS1, SOX2, SRC, SRGAP3, SRSF2, SS18, SS18L1, SSX1, SSX2, SSX4, STAG2, STAT3, STAT5B, STAT6, STK11, STL, SUFU, SUZ12, SYK, TAF15, TAL1, TAL2, TALI, TBL1XR1, TCEA1, TCF1, TCF12, TCF3, TCF7L2, TCL1A, TCL6, TERT, TET2, TFE3, TFEB, TFG, TERT, TFRC, THRAP3, TIAF1, TIF1, TLX1, TLX3, TMPRSS2, TNFAIP3, TNFRSF14, TNFRSF17, TOPI, TP53, TPM3, TPM4, TPR, TRA, TRAF7, TRB, TRD, TRIM27, TRIM33, TRIP11, TRRAP, TSC1, TSC2, TSHR, TTL, U2AF1, UBR5, USP6, VHL, VT11A, WAS, WHSC1, WHSC1L1, WIF1, WRN, WT1, WWTR1, XPA, XPC, XPO1, YWHAE, ZCCHC8, ZNF145, ZNF198, ZNF217, ZNF278, ZNF331, ZNF384, ZNF521, ZNF9, ZRSR2, und ZRSR2;
(b) jede Nukleotidsequenz der Vielzahl von Nukleotidsequenzen eine Teilsequenz eines Gens ist, ausgewählt aus BMPRIA (Bone MorphoGentic Protein Receptor, Typ IA), BRCA1 (Brustkrebs 1), BRCA2 (Brustkrebs 2), CDH1 (Cadherin-1), CDKN2A (Cyclinabhängiger Kinase-Inhibitor 2A), EPCAM (Epithelzelladhäsionsmolekül), MLH1 (mutL-Homolog 1), MSH2 (mutS-Homolog 2), MSH6 (mutS-Homolog 6), MUTYH (mutY-DNA-Glykosylase)), PALB2 (Partner und Lokalisierer von BRCA2), PMS2 (PMS1-Homolog 2, Komponente des Mismatch-Reparatursystems), PTEN (Phosphatase- und Tensin-Homolog), SMAD4 (SMAD-Familienmitglied 4; Mütter gegen dekapentaplegisches Homolog 4), STK11 (Serin/Threonin Kinase 11) und TP53 (Tumorprotein p53);
(c) die Nukleinsäure wie in Teil (b) dargelegt ist und jeder Genotyp einer Nukleotidsequenz der Vielzahl von Nukleotidsequenzen ausgewählt ist aus der Gruppe bestehend aus Mutation 942+3A>T zum Gen MSH2, Mutation c.1662-121677del zum Gen MSH2, Mutation c.2056_2060delinsCTTCTACCTCAAAAA (SEQ ID Nr.13) zum Gen MSH6, Mutation c.2308_2312delGGTAAinsT zum Gen MSH6, Mutation c.2641delGinsAAAA zum Gen MSH6, Mutation c.3163_3164insG zum Gen MSH6, Mutation c.232_243delinsATGTAAGG zum Gen MLH1, Mutation c.1852_1854delAAG zum Gen MLH1, Mutation c.2445+1G>C zum Gen PMS2, Mutation c.2243_2246delAGAA zum Gen PMS2, Mutation c.2253T>C zum Gen PMS2, Mutation c.861_864delACAG zum Gen PMS2, Mutation c.9_32dup24 zum Gen CDKN2A, Mutation c.8975_9100del126 zum Gen BRCA2, Mutation c.9203del126 zum Gen BRCA2, Mutation c.1310_1313delAAGA zum Gen BRCA2, Mutation c.1813dupA zum Gen BRCA2, Mutation c.9342_9343insAluY zum Gen BRCA2, Mutation c.5266_5267insC zum Gen BRCA 1, Mutation c.5177_5180delGAAA zum Gen BRCA1, Mutation c.3756_3759delGTCT zum Gen BRCA1, Mutation c.3481_3491delGAAGATACTAG (SEQ ID Nr.14) zum Gen BRCA1, Mutation c.3113A>G zum Gen BRCA1, Mutation c.3084_3094delTAATAACATTA (SEQ ID Nr.15) zum Gen BRCA1, Mutation c.2834_2836delinsC zum Gen BRCA1 und Mutation c.68_69delAG zum Gen BRCA1;
(d) die Nukleinsäure wie in Teil (b) oder (c) dargelegt ist und
die Nukleinsäure mindestens 80 % Sequenzhomologie mit der in SEQ ID Nr.2 oder SEQ ID Nr.3 dargelegten Nukleotidsequenz aufweist, oder
die in SEQ ID Nr.2 oder SEQ ID Nr.3 dargelegte Nukleotidsequenz aufweist;
(e) die Nukleinsäure wie in Teil (b) dargelegt ist und jeder Genotyp einer Nukleotidsequenz der Vielzahl von Nukleotidsequenzen ist ausgewählt aus der Gruppe bestehend aus Mutation c.675+1G>C zum Gen BMPRIA, Mutation c.817C>T zum Gen BMPR1A, Mutation c.1016dupA zum Gen BRCA1, Mutation c.1175_1214delTGTTAGGTTCTGATGACTCACATGATGGGGAGTCTGAATC (SEQ ID Nr.23) zum Gen BRCA1, Mutation c.1387_1390delinsGAAAG zum Gen BRCA1, Mutation C.181T> G zum Gen BRCA1, Mutation C.1953_1956delGAAA zum Gen BRCA1, Mutation c.2834_2836delinsC zum Gen BRCA1, Mutation c.3084_3094delTAATAACATTA (SEQ ID Nr.15) zum Gen BRCA1, Mutation c.3113A>G zum Gen BRCA1, Mutation c.3481_3491delGAAGATACTAG (SEQ ID Nr.14) zum Gen BRCA1, Mutation c.3756_3759delGTCT zum Gen BRCA1, Mutation c.406_407insA zum Gen BRCA1, Mutation c.4964_4982delCTGGCCTGACCCCAGAAGA (SEQ ID Nr.19) zum Gen BRCA1, Mutation c.5096G>A zum Gen BRCA1, Mutation c.5177_5180delGAAA zum Gen BRCA1, Mutation c.5177_5180delGAAA zum Gen BRCA1, Mutation c.5266_5267insC zum Gen BRCA1, Mutation c.68_69delAG zum Gen BRCA1, Mutation c.815_824dupAGCCATGTGG (SEQ ID Nr.25) zum Gen BRCA1, Mutation c.10095delCinsGAATTATATCT (SEQ ID Nr.29) zum Gen BRCA2, Mutation c.1310_1313delAAGA zum Gen BRCA2, Mutation c.1310_1313delAAGA zum Gen BRCA2, Mutation c.156_157insAluYa5 zum Gen BRCA2, Mutation c.1813dupA zum Gen BRCA2, Mutation c.2588dupA zum Gen BRCA2, Mutation c.2808_2811delACAA zum Gen BRCA2, Mutation c.4037_4043delinsT zum Gen BRCA2, Mutation c.5073dupA zum Gen BRCA2, Mutation c.5350_5351delAA zum Gen BRCA2, Mutation c.5946delT zum Gen BRCA2, Mutation c.6275_6276delTT zum Gen BRCA2, Mutation c.7762_7764delinsTT zum Gen BRCA2, Mutation c.8537_8538delAG zum Gen BRCA2, Mutation c.8902_8913delinsTCCC zum Gen BRCA2, Mutation c.9203del126 zum Gen BRCA2, Mutation c.9253dupA zum Gen BRCA2, Mutation c.9342_9343insAluY zum Gen BRCA2, Mutation c.1792C>T zum Gen CDH1, Mutation c.9_32dup24 zum Gen CDKN2A, Mutation c.429G>A zum Gen EPCAM, Mutation c.523C> T zum Gen EPCAM, Mutation c.556-14A>G zum Gen EPCAM, Mutation c.1852_1854delAAG zum Gen MLH1, Mutation c.1852_1854delAAG zum Gen MLH1, Mutation c.232_243delinsATGTAAGG zum Gen MLH1, Mutation c.382delG zum Gen MLH1, Mutation c.704_723delATAAAACCCTAGCCTTCAAA (SEQ ID Nr.33) zum Gen MLH1, Mutation c.1076G>C zum Gen MSH2, Mutation c.1662-12_1677del zum Gen MSH2, Mutation c.942+3A>T zum Gen MSH2, Mutation c. 942+3A>T zum Gen MSH2, Mutation c.2056_2060delinsCTTCTACCTCAAAAA (SEQ ID Nr.13) zum Gen MSH6, Mutation c.2308_2312delGGTAAinsT zum Gen MSH6, Mutation c.2641delGinsAAAA zum Gen MSH6, Mutation c.3163_3164insG zum Gen MSH6, Mutation c.3261delC zum Gen MSH6, Mutation c.3939_3957dupTCAAAAGGGACATAGAAAA (SEQ ID Nr.36) zum Gen MSH6, Mutation c.741delA zum Gen MSH6, Mutation c.1147delC zum Gen MUTYH, Mutation c.1187G>A zum Gen MUTYH, Mutation c.1435G>T zum Gen MUTYH, Mutation c.536A>G zum Gen MUTYH, Mutation c.933+3A>C zum Gen MUTYH, Mutation c.2386G>T zum Gen PALB2, Mutation c.3113G>A zum Gen PALB2, Mutation c.1261C>T zum Gen PMS2, Mutation c.137G>T zum Gen PMS2, Mutation c.2117delA zum Gen PMS2, Mutation c.2243_2246delAGAA, c.2253T>C zum Gen PMS2, Mutation c.2445+1G>C zum Gen PMS2, Mutation c.861_864delACAG zum Gen PMS2, Mutation c.861_864delACAG zum Gen PMS2, Mutation c.511C>T zum Gen PTEN, Mutation c.758_759insAT zum Gen PTEN, Mutation c.987_996dupTAAAGACAAA (SEQ ID Nr.38) zum Gen PTEN, Mutation c.1206_1207insT zum Gen SMAD4 , Mutation c.1353_1354insGCTACTGCACAAGCTGCAGCAGCTGCCC (SEQ ID Nr.40) zum Gen SMAD4, Mutation c.1529delG zum Gen SMAD4, Mutation c.842_843insC zum Gen STK11, Mutation c.637C>T zum Gen TP53 und Mutation c.916C>T zum Gen TP53.

6. Nukleinsäure nach Anspruch 1 oder 2, wobei:
(a) der Genotyp jeder Nukleotidsequenz der Vielzahl von Nukleotidsequenzen mit einer Herzerkrankung, optional Kardiomyopathie, verbunden ist;
(b) der Genotyp jeder Nukleotidsequenz der Vielzahl von Nukleotidsequenzen mit einer Herzerkrankung, optional Kardiomyopathie, verbunden ist, und
jede Nukleotidsequenz der Vielzahl von Nukleotidsequenzen eine Teilsequenz eines Gens umfasst, ausgewählt aus der Gruppe bestehend aus kardialem Myosin-bindendem Protein C (MYBPC3); Myosin, schwerer Kette 7, Herzmuskel, Beta (MYH7); Troponin I Typ 3 (TNNI3); kardialem Troponin T (TNNT2); und Tropomyosin-Alpha-1-Kette (TPM1); und
jede Teilsequenz eine Mutation umfasst, die mit Kardiomyopathie assoziiert ist;
optional
wobei jede Mutation aus der Gruppe ausgewählt ist, bestehend aus:
einer Mutation des Gens für kardiales Myosin-bindendes Protein C (MYBPC3), ausgewählt aus der Gruppe bestehend aus 1504C>T, 2373_2374insG und 3628-41_3628-17del;
einer Mutation des Gens für Myosin, schwere Kette 7, Herzmuskel, Beta (MYH7), ausgewählt aus der Gruppe bestehend aus 1988G>A, 1357C>T und 1750G>C;
einer Mutation des Gens für Troponin I Typ 3 (TNNI3), ausgewählt aus der Gruppe bestehend aus 532_534delAAG und 575G>A;
einer Mutation im Gen für kardiales Troponin T (TNNT2), wobei die Mutation 487_489delGAG ist; und
einer Mutation im Gen für die Tropomyosin-Alpha-1-Kette (TPM1), wobei die Mutation 574G>A ist;
(c) der Genotyp jeder Nukleotidsequenz der Vielzahl von Nukleotidsequenzen mit einer Herzerkrankung, optional Kardiomyopathie, verbunden ist, und
jede Nukleotidsequenz der Vielzahl von Nukleotidsequenzen eine Teilsequenz eines Gens ist, ausgewählt aus MYBPC3, MYH7, TNNI3, TNNT2 und TPM1, optional
wobei jeder Genotyp einer Nukleotidsequenz der Vielzahl von Nukleotidsequenzen aus der Gruppe bestehend aus Mutation 1504C>T zum Gen MYBPC3, Mutation 2373_2374insG zum Gen MYBPC3, Mutation 3628-41_3628-17del zum Gen MYBPC3, Mutation 1988G>A zum Gen MYH7, Mutation 1357C>T zum Gen MYH7, Mutation 1750G>C zum Gen MYH7, Mutation 532_534delAAG zum Gen TNNI3, Mutation 575G>A zum Gen TNNI3, Mutation 487_489delGAG zum Gen TNNT2 und Mutation 574G> A zum Gen TPM1 ausgewählt ist;
(d) der Genotyp jeder Nukleotidsequenz der Vielzahl von Nukleotidsequenzen mit einer Herzerkrankung, optional Kardiomyopathie, verbunden ist, und
jede Nukleotidsequenz der Vielzahl von Nukleotidsequenzen eine Teilsequenz eines Gens ist, ausgewählt aus ABCC9, ACTC1, ACTN2, AKAP9, ANK2, ANKRD1, BAG3, BRAF, CACNA1C, CACNB2, CASQ2, CAV3, CRYAB, CSRP3, DES, DMD, DSC2, DSG2, DSP, DTNA, EMD, FKTN, GATAD1, GLA, GPD1L, HCN4, HRAS, ILK, JPH2, JUP, KCNE1, KCNE2, KCNE3, KCNH2, KCNJ2, KCNJ5, KCNJ8, KCNQ1, KRAS, LAMA4, LAMP2, LDB3, LMNA, MAP2K1, MAP2K2, MTND1, MTND5, MTND6, MTTD, MTTG, MTTH, MTTI, MTTK, MTTL1, MTTL2, MTTM, MTTQ, MTTS1, MTTS2, MYBPC3, MYH7, MYL2, MYL3, MYLK2, MYOZ2, MYPN, NEBL, NEXN, NKX2.5, NRAS, PDLIM3, PKP2, PLN, PRKAG2, PTPN11, RAF1, RANGRF, RBM20, RYR2, SCN1B, SCN3B, SCN4B, SCN5A, SGCD, SNTA1, SOS1, TAZ, TCAP, TMEM43, TMPO, TNNC1, TNNI3, TNNT2, TPM1, TTN, TTR, und VCL; und/oder
(e) die Nukleinsäure mindestens 80 % Sequenzhomologie mit der in SEQ ID Nr.4 dargestellten Nukleotidsequenz aufweist oder die in SEQ ID Nr.4 dargelegte Nukleotidsequenz aufweist.

7. Nukleinsäure nach einem der Ansprüche 1 bis 6, wobei:
(a) jede Nukleotidsequenz der Vielzahl mindestens 600 Nukleotide lang,
etwa 600 Nukleotide bis etwa 2000 Nukleotide lang,
etwa 600 Nukleotide bis etwa 1200 Nukleotide lang,
etwa 1000 Nukleotide bis etwa 10.000 Nukleotide lang ist; und/oder
(b) die Nukleinsäure optional weiterhin einen Replikationsursprung umfasst,
wobei der Replikationsursprung ein Replikationsursprung von *Escherichia coli* oder *Saccharomyces cerevisiae* ist und/oder
(c) die Nukleinsäure weiterhin mindestens ein methyliertes Nukleosid oder Nukleotid umfasst; und/oder
(d) die Nukleinsäure weiterhin einen Promotor umfasst, optional wobei der Promotor ein SP6-Promotor ist; und/oder
(e) die Nukleinsäure weiterhin einen Promotor umfasst, wobei der Promotor optional von einer anderen Spezies als die Nukleotidsequenzen der Vielzahl stammt wobei der Promotor ein SP6-Promotor ist; und/oder die Nukleinsäure ein Plasmid ist.

8. Zelle, die die Nukleinsäure nach einem der Ansprüche 1 bis 7 umfasst.

9. Zelle nach Anspruch 8, wobei:
(a) die Zelle eine menschliche Zelle ist; und/oder
(b) wobei die Zelle ein Fibroblast oder ein Lymphozyt, optional
ein immortalisierter B-Lymphozyten ist; und/oder
(c) die Zelle GM12878, GM24149, GM24143, GM24385, GM24631, GM24694 oder GM24695, optional GM24385 ist; und/oder
(d) die Zelle 1 bis 1000 Kopien oder 5 bis 500 Kopien der Nukleinsäure umfasst; oder
(e) die Zelle *E. coli* ist.

10. Zusammensetzung, umfassend eine erste Vielzahl von Zellen und eine zweite Vielzahl von Zellen, wobei:
die erste Vielzahl von Zellen aus Zellen gemäß Anspruch 9 Teil (d) besteht, wobei die Zellen 5 bis 500 Kopien der Nukleinsäure umfassen;
die zweite Vielzahl von Zellen aus Zellen besteht, die die Nukleinsäure nicht umfassen;
die erste Vielzahl von Zellen und die zweite Vielzahl von Zellen menschliche Zellen sind;
die erste Vielzahl von Zellen und die zweite Vielzahl von Zellen in der Zusammensetzung vermischt werden; und
das Verhältnis der Anzahl der Zellen der ersten Vielzahl zur Anzahl der Zellen der zweiten Vielzahl in der Zusammensetzung etwa 1:1 bis etwa 1:10.000 beträgt.

11. Verfahren zur Herstellung eines biologischen Referenzmaterials, umfassend die Transfektion einer Vielzahl von Zellen mit der Nukleinsäure nach einem der Ansprüche 1 bis 7; optional
weiter umfassend:
(a) Fixieren der Zellen der Vielzahl, optional wobei das Fixieren der Zellen das Fixieren der Zellen mit Formalin umfasst; und/oder
(b) Einbetten der Zellen in Paraffin; und/oder
(c) Verdünnen der Vielzahl von Zellen mit nicht transfizierten Zellen, optional
wobei das Verdünnen der Vielzahl von Zellen mit nicht transfizierten Zellen das Verdünnen in einem Verhältnis von transfizierten Zellen zu nicht transfizierten Zellen von etwa 1:1 bis etwa 1:10.000 umfasst.

12. Biologisches Referenzmaterial, umfassend
(a) eine Vielzahl von Zellen nach einem der Ansprüche 8 oder 9, Teile (a)-(d) und Paraffin, wobei die Vielzahl von Zellen im Paraffin fixiert und eingebettet ist, optional weiterhin umfassend nicht transfizierte Zellen, wobei die nicht transfizierten Zellen nicht die Nukleinsäure umfassen, optional wobei das Verhältnis von Zellen, die die Nukleinsäure umfassen, zu nicht transfizierten Zellen etwa 1:1 bis etwa 1:10.000, etwa 1:5 bis etwa 1:5.000 oder etwa 1:10 bis etwa 1:1.000 beträgt; oder
(b) eine Vielzahl von Zellen nach einem der Ansprüche 8 oder 9, Teile (a)-(d); und eine Flüssigkeit, optional wobei die Flüssigkeit Plasma ist.

13. Zusammensetzung, die eine Nukleinsäure und einen wässrigen Puffer umfasst, wobei die Nukleinsäure eine Nukleinsäure nach einem der Ansprüche 1 bis 7 ist, die aus dem Referenzmaterial nach Anspruch 12 extrahiert wurde, optional
wobei der Puffer Tris(hydroxymethyl)aminomethan und Ethylendiamintetraessigsäure oder ein Salz von einem der Vorstehenden umfasst.

## Revendications

1. Acide nucléique, comprenant une pluralité de séquences nucléotidiques, dans lequel chaque séquence nucléotidique de la pluralité comprend un génotype qui est associé à une maladie ou un état pathologique ; chaque séquence nucléotidique a une longueur de 600 à 10 000 paires de bases ; et la pluralité de séquences nucléotidiques comprend des séquences nucléotidiques provenant d'au moins 2 chromosomes différents.

2. Acide nucléique selon la revendication 1, dans lequel :
(a) le génotype de chaque séquence nucléotidique de la pluralité est une mutation somatique ou une mutation héréditaire ; et/ou
(b) le génotype de chaque séquence nucléotidique de la pluralité est un polymorphisme d'un seul nucléotide, une mutation ponctuelle, un codon d'arrêt prématuré, une répétition trinucléotidique, une translocation, un réarrangement somatique, un allélomorphe, un variant d'un seul nucléotide, une insertion ou une délétion (« indel »), un variant régulateur ou une fusion de gène ; et/ou
(c) la pluralité de séquences nucléotidiques comprend des séquences nucléotidiques provenant d'au moins 3 chromosomes différents ; et/ou
(d) l'acide nucléique est l'ADN ; et/ou
(e) la pluralité de séquences nucléotidiques comprend des séquences nucléotidiques provenant d'au moins 3 ou d'au moins 5 chromosomes différents ; et/ou
(f) chaque séquence nucléotidique de la pluralité est une sous-séquence d'un gène ou d'une région régulatrice de celui-ci, facultativement dans lequel chaque séquence nucléotidique de la pluralité est une sous-séquence d'un gène, facultativement dans lequel chaque séquence nucléotidique de la pluralité est une sous-séquence d'un exon, facultativement dans lequel le génotype de chaque séquence nucléotidique de la pluralité provoque une mutation, une délétion ou une insertion d'au moins un acide aminé dans la séquence d'acides aminés codée par l'exon ; et/ou
(g) l'acide nucléique est tel qu'indiqué en (e) ou (f) et chaque gène est un gène humain ; et/ou
(h) chaque séquence nucléotidique de la pluralité est une séquence nucléotidique humaine.

3. Acide nucléique selon la revendication 1 ou 2, dans lequel :
(a) le génotype de chaque séquence nucléotidique de la pluralité de séquences nucléotidiques est associé à un néoplasme, facultativement à une tumeur solide ; et/ou
(b) le génotype de chaque séquence nucléotidique de la pluralité de séquences nucléotidiques est associé à un néoplasme qui est un cancer du poumon, un cancer lymphoïde, un cancer lymphoïde aigu
la leucémie, la leucémie myéloïde aiguë, la leucémie myéloïde chronique, le cancer de la thyroïde, le cancer de l'estomac, le cancer du gros intestin, le cancer des voies urinaires, le cancer du système nerveux central, le cancer du rein, le cancer du sein et/ou le cancer des tissus mous, facultativement une tumeur solide ; et/ou
(c) le génotype de chaque séquence nucléotidique de la pluralité de séquences nucléotidiques est associé à un néoplasme en (a) ou (b) et chaque séquence nucléotidique de la pluralité de séquences nucléotidiques est une sous-séquence d'un gène sélectionné dans le groupe constitué de ABL1, AKT1, AKT2, AKT3, ALK, APC, AR, AR1D1A, ARAF, ARHGAP5, ATM, ATR, BCL2, BCR, BRAF, BRC42, BRCA1, BRCA2, BRIP1, CBFB, CCND1, CCND2, CCNE1, CDH1, CDK4, CDK6, CDKN2A, CDKN2B, CSF1R, CTNNB1, DDR2, DNMT3A, EGFR, ERBB2 (« HER2 »), ERBB3, ERBB4 (« HER4 »), ERCC1, ESR1, ETV1, ETV4, ETV6, EWSR1, EZH2, FANCA, FANCC, FANCD2, FANCE, FANGE, FANCG, FANCL, FBXW7, FGFR1, FGFR2, FGFR3, FLT3, FOXL2, GABRA6, GABRG2, GATA3, GNA11, GNAQ, GNAS, HNF1A, HPAS, HRAS, IDH1, IDH2, IHD2, JAK2, JAK3, KDR, KIT, KMT2A, KRAS, MAP2K1, MAP2K2, MECOM, MET, MKL1, MLH1, MLL, MPL, MSH2, MSH6, MTOR, MYC, MYCN, MYD88, NF1, NF2, NFE2L2, NOTCH1, NPM1, NRAS/CSDE1, NTRK1, NUP214, PALB2, PARP1, PARP2, PDGFRA, PDGFRB, PICALM, PIK3CA, PMS2, PTCH1, PTEN, PTPN11, RAD51, RAFT, RARA, RB1, RET, RHEB, RHOA, RIT1, ROS1, RUNX1, RUNX1T1, SMAD4, SMARCB1, SMO, SRC, STK11, TAL1, TCF3, TERT, TMPRSS2, TP53, TSC1, TSC2 et VHL ; et/ou
(d) le génotype de chaque séquence nucléotidique de la pluralité de séquences nucléotidiques est associé à un néoplasme comme en (a) ou (b).
et chaque séquence nucléotidique de la pluralité de séquences nucléotidiques est une sous-séquence d'un gène sélectionné dans le groupe constitué de AKT1, AKT2, AKT3, APC, ARHGAP5, ATM, ATR, BRAF, CTNNB1, DNMT3A, EGFR, ERBB2, ERCC1, EZH2, FBXW7, FGFR3, FLT3, GABRA6, GABRG2, GNAQ, GNAS, IDH1, IDH2, JAK2, KIT, KRAS, MET, MLH1, MPL, MSH2, MTOR, MYD88, NF1, NPM1, NRAS/CSDE1, PARP1, PARP2, PDGFRA, PIK3CA, PTEN, PTPN11, RAD51, RB1, RET, SMAD4, TP53 et VHL, facultativement dans lequel le génotype de chaque séquence nucléotidique de la pluralité de séquences nucléotidiques est sélectionné dans le groupe constitué de la mutation 145G>A au gène AKT1, de la mutation c.49G>A au gène AKT1, de la mutation c.268G>T au gène AKT2, de la mutation c.371A>T au gène AKT3, de la mutation c.4248delC au gène APC, de la mutation c.4348C>T au gène APC, de la mutation c.4666_4667insA au gène APC, de la mutation c.1864G>A au gène ARHGAP5, de la mutation c.1058_1059delGT au gène ATM, de la mutation c.5557G>A au gène ATM, de la mutation c.3790_3796delATAAAAG au gène ATR, de la mutation c.1799T>A au gène BRAF, de la mutation c.121A>G au gène CTNNB1, de la mutation c.2644C>T au gène DNMT3A, de la mutation c.2236_2250del15 au gène EGFR, de la mutation c.2310_2311insGGT au gène EGFR, de la mutation c.2369C>T au gène EGFR, de la mutation c.2573T>G au gène EGFR, de la mutation c.2324_2325ins12 au gène ERBB2, de la mutation c.287C>A au gène ERCC1, de la mutation c.1937A>T au gène EZH2, de la mutation c.1394G>A au gène FBXW7, de la mutation c.746C>G au gène FGFR3, de la mutation c.2503G>T au gène FLT3, de la mutation c.763G>C au gène GABRA6, de la mutation c.1355A>G au gène GABRG2, de la mutation c.626A>C au gène GNAQ, de la mutation c.601C>T au gène GNAS, de la mutation c.394C>T au gène IDH1, de la mutation c.515G>A au gène IDH2, de la mutation c.419G>A au gène IDH2, de la mutation c.1849G>T au gène JAK2, de la mutation c.2447A>T au gène KIT, de la mutation c.1679T>A au gène KIT, de la mutation c.35G>A au gène KRAS, de la mutation c.3757T>G au gène MET, de la mutation c.1151T>A au gène MLH1, de la mutation c.1544G>T au gène MPL, de la mutation c.2250delG au gène MSH2, de la mutation c.2359_2360delCT au gène MSH2, de la mutation c.2664A>T au gène MTOR, de la mutation c.794T>C au gène MYD88, de la mutation c.2987_2988insAC au gène NF1, de la mutation c.4084C>T au gène NF1, de la mutation c.7501delG au gène NF1, de la mutation c.863_864insTCTG au gène NPM1, de la mutation c.182A>G au gène NRAS, de la mutation c.2738delG au gène PARP1, de la mutation 398A>C au gène PARP2, de la mutation c.1694_1695insA au gène PDGFRA, de la mutation c.2525A>T au gène PDGFRA, de la mutation c.1633G>A au gène PIK3CA, de la mutation c.3140A>G au gène PIK3CA, de la mutation c.3204_3205insA au gène PIK3CA, de la mutation c.388C>T au gène PTEN, de la mutation c.741_742insA au gène PTEN, de la mutation c.800delA au gène PTEN, de la mutation c.226G>A au gène PTPN11, de la mutation c.433C>T au gène RAD51, de la mutation c.958C>T au gène RB1, de la mutation c.2753T>C au gène RET, de la mutation c.1394_1395insT au gène SMAD4, de la mutation c.818G>A au gène TP53, de la mutation c.743G> A au gène TP53, de la mutation c.723delC au gène TP53, de la mutation c.524G>A au gène TP53, de la mutation 263delC au gène TP53 et de la mutation c.426_429delTGAC au gène VHL ; et/ou
(e) l'acide nucléique est tel qu'indiqué dans la partie (d) et
chaque séquence nucléotidique de la pluralité de séquences nucléotidiques est une sous-séquence d'un gène et est sélectionnée dans le groupe constitué de BRAF, CTNNB1, EGFR, ERBB2, IDH1, KIT, KRAS, NRAS/CSDE1, PDGFRA, PIK3CA, PTEN, RET et TP53 ; et/ou
(f) l'acide nucléique est tel qu'indiqué dans la partie (d) ou (e) et
la pluralité de séquences nucléotidiques comprend au moins 5 séquences nucléotidiques ;
chaque séquence nucléotidique de la pluralité de séquences nucléotidiques est une sous-séquence d'un gène humain ; et
chaque gène est sélectionné dans le groupe constitué de BRAF, CTNNB1, EGFR, ERBB2, IDH1, KIT, KRAS, NRAS/CSDE1, PDGFRA, PIK3CA, PTEN, RET et TP53 ; et/ou
(g) l'acide nucléique est tel qu'indiqué dans la partie (e) ou (f) et
le génotype de chaque séquence nucléotidique de la pluralité de séquences nucléotidiques est sélectionné dans le groupe constitué de la mutation c.1799T>A au gène BRAF, de la mutation c.121A>G au gène CTNNB1, de la mutation c.2236_2250del15 au gène EGFR, de la mutation c.2369C>T au gène EGFR, de la mutation c.2573T>G au gène EGFR, de la mutation c.2324_2325ins12 au gène ERBB2, de la mutation c.394C>T au gène IDH1, de la mutation c.1679T>A au gène KIT, de la mutation c.35G>A au gène KRAS, de la mutation c.182A>G au gène NRAS/CSDE1, de la mutation c.2525A>T au gène PDGFRA, de la mutation c.1633G>A au gène PIK3CA, de la mutation c.3140A>G au gène PIK3CA, de la mutation c.800delA au gène PTEN, de la mutation c.2753T>C au gène RET et de la mutation c.524G>A au gène TP53 ; et/ou
(h) l'acide nucléique est tel qu'indiqué dans la partie (g) et chaque séquence nucléotidique de la pluralité de séquences nucléotidiques présente au moins 95 % d'homologie de séquence avec la séquence nucléotidique couvrant les nucléotides 8545 à 9153 de SEQ ID NO:1 (mutation c.800delA au gène PTEN), les nucléotides 7937 à 8544 de SEQ ID NO:1 (mutation c.1799T>A au gène BRAF), les nucléotides 7327 à 7936 de SEQ ID NO:1 (mutation c.121A>G au gène CTNNB1), les nucléotides 6712 à 7326 de SEQ ID NO:1 (mutation c.2573T>G au gène EGFR), les nucléotides 6115 à 6711 de SEQ ID NO:1 (mutation c.2236_2250del15 au gène EGFR), les nucléotides 5503 à 6114 de SEQ ID NO:1 (mutation c.2369C>T au gène EGFR), les nucléotides 4879 à 5502 de SEQ ID NO:1 (mutation c.2324_2325ins12 au gène ERBB2), les nucléotides 4271 à 4878 de SEQ ID NO:1 (mutation c.35G>A au gène KRAS), les nucléotides 3661 à 4270 de SEQ ID NO:1 (mutation c.182A>G au gène NRAS), les nucléotides 3051 à 3660 de SEQ ID NO:1 (mutation c.2525A>T au gène PDGFRA), les nucléotides 2445 à 3050 de SEQ ID NO:1 (mutation c.2525A>T au gène PDGFRA), les nucléotides 2445 à 3050 de SEQ ID NO:1 (mutation c.1633G>A au gène PIK3CA), les nucléotides 1839 à 2444 de SEQ ID NO:1 (mutation c.3140A>G au gène PIK3CA), les nucléotides 1227 à 1838 de SEQ ID NO:1 (mutation c.2753T>C au gène RET), les nucléotides 1 à 610 de SEQ ID NO:1 (mutation c.524G>A au gène TP53), les nucléotides 611 à 1226 de SEQ ID NO:1 (mutation c.1679T>A au gène KIT), ou les nucléotides 9154 à 9854 de SEQ ID NO:1 (mutation c.394C>T au gène IDH1), facultativement dans lequel chaque séquence nucléotidique de la pluralité de séquences nucléotidiques présente la séquence nucléotidique couvrant les nucléotides 8545 à 9153 de SEQ ID NO:1 (mutation c.800delA au gène PTEN), les nucléotides 7937 à 8544 de SEQ ID NO:1 (mutation c.1799T>A au gène BRAF), les nucléotides 7327 à 7936 de SEQ ID NO:1 (mutation c.121A>G au gène CTNNB1), les nucléotides 6712 à 7326 de SEQ ID NO:1 (mutation c.2573T>G au gène EGFR), les nucléotides 6115 à 6711 de SEQ ID NO:1 (mutation c.2236_2250del15 au gène EGFR), les nucléotides 5503 à 6114 de SEQ ID NO:1 (mutation c.2369C>T au gène EGFR), les nucléotides 4879 à 5502 de SEQ ID NO:1 (mutation c.2324_2325ins12 au gène ERBB2), les nucléotides 4271 à 4878 de SEQ ID NO:1 (mutation c.35G>A au gène KRAS), les nucléotides 3661 à 4270 de SEQ ID NO:1 (mutation c.182A>G au gène NRAS), les nucléotides 3051 à 3660 de SEQ ID NO:1 (mutation c.2525A>T au gène PDGFRA), les nucléotides 2445 à 3050 de SEQ ID NO:1 (mutation c.1633G>A au gène PIK3CA), les nucléotides 1839 à 2444 de SEQ ID NO:1 (mutation c.3140A>G au gène PIK3CA), les nucléotides 1227 à 1838 de SEQ ID NO:1 (mutation c.2753T>C au gène RET), les nucléotides 1 à 610 de SEQ ID NO:1 (mutation c.524G>A au gène TP53), les nucléotides 611 à 1226 de SEQ ID NO:1 (mutation c.1679T>A au gène KIT), ou les nucléotides 9154 à 9854 de SEQ ID NO:1 (mutation c.394C>T au gène DH1).

4. Acide nucléique selon l'une quelconque des revendications 1 à 3, partie (g), dans lequel :
(a) la pluralité de séquences nucléotidiques comprend au moins 5 séquences nucléotidiques ;
chaque séquence nucléotidique de la pluralité de séquences nucléotidiques est une sous-séquence d'un gène humain ;
chaque gène est sélectionné dans le groupe constitué de BRAF, CTNNB1, EGFR, EERBB2, IDH1, KIT, KRAS, NRAS/CSDE1, PDGFRA, PIK3CA, PTEN, RET et TP53 ; et
le génotype de chaque séquence nucléotidique de la pluralité est sélectionné dans le groupe constitué de la mutation c.1799T>A au gène BRAF, de la mutation c.121A>G au gène CTNNB1, de la mutation c.2236_2250del15 au gène EGFR, de la mutation c.2369C>T au gène EGFR, de la mutation c.2573T>G au gène EGFR, de la mutation c.2324_2325ins12 au gène ERBB2, de la mutation c.394C>T au gène IDH1, de la mutation c.1679T>A au gène KIT, de la mutation c.35G>A au gène KRAS, de la mutation c.182A>G au gène NRAS/CSDE1, de la mutation c.2525A>T au gène PDGFRA, de la mutation c.1633G>A au gène PIK3CA, de la mutation c.3140A>G au gène PIK3CA, de la mutation c.800delA au gène PTEN, de la mutation c.2753T>C au gène RET et de la mutation c.524G>A au gène TP53 ; et/ou
(b) l'acide nucléique est tel qu'indiqué dans la partie (a) et
chaque séquence nucléotidique de la pluralité de séquences nucléotidiques présente au moins 95 % d'homologie de séquence avec la séquence nucléotidique couvrant les nucléotides 8545 à 9153 de SEQ ID NO:1 (mutation c.800delA au gène PTEN), les nucléotides 7937 à 8544 de SEQ ID NO:1 (mutation c.1799T>A au gène BRAF), les nucléotides 7327 à 7936 de SEQ ID NO:1 (mutation c.121A>G au gène CTNNB1), les nucléotides 6712 à 7326 de SEQ ID NO:1 (mutation c.2573T>G au gène EGFR), les nucléotides 6115 à 6711 de SEQ ID NO:1 (mutation c.2236_2250del15 au gène EGFR), les nucléotides 5503 à 6114 de SEQ ID NO:1 (mutation c.2369C>T au gène EGFR), les nucléotides 4879 à 5502 de SEQ ID NO:1 (mutation c.2324_2325ins12 au gène ERBB2), les nucléotides 4271 à 4878 de SEQ ID NO:1 (mutation c.35G>A au gène KRAS), les nucléotides 3661 à 4270 de SEQ ID NO:1 (mutation c.182A>G au gène NRAS), les nucléotides 3051 à 3660 de SEQ ID NO:1 (mutation c.2525A>T au gène PDGFRA), les nucléotides 2445 à 3050 de SEQ ID NO:1 (mutation c.1633G>A au gène PIK3CA), les nucléotides 1839 à 2444 de SEQ ID NO:1 (mutation c.3140A>G au gène PIK3CA), les nucléotides 1227 à 1838 de SEQ ID NO:1 (mutation c.2753T>C au gène RET), les nucléotides 1 à 610 de SEQ ID NO:1 (mutation c.524G>A au gène TP53), les nucléotides 611 à 1226 de SEQ ID NO:1 (mutation c.1679T>A au gène KIT), ou les nucléotides 9154 à 9854 de SEQ ID NO:1 (mutation c.394C>T au gène IDH1), facultativement dans lequel chaque séquence nucléotidique de la pluralité de séquences nucléotidiques présente la séquence nucléotidique couvrant les nucléotides 8545 à 9153 de SEQ ID NO:1 (mutation c.800delA au gène PTEN), les nucléotides 7937 à 8544 de SEQ ID NO:1 (mutation c.1799T>A au gène BRAF), les nucléotides 7327 à 7936 de SEQ ID NO:1 (mutation 121A>G au gène CTNNB1), les nucléotides 6712 à 7326 de SEQ ID NO:1 (mutation c.2573T>G au gène EGFR), les nucléotides 6115 à 6711 de SEQ ID NO:1 (mutation c.2236_2250deI15 au gène EGFR), les nucléotides 5503 à 6114 de SEQ ID NO:1 (mutation c.2369C>T au gène EGFR), les nucléotides 4879 à 5502 de SEQ ID NO:1 (mutation c.2324_2325ins12 au gène ERBB2), les nucléotides 4271 à 4878 de SEQ ID NO:1 (mutation c.35G>A au gène KRAS), les nucléotides 3661 à 4270 de SEQ ID NO:1 (mutation c.182A>G au gène NRAS), les nucléotides 3051 à 3660 de SEQ ID NO:1 (mutation c.2525A>T au gène PDGFRA), les nucléotides 2445 à 3050 de SEQ ID NO:1 (mutation c.2525A>T au gène PDGFRA), les nucléotides 2445 à 3050 de SEQ ID NO:1 (mutation c.1633G>A au gène PIK3CA), les nucléotides 1839 à 2444 de SEQ ID NO:1 (mutation c.3140A>G au gène PIK3CA), les nucléotides 1227 à 1838 de SEQ ID NO:1 (mutation c.2753T>C au gène RET), les nucléotides 1 à 610 de SEQ ID NO:1 (mutation c.524G>A au gène TP53), les nucléotides 611 à 1226 de SEQ ID NO:1 (mutation c.1679T>A au gène KIT), ou les nucléotides 9154 à 9854 de SEQ ID NO:1 (mutation c.394C>T au gène IDH1) ; et/ou (c) l'acide nucléique présente au moins 80 % d'homologie de séquence avec la séquence nucléotidique présentée dans SEQ ID NO:1 ou
présente la séquence nucléotidique présentée dans SEQ ID NO:1.

5. Acide nucléique selon l'une quelconque des revendications 1, 2 ou 3, parties (a) et (b), dans lequel :
(a) chaque séquence nucléotidique de la pluralité de séquences nucléotidiques est une sous-séquence d'un gène sélectionné parmi ABI1, ABL1, ABL2, ACSL3, ACUR1, AF15Q14, AF1Q, AF3P21, AF5Q31, AKAP9, AKT1, AKT2, AKT3, AL017, ALDH2, ALK, AMER1, APC, APEX1, AR, AR1D1A, ARAF, ARHGAP5, ARHGEF12, ARHH, ARID1A, ARID2, ARNT, ASPSCR1, ASXL1, ATF1, ATIC, ATM, ATP11B, ATP1A1, ATP2B3, ATR, ATRX, AXIN1, BAP1, BCL10, BCL11A, BCL11B, BCL2, BCL2L1, BCL3, BCL5, BCL6, BCL7A, BCL9, BCOR, BCR, BHD, BIRC2, BIRC3, BLM, BMPR1A, BRAF, BRC42, BRCA1, BRCA2, BRD3, BRD4, BRIP1, BTG1, BUB1B, C12ORF9, C15ORF21, C15ORF55, C16ORF75, C2ORF44, CACNA1D, CALR, CAMTA1, CANT1, CARD11, CARS, CASP8, CBFA2T1, CBFA2T3, CBFB, CBL, CBLB, CBLC, CCDC6, CCNB1IP1, CCND1, CCND2, CCND3, CCNE1, CD273, CD274, CD44, CD74, CD79A, CD79B, CDC73, CDH1, CDH11, CDK12, CDK4, CDK6, CDKN2A, CDKN2A(PL4), CDKN2B, CDKN2C, CDX2, CEBPA, CEP1, CEP89, CHCHD7, CHEK2, CHIC2, CHN1, CIC, CIITA, CLIP1, CLTC, CLTCL1, CMKOR1, CNOT3, COL1A1, COL2A1, COPEB, COX6C, CREB1, CREB3L1, CREB3L2, CREBBP, CRLF2, CRTC3, CSF1R, CSF3R, CSNK2A1, CTNNB1, CUX1, CYLD, D10S170, DAXX, DCTN1, DDB2, DDIT3, DDR2, DDX10, DDX5, DDX6, DEK, DICERL, DNM2, DNMT3A, DUX4, EBFL, ECT2L, EGFR, EIF3E, EIF4A2, ELF4, ELK4, ELKS, ELL, ELN, EML4, EP300, EPS 15, ERBB2, ERBB2 (« HER2 »), ERBB3, ERBB4 (« HER4 »), ERC1, ERCC1, ERCC2, ERCC3, ERCC4, ERCC5, ERG, ESR1, ETV1, ETV4, ETV5, ETV6, EVI1, EWSR1, EXT1, EXT2, EZH2, EZR, FACL6, FAM46C, FANCA, FANCC, FANCD2, FANCE, FANGE, FANCG, FANCL, FAS, FBXOI1, FBXW7, FCGR2B, FEV, FGFR1, FGFR10P, FGFR2, FGFR3, FGFR4, FH, FHIT, FIP1L1, FLJ27352, FLT3, FLU, FNBP1, FOX03A, FOX04, FOXA1, FOXL2, FOXOIA, FOXP1, FSTL3, FUBP1, FUS, FVT1, GABRA6, GABRG2, GAS6, GAS7, GATA1, GATA2, GATA3, GMPS, GNA11, GNAQ, GNAS, GOLGA5, GOPC, GPC3, GPHN, GRAF, H3F3A, H3F3B, HCMOGT-1, HEAB, HERPUD1, HEY1, HIP1, HIST1H3B, HIST1H4L, HLA-A, HLF, HLXB9, HMGA1, HMGA2, HNF1A, HNRNPA2B1, HOOK3, HOXA11, HOXA13, HOXA9, HOXC11, HOXC13, HOXD11, HOXD13, HPAS, HRAS, HSPCA, HSPCB, IDH1, IDH2, IGF1R, IGH, IGK, IGL, IHD2, IKZF1, IL2, IL21R, IL6, IL6ST, IL7R, IRF4, IRTA1, ITK, JAK1, JAK2, JAK3, JAZF1, JUN, KCNJ5, KDM5A, KDM5C, KDM6A, KDR, KIAA1549, KIAA1598, KIF5B, KIT, KLF4, KLK2, KMT2A, KMT2D, KRAS, KTN1, LAF4, LASP1, LCK, LCP1, LCX, LHFP, LIFR, LM02, LMNA, LMOL, LPP, LRIG3, LSM14A, LYL1, MAF, MAFB, MALAT1, MALT1, MAML2, MAP2K1, MAP2K2, MAP2K4, MAX, MCL1, MDM2, MDM4, MDS1, MDS2, MECOM, MECT1, MED 12, MEN1, MET, MITF, MKL1, MLF1, MLH1, MLL, MLL3, MLLT1, MLLT10, MLLT2, MLLT3, MLLT4, MLLT6, MLLT7, MN1, MPL, MSF, MSH2, MSH6, MSI2, MSN, MTCP1, MTOR, MUC1, MUTYH, MY018A, MY05A, MYB, MYC, MYC1, MYCL1, MYCN, MYD88, MYH11, MYH9, MYST4, NAB2, NACA, NBSL, NCOA1, NCOA2, NCOA4, NDRG1, NF1, NF2, NFATC2, NFE2L2, NFIB, NFKB2, NIN, NKX2-1, NKX2-1, NKX2-8, NONO, NOTCH1, NOTCH2, NPM1, NR4A3, NRAS, NRAS/CSDE1, NRG1, NSD1, NT5C2, NTRK1, NTRK3, NUMA1, NUP214, NUP98, NUTM2A, NUTM2B, OLIG2, OMD, P2RY8, PAFAH1B2, PALB2, PARP1, PARP2, PAX3, PAX5, PAX7, PAX8, PBRM1, PBX1, PCM1, PCSK7, PDCD1LG2, PDE4DIP, PDGFB, PDGFRA, PDGFRB, PERI, PHF6, PHOX2B, PICALM, PIK3CA, PIK3R1, PIM1, PLAG1, PLCG1, PML, PMS1, PMS2, PMX1, PNP, PNUTL1, POT1, POU2AF1, POU5F1, PPARG, PPFIBP1, PPP2R1A, PRCC, PRDM1, PRDM16, PRF1, PRKAR1A, PSIP1, PTCH1, PTEN, PTPN11, PTPRB, PTPRC, PTPRK, PWWP2A, RAB5EP, RAC1, RAD21, RAD51, RAD51L1, RAF1, RAFT, RALGDS, RANBP17, RAP1GDS1, RARA, RB1, RBI, RBM15, RECQL4, REL, RET, RHEB, RHOA, RIT1, RNF43, ROS1, RPL10, RPL22, RPL5, RPN1, RPS6KB1, RSP02, RSP03, RUNDC2A, RUNX1, RUNX1T1, RUNXBP2, SBDS, SDC4, SDH5, SDHB, SDHC, SDHD, SET, SETBP1, SETD2, SF3B1, SFPQ, SFRS3, SH2B3, SH3GL1, SIL, SLC34A2, SLC45A3, SMAD4, SMARCA4, SMARCB1, SMARCE1, SMO, SOCS1, SOX2, SRC, SRGAP3, SRSF2, SS18, SS18L1, SSX1, SSX2, SSX4, STAG2, STAT3, STAT5B, STAT6, STK11, STL, SUFU, SUZ12, SYK, TAF15, TAL1, TAL2, TALI, TBL1XR1, TCEA1, TCF1, TCF12, TCF3, TCF7L2, TCL1A, TCL6, TERT, TET2, TFE3, TFEB, TFG, TFPT, TFRC, THRAP3, TIAF1, TIF1, TLX1, TLX3, TMPRSS2, TNFAIP3, TNFRSF14, TNFRSF17, TOPI, TP53, TPM3, TPM4, TPR, TRA, TRAF7, TRB, TRD, TRIM27, TRIM33, TRIP11, TRRAP, TSC1, TSC2, TSHR, TTL, U2AF1, UBR5, USP6, VHL, VT11A, WAS, WHSC1, WHSC1L1, WIF1, WRN, WT1, WWTR1, XPA, XPC, XPO1, YWHAE, ZCCHC8, ZNF145, ZNF198, ZNF217, ZNF278, ZNF331, ZNF384, ZNF521, ZNF9, ZRSR2 et ZRSR2 ;
(b) chaque séquence nucléotidique de la pluralité de séquences nucléotidiques est une sous-séquence d'un gène sélectionné parmi BMPR1A (récepteur de protéine morphogénétique osseuse, type IA), BRCA1 (cancer du sein 1), BRCA2 (cancer du sein 2), CDH1 (cadhérine-1), CDKN2A (inhibiteur de kinase dépendant de la cycline 2A), EPCAM (molécule d'adhésion des cellules épithéliales), MLH1 (homologue 1 de mutL), MSH2 (homologue 2 de mutS), MSH6 (homologue 6 de mutS), MUTYH (ADN glycosylase mutY), PALB2 (partenaire et localisateur de BRCA2), PMS2 (homologue 2 de PMS1, composant du système de réparation des mésappariements), PTEN (homologue de la phosphatase et de la tensine), SMAD4 (membre de la famille SMAD 4 ; mères contre homologue de décapentaplégique 4), STK11 (sérine/thréonine kinase 11) et TP53 (protéine tumorale p53) ;
(c) l'acide nucléique est tel qu'indiqué dans la partie (b) et chaque génotype d'une séquence nucléotidique de la pluralité de séquences nucléotidiques est sélectionné dans le groupe constitué de la mutation 942+3A>T au gène MSH2, de la mutation c.1662-121677del au gène MSH2, de la mutation c.2056_2060delinsCTTCTACCTCAAAAA (SEQ ID NO:13) au gène MSH6, de la mutation c.2308_2312delGGTAAinsT au gène MSH6, de la mutation c.2641delGinsAAAA au gène MSH6, de la mutation c.3163_3164insG au gène MSH6, de la mutation c.232_243delinsATGTAAGG au gène MLH1, de la mutation c.1852_1854delAAG au gène MLH1, de la mutation c.2445+1G>C au gène PMS2, de la mutation c.2243_2246delAGAA au gène PMS2, de la mutation c.2253T>C au gène PMS2, de la mutation c.861_864delACAG au gène PMS2, de la mutation c.9_32dup24 au gène CDKN2A, de la mutation c.8975_9100del126 au gène BRCA2, de la mutation c.9203del126 au gène BRCA2, de la mutation c.1310_1313delAAGA au gène BRCA2, de la mutation c.1813dupA au gène BRCA2, de la mutation c.9342_9343insAluY au gène BRCA2, de la mutation c.5266_5267insC au gène BRCA1, de la mutation c.5177_5180delGAAA au gène BRCA1, de la mutation c.3756_3759delGTCT au gène BRCA1, de la mutation c.3481_3491delGAAGATACTAG (SEQ ID NO:14) au gène BRCA1, de la mutation c.3113A>G au gène BRCA1, de la mutation c.3084_3094delTAATAACATTA (SEQ ID NO:15) au gène BRCA1, de la mutation c.2834_2836delinsC au gène BRCA1 et de la mutation c.68_69delAG au gène BRCA1 ;
(d) l'acide nucléique est tel qu'indiqué dans la partie (b) ou (c) et
l'acide nucléique présente au moins 80 % d'homologie de séquence avec la séquence nucléotidique présentée dans SEQ ID NO:2 ou SEQ ID NO:3, ou
présente la séquence nucléotidique présentée dans SEQ ID NO:2 ou SEQ ID NO:3 ;
(e) l'acide nucléique est tel qu'indiqué dans la partie (b) et
chaque génotype d'une séquence nucléotidique de la pluralité de séquences nucléotidiques est sélectionné dans le groupe constitué de la mutation c.675+1G>C au gène BMPR1A, de la mutation c.817C>T au gène BMPR1A, de la mutation c.1016dupA au gène BRCA1, de la mutation c.1175_1214delTGTTAGGTTCTGATGACTCACATGATGGGGAGTCTGAATC (SEQ ID NO:23) au gène BRCA1, de la mutation c.1387_1390delinsGAAAG au gène BRCA1, de la mutation c.181T>G au gène BRCA1, de la mutation c.1953_1956delGAAA au gène BRCA1, de la mutation c.2834_2836delinsC au gène BRCA1, de la mutation c.3084_3094delTAATAACATTA (SEQ ID NO:15) au gène BRCA1, de la mutation c.3113A>G au gène BRCA1, de la mutation c.3481_3491delGAAGATACTAG (SEQ ID NO:14) au gène BRCA1, de la mutation c.3756_3759delGTCT au gène BRCA1, de la mutation c.406_407insA au gène BRCA1, de la mutation c.4964_4982delCTGGCCTGACCCCAGAAGA (SEQ ID NO:19) au gène BRCA1, mutation c.5096G>A au gène BRCA1, de la mutation c.5177_5180delGAAA au gène BRCA1, mutation c.5177_5180delGAAA au gène BRCA1, de la mutation c.5266_5267insC au gène BRCA1, de la mutation c.68_69delAG au gène BRCA1, de la mutation c.815_824dupAGCCATGTGG (SEQ ID NO:25) au gène BRCA1, de la mutation c.10095delCinsGAATTATATCT (SEQ ID NO:29) au gène BRCA2, de la mutation c.1310_1313delAAGA au gène BRCA2, de la mutation c.1310_1313delAAGA au gène BRCA2, de la mutation c.156_157insAluYa5 au gène BRCA2, de la mutation c.1813dupA au gène BRCA2, de la mutation c.2588dupA au gène BRCA2, de la mutation c.2808_2811delACAA au gène BRCA2, de la mutation c.4037_4043delinsT au gène BRCA2, de la mutation c.5073dupA au gène BRCA2, de la mutation c.5350_5351delAA au gène BRCA2, de la mutation c.5946delT au gène BRCA2, de la mutation c.6275_6276delTT au gène BRCA2, de la mutation c.7762_7764delinsTT au gène BRCA2, de la mutation c.8537_8538delAG au gène BRCA2, de la mutation c.8902_8913delinsTCCC au gène BRCA2, de la mutation c.9203dell26 au gène BRCA2, de la mutation c.9253dupA au gène BRCA2, de la mutation c.9342_9343insAluY au gène BRCA2, de la mutation c.1792C>T au gène CDH1, de la mutation c.9_32dup24 au gène CDKN2A, de la mutation c.429G >A au gène EPCAM, de la mutation c.523C>T au gène EPCAM, de la mutation c.556-14A>G au gène EPCAM, de la mutation c.1852_1854delAAG au gène MLH1, de la mutation c.1852_1854delAAG au gène MLH1, de la mutation c.232_243delinsATGTAAGG au gène MLH1, de la mutation c.382delG au gène MLH1, de la mutation c.704_723delATAAAACCCTAGCCTTCAAA (SEQ ID NO:33) au gène MLH1, de la mutation c.1076G>C au gène MSH2, de la mutation c.1662-12_1677del au gène MSH2, de la mutation c.942+3A>T au gène MSH2, de la mutation c.942+3A>T au gène MSH2, de la mutation c.2056_2060delinsCTTCTACCTCAAAAA (SEQ ID NO:13) au gène MSH6, de la mutation c.2308_2312delGGTAAinsT au gène MSH6, de la mutation c.2641delGinsAAAA au gène MSH6, de la mutation c.3163_3164insG au gène MSH6, de la mutation c.3261delC au gène MSH6, de la mutation c.3939_3957dupTCAAAAGGGACATAGAAAA (SEQ ID NO:36) au gène MSH6, de la mutation c.741delA au gène MSH6, de la mutation c.1147delC au gène MUTYH, de la mutation c.1187G>A au gène MUTYH, de la mutation c.1435G>T au gène MUTYH, de la mutation c.536A>G au gène MUTYH, de la mutation c.933+3A>C au gène MUTYH, de la mutation c.2386G>T au gène PALB2, de la mutation c.3113G>A au gène PALB2, de la mutation c.1261C>T au gène PMS2, de la mutation c.137G>T au gène PMS2, de la mutation c.2117delA au gène PMS2, de la mutation c.2243_2246delAGAA, c.2253T>C au gène PMS2, de la mutation c.2445+1G>C au gène PMS2, de la mutation c.861_864delACAG au gène PMS2, de la mutation 861_864delACAG au gène PMS2, de la mutation c.511C>T au gène PTEN, de la mutation c.758_759insAT au gène PTEN, de la mutation c.987_996dupTAAAGACAAA (SEQ ID NO:38) au gène PTEN, de la mutation c.1206_1207insT au gène SMAD4, de la mutation c.1353_1354insGCTACTGCACAAGCTGCAGCAGCTGCCC (SEQ ID NO:40) au gène SMAD4, de la mutation c.1529delG au gène SMAD4, de la mutation c.842_843insC au gène STK11, de la mutation c . 637C>T au gène TP53 et de la mutation c.916C>T au gène TP53.

6. Acide nucléique selon la revendication 1 ou 2, dans lequel :
(a) le génotype de chaque séquence nucléotidique de la pluralité de séquences nucléotidiques est associé à une maladie cardiaque, facultativement une cardiomyopathie ;
(b) le génotype de chaque séquence nucléotidique de la pluralité de séquences nucléotidiques est associé à une maladie cardiaque, facultativement une cardiomyopathie, et
chaque séquence nucléotidique de la pluralité de séquences nucléotidiques comprend une sous-séquence d'un gène sélectionné dans le groupe constitué de la protéine C de liaison à la myosine cardiaque (MYBPC3) ; de la myosine, de la chaîne lourde 7, du muscle cardiaque, du gène bêta (MYH7) ; de la troponine I de type 3 (TNNI3) ; de la troponine T cardiaque (TNNT2) ; et de la chaîne alpha-1 de tropomyosine (TPM1) ; et
chaque sous-séquence comprend une mutation associée à une cardiomyopathie ; facultativement
dans lequel chaque mutation est sélectionnée dans le groupe constitué :
d'une mutation au gène de la protéine C de liaison à la myosine cardiaque (MYBPC3) sélectionnée dans le groupe constitué de 1504C>T, 2373_2374insG et 3628-41_3628-17del ;
d'une mutation au gène de la myosine, de la chaîne lourde 7, du muscle cardiaque, du gène bêta (MYH7) sélectionné dans le groupe constitué de 1988G>A, 1357C>T et 1750G>C ;
d'une mutation du gène de la troponine I de type 3 (TNNI3) sélectionné dans le groupe constitué de 532_534delAAG et 575G>A ;
d'une mutation au gène de la troponine T cardiaque (TNNT2), dans lequel la mutation est 487_489delGAG ; et
d'une mutation au gène de la chaîne alpha-1 de la tropomyosine (TPM1), dans lequel la mutation est 574G>A ;
(c) le génotype de chaque séquence nucléotidique de la pluralité de séquences nucléotidiques est associé à une maladie cardiaque, facultativement une cardiomyopathie, et
chaque séquence nucléotidique de la pluralité de séquences nucléotidiques est une sous-séquence d'un gène sélectionné parmi MYBPC3, MYH7, TNNI3, TNNT2 et TPM1, facultativement
dans lequel chaque génotype d'une séquence nucléotidique de la pluralité de séquences nucléotidiques est sélectionné dans le groupe constitué de la mutation 1504C>T au gène MYBPC3, de la mutation 2373_2374insG au gène MYBPC3, de la mutation 3628-41_3628-17del au gène MYBPC3, de la mutation 1988G>A au gène MYH7, de la mutation 1357C>T au gène MYH7, mutation 1750G>C au gène MYH7, de la mutation 532_534delAAG au gène TNNI3, de la mutation 575G>A au gène TNNI3, de la mutation 487_489delGAG au gène TNNT2 et de la mutation 574G>A au gène TPM1 ;
(d) le génotype de chaque séquence nucléotidique de la pluralité de séquences nucléotidiques est associé à une maladie cardiaque, facultativement une cardiomyopathie, et
chaque séquence nucléotidique de la pluralité de séquences nucléotidiques est une sous-séquence d'un gène sélectionné parmi ABCC9, ACTC1, ACTN2, AKAP9, ANK2, ANKRD1, BAG3, BRAF, CACNA1C, CACNB2, CASQ2, CAV3, CRYAB, CSRP3, DES, DMD, DSC2, DSG2, DSP, DTNA, EMD, FKTN, GATAD1, GLA, GPD1L, HCN4, HRAS, ILK, JPH2, JUP, KCNE1, KCNE2, KCNE3, KCNH2, KCNJ2, KCNJ5, KCNJ8, KCNQ1, KRAS, LAMA4, LAMP2, LDB3, LMNA, MAP2K1, MAP2K2, MTND1, MTND5, MTND6, MTTD, MTTG, MTTH, MTTI, MTTK, MTTL1, MTTL2, MTTM, MTTQ, MTTS1, MTTS2, MYBPC3, MYH7, MYL2, MYL3, MYLK2, MYOZ2, MYPN, NEBL, NEXN, NKX2.5, NRAS, PDLIM3, PKP2, PLN, PRKAG2, PTPN11, RAF1, RANGRF, RBM20, RYR2, SCN1B, SCN3B, SCN4B, SCN5A, SGCD, SNTA1, SOS1, TAZ, TCAP, TMEM43, TMPO, TNNC1, TNNI3, TNNT2, TPM1, TTN, TTR et VCL ; et/ou
(e) l'acide nucléique présente au moins 80 % d'homologie de séquence avec la séquence nucléotidique présentée dans SEQ ID NO:4 ou
présente la séquence nucléotidique présentée dans SEQ ID NO:4.

7. Acide nucléique selon l'une quelconque des revendications 1 à 6, dans lequel :
(a) chaque séquence nucléotidique de la pluralité a une longueur d'au moins 600 nucléotides, une longueur d'environ 600 nucléotides à environ 2 000 nucléotides, une longueur d'environ 600 nucléotides à environ 1 200 nucléotides, une longueur d'environ 1 000 nucléotides à environ 10 000 nucléotides ; et/ou
(b) l'acide nucléique comprend en outre une origine de réplication, facultativement dans lequel l'origine de réplication est une origine de réplication provenant *d'Escherichia coli* ou de *Saccharomyces cerevisiae* et/ou
(c) l'acide nucléique comprend en outre au moins un nucléoside ou nucléotide méthylé ; et/ou
(d) l'acide nucléique comprend en outre un promoteur, dans lequel facultativement le promoteur est un promoteur SP6 ; et/ou
(e) l'acide nucléique comprend en outre un promoteur, dans lequel le promoteur provient d'une espèce différente de celle des séquences nucléotidiques de la pluralité, facultativement dans lequel le promoteur est un promoteur SP6 ; et/ou l'acide nucléique est un plasmide.

8. Cellule comprenant l'acide nucléique selon l'une quelconque des revendications 1 à 7.

9. Cellule selon la revendication 8, dans laquelle :
(a) la cellule est une cellule humaine ; et/ou
(b) dans laquelle la cellule est un fibroblaste ou un lymphocyte, facultativement un lymphocyte B immortalisé ; et/ou
(c) la cellule est GM12878, GM24149, GM24143, GM24385, GM24631, GM24694 ou GM24695, facultativement GM24385 ; et/ou
(d) la cellule comprend 1 à 1 000 copies ou 5 à 500 copies de l'acide nucléique ; ou
(e) la cellule est *E. coli.*

10. Composition, comprenant une première pluralité de cellules et une seconde pluralité de cellules, dans laquelle :
la première pluralité de cellules est constituée de cellules selon la revendication 9, partie (d), lesquelles cellules comprennent 5 à 500 copies de l'acide nucléique ;
la seconde pluralité de cellules est constituée de cellules qui ne comprennent pas l'acide nucléique ; la première pluralité de cellules et la seconde pluralité de cellules sont des cellules humaines ;
la première pluralité de cellules et la seconde pluralité de cellules sont mélangées dans la composition ; et
le rapport du nombre de cellules de la première pluralité au nombre de cellules de la seconde pluralité est d'environ 1:1 à environ 1:10 000 dans la composition.

11. Procédé de fabrication d'un matériau biologique de référence, comprenant la transfection d'une pluralité de cellules avec l'acide nucléique selon l'une quelconque des revendications 1 à 7 ; comprenant facultativement en outre :
(a) la fixation des cellules de la pluralité, facultativement dans lequel la fixation des cellules comprend la fixation des cellules avec du formol ; et/ou
(b) l'incorporation des cellules dans de la paraffine ; et/ou
(c) la dilution de la pluralité de cellules avec des cellules non transfectées, facultativement, dans lequel la dilution de la pluralité de cellules avec des cellules non transfectées comprend la dilution à un rapport des cellules transfectées aux cellules non transfectées d'environ 1:1 à environ 1:10 000.

12. Matériau biologique de référence, comprenant
(a) une pluralité de cellules selon l'une quelconque des revendications 8 ou 9, parties (a) à (d) et de la paraffine, dans lequel la pluralité de cellules est fixée et incorporée dans la paraffine, comprenant facultativement en outre des cellules non transfectées, dans lequel les cellules non transfectées ne comprennent pas l'acide nucléique, facultativement dans lequel le rapport des cellules comprenant l'acide nucléique aux cellules non transfectées est d'environ 1:1 à environ 1:10 000, d'environ 1:5 à environ 1:5 000 ou d'environ 1:10 à environ 1:1 000 ; ou
(b) une pluralité de cellules selon l'une quelconque des revendications 8 ou 9, parties (a) à (d) ; et un liquide, facultativement dans lequel le liquide est du plasma.

13. Composition comprenant un acide nucléique et un tampon aqueux, dans laquelle l'acide nucléique est un acide nucléique selon l'une quelconque des revendications 1 à 7 qui a été extrait du matériau de référence selon la revendication 12, facultativement dans laquelle le tampon comprend du tris(hydroxyméthyl)aminométhane et de l'acide éthylènediaminetétraacétique, ou un sel de l'un quelconque de ceux-ci.
